# EUROPEAN PATENT SPECIFICATION

(11) **EP 2 257 299 B1**
(45) Date of publication and mention of the grant of the patent: **25.07.2018**
(21) Application number: 09718390.9
(22) Date of filing: 03.03.2009
(51) Int. Cl.: A61K 31/713, C07K 14/435, C12N 15/11, A61K 39/395

(54) **MODULATION OF SRPX2-MEDIATED ANGIOGENESIS**
MODULATION VON SRPX2-VERMITTELTER ANGIOGENESE
MODULATION DE L ANGIOGENÈSE MÉDIÉE PAR SRPX2

(30) Priority: 07.03.2008 US 34786 P
(43) Date of publication of application: 08.12.2010
(73) Proprietor: Research Development Foundation, Carson City, NV 89703 (US)
(72) Inventor: IMHOF, Beat, A., CH-1211 Geneva 4 (CH); MILJKOVIC-LICINA, Marijana, CH-1211 Geneva 4 (CH); HAMMEL, Philippe, CH-1211 Geneva 4 (CH)
(74) Representative: Vossius & Partner Patentanwälte Rechtsanwälte mbB
(86) International application number: PCT/US2009/035831
(87) International publication number: WO 2009/111444

(56) References cited:
- WO-A-03/080640
- TANAKA KAORU ET AL: "SRPX2 is a novel therapeutic target in gastric cancer." PROCEEDINGS OF THE AMERICAN ASSOCIATION FOR CANCER RESEARCH ANNUAL MEETING, vol. 48, April 2007 (2007-04), page 474, XP002540495 & 98TH ANNUAL MEETING OF THE AMERICAN-ASSOCIATION-FOR-CANCER-RESEARCH; LOS ANGELES, CA, USA; APRIL 14 -18, 2007 ISSN: 0197-016X

### SEQUENCE LISTING

<110> LICINA-MILJKOVIC, MARIJANA
   HAMMEL, PHILIPPE
   IMHOF, BEAT A.
<120> MODULATION OF SRPX2-MEDIATED ANGIOGENESIS
<130> CLFR:311WO
<140> UNKNOWN
   <141> 2009-03-03
<150> 61/034,786
   <151> 2008-03-07
<160> 31
<170> PatentIn version 3.5
<210> 1
   <211> 2206
   <212> DNA
   <213> Homo sapiens
<400> 1
   actatattca caggcttgga gccagtgcca ttcacacttc cccctcttct gcagcagacg      60
   gactgagttc ctctaatccc tgtgttcctt ctcccccatc tttctaaaac ccttctctga     120
   gagaggaata actatagctt cagggataat atagctttaa ggaaactttt ggcagatgtg     180
   gacgtcgtaa catctgggca gtgttaacag aatcccggag gccgggacag accaggagcc     240
   actcgttcta ggaatgttaa agtagaaggt tttttccaat tgatgagagg agcagagagg     300
   aaggagaaag aggaggagag agaaaaaggg cacaaaatac cataaaacag atcccatatt     360
   tctgcttccc ctcactttta gaagttaatt gatggctgac ttctgaaagt cactttcctt     420
   tgccctggta cttcaggcca tatacatctt ttcttgtctc cataatcctc cctttcaagg     480
   atggccagtc agctaactca aagaggagct ctctttctgc tgttcttcct aactccggca     540
   gtgacaccaa catggtatgc aggttctggc tactatccgg atgaaagcta caatgaagta     600
   tatgcagagg aggtcccaca ggctcctgcc ctggactacc gagtcccccg atggtgttat     660
   acattaaata tccaggatgg agaagccaca tgctactcac cgaagggagg aaattatcac     720
   agcagcctgg gcacgcgttg tgagctctcc tgtgaccggg gctttcgatt gattggaagg     780
   aggtcggtgc aatgcctgcc aagccgtcgt tggtctggaa ctgcctactg caggcagatg     840
   agatgccacg cactaccatt catcactagt ggcacttaca cctgcacaaa tggagtgctt     900
   cttgactctc gctgtgacta cagctgttcc agtggctacc acctggaagg tgatcgcagc     960
   cgaatctgca tggaagatgg gagatggagt ggaggcgagc ctgtatgtgt agacatagat    1020
   ccccccaaga tccgctgtcc ccactcacgt gagaagatgg cagagccaga gaaattgact    1080
   gctcgagtat actgggaccc accgttggtg aaagattctg ctgatggtac catcaccagg 1140
   gtgacacttc ggggccctga gcctggctct cactttcccg aaggagagca tgtgattcgt 1200
   tacactgcct atgaccgagc ctacaaccgg gccagctgca agttcattgt gaaagtacaa 1260
   gtgagacgct gcccaactct gaaacctccg cagcacggct acctcacctg cacctcagcg 1320
   ggggacaact atggtgccac ctgtgaatac cactgtgatg gcggttatga tcgccagggg 1380
   acaccctccc gggtctgtca gtccagccgc cagtggtcag gttcaccacc aatctgtgct 1440
   cctatgaaga ttaacgtcaa cgtcaactca gctgctggtc tcttggatca attctatgag 1500
   aaacagcgac tcctcatcat ctcagctcct gatccttcca accgatatta taaaatgcag 1560
   atctctatgc tacagcaatc cacctgtgga ctggatttgc ggcatgtgac catcattgaa 1620
   ctggtgggac agccacctca ggaggtgggg cgcatccggg agcaacagct gtcagccaac 1680
   atcatcgagg agctcaggca atttcagcgc ctcactcgct cctacttcaa catggtgttg 1740
   attgacaagc agggtattga ccgagaccgc tacatggaac ctgtcacccc cgaggaaatc 1800
   ttcacattca ttgatgacta cctactgagc aatcaggagt tgacccagcg tcgggagcaa 1860
   agggacatat gcgagtgaac ttgagccagg gcatggttaa agtcaaggga aaagctcctc 1920
   tagttagctg aaactgggac ctaataaaag gaggaaatgt tttcccacag ttctagggac 1980
   aggactctga ggtgggtgag tttgacaaat cctgcagtgt ttccaggcat ccttttagga 2040
   ctgtgtaata gtttccctag aagctaggta gggactgagg acaggccttg ggcagtgggt 2100
   tgggggtaga agttcttcct ttcctaaccc gggcccctgc ccagctctcc aaagtctttc 2160
   agaaaagtaa atcctaaatt cagtgatgaa aaaaaaaaaa aaaaaa 2206
<210> 2
   <211> 2938
   <212> DNA
   <213> Mus musculus
<400> 2
   ctccacccag agcttgaaac cagtgacagt cacacttccc ctcttctgca gcagacagca      60
   ctagctcctc taatcctctt gcttccccct cccccaacca tttcttgggg aataacaaat     120
   atagctttgg ggataatata gctttaagac gacttttggc aaatgtaaat gtcctaacat     180
   ctgggcagtg ttaccagaat cccggaggcc ctgacagacc aggagccact ggttctagga     240
   atgttaaagt acaagggctt tttcccaccc ccgactgact gatgagagga gcagagagca     300
   aagaaaaaga agagagatga acgagaccac aaaaatcata aaataaaaag cagatgcatg     360
   ttcctgctct tttcaaagct tccagtagaa cgatagctcc ctccgatgtc atatgcctgc     420
   ttcctctcac ttttgggagt tcatagctgg ttctgctctg aaagtcattc ccctttatcc     480
   tggtacttct ggccacatag ccctagtctt gtcttctgaa gcttccctgt cgacgatgat      540
   gaccagtcca ctgactcaga gaggtgctct ctcactgctg ctcctcctaa tgcccgcagt      600
   gacaccaaca tggtacgcag gctcaggtta ctctccagat gaaagctaca atgaagtata      660
   tgcagaagag gtccccgctg cccgtgcccg tgccctggac tacagagtcc cccgatggtg      720
   ttacacattg aatatccagg atggagaagc cacatgctac tcaccaaggg gaggaaatta      780
   tcacagtagc ctaggcacac gttgtgagct ctcctgtgac cggggctttc gattgattgg      840
   acggaagtca gtgcaatgtt tgccaagccg gcgttggtct ggaactgcct actgcaggca      900
   gataaggtgc cacacactgc cattcatcac tagtggcacc tatacgtgca ccaatggaat      960
   gctgcttgac tcccgttgtg actatagctg ttctagtggc taccacctag aaggagatcg     1020
   cagccgcatc tgcatggaag atgggcgatg gagtggaggc gagcctgtat gtgtagacat     1080
   agatccaccc aagatccgtt gtcctcactc tcgtgagaaa atggcagagc cagagaaact     1140
   aactgctcga gtatactggg acccaccctt agtgaaagat tctgctgatg gtaccatcac     1200
   cagggtgaca cttcggggtc cagagcctgg ttctcacttc cctgaaggag aacatgtgat     1260
   tcgttatact gcctatgacc gagcctacaa ccgggccagc tgcaaattca ttgtaaaagt     1320
   acaagtgaga cgctgtccca ttctgaagcc accacagcac ggctacctca cctgcagctc     1380
   agcgggggac aactatggtg ccatctgtga ataccactgt gatggtggtt atgaacgcca     1440
   ggggacccca tcccgagtct gccagtcaag tcgacagtgg tcaggaacac cacctgtctg     1500
   tactcctatg aagattaatg tcaatgttaa ctcggctgct ggcctcctgg atcagttcta     1560
   tgagaaacag cgactcctca tagtctctgc tcctgatccc tccaatcggt actacaaaat     1620
   gcaaatctct atgctgcagc aatccacctg tggactcgac ctgcggcatg tgaccattat     1680
   tgagctggtg ggacagccac ctcaggaggt ggggcgcatc cgggagcaac aactatcagc     1740
   aggcatcatt gaggagctca ggcaattcca gcgcctcact cgttcctact tcaacatggt     1800
   gttaattgac aagcagggta ttgaccggga acgctatatg gaacctgtca cccctgagga     1860
   aatcttcaca ttcatcgatg actacctgct gagcaatgag gagctggcac ggagagtgga     1920
   gcagagggac ctatgcgagt gaccttgagt cagggtgtgg ctgaagcacc tgtcctaggg     1980
   agcttaaact aaggaggaaa tgtcgtgtcc tccccccccc acacacacac acacacactg     2040
   ttgggagcag ccctcgggtg ggggtgaggt ttgccaaatc taggacagtt ccaggcagca     2100
   ttttagggca gaatattcat ttccctaagc aagtctccac ttcgggtagc actggaggag     2160
   cctatgaaac tgaggacatg ccctgggtgg tgagttgtag accgaaggcc ttcttcacct     2220
   gcctggagcc tctctctcta gactcttccc aaagcctttc agataagtaa ataccaaatt     2280
   cattccttta cggtgttgta aatggttcct ctaccctaca ataacagcag ggggcagcat     2340
   tgcaacagac aaacaagaca ctttgaccaa gtataaatag atttccctca cagttagtta     2400
   tgtgaggaac aggagagagg agactaggat ctacaaaaca ttcttgaagc tgctcgtcat     2460
   cctctaggat gctggcctta aaaacaatgt tgcttgagcc atttcttcat caagagttag     2520
   aaaaacattt tctccagggg gagtttggga agcaacgtct agccatagct ggctgttccc     2580
   tcctagatgc tccaactagc ttgtaggcaa gaacttctaa taactgaggt gtttagtacc     2640
   ctggtgacag ctcttcctta gaggattctg cagtctgtga acaacattac ctctaagaat     2700
   tagggagatg gctccgttgg tgaagtgctt gctatcaagc actgagaccc gagtgattcc     2760
   cagaacccat gtaaaatagc cagatgtggt ggtgtgcact tggaatccca gcagagaggt     2820
   agacataaac agatctgtgg ggatcactgc ttagcaagcc aagtctaatg atgagtacca     2880
   ggtcatgtaa gaaacactat ctcaaaaacc atggtaaatg gcataaaaaa aaaaaaac       2938
<210> 3
   <211> 1905
   <212> DNA
   <213> Bos taurus
<400> 3
   gaaggagaaa gagaagagag agtacaaaaa acccgtacat cagatcctgc atttctgctt      60
   cctctcactt ttggaagttc attgatgcct gatttctgaa agtcatcctc ctttgccttg     120
   gtgcttctgg ccacatacct ctattcttgt ctccctaacc tcccttccca tccctcccca     180
   ccaccttttc aaggatggcc attcaactaa ctcgaagagg agctctctct ctgctgctct     240
   tcctcactcc agcagtgatg ccaacatggt atgcaggctc aggctactac ccagatgaaa     300
   gctacaatga agtctatgct gaagaggtcc cacagactcc catcctagac tacaaagtcc     360
   cccgatggtg ttatacatta aatatccagg atggagaagc cacatgctac tcacctagag     420
   gaggaaatta tcacagcagc ctgggtactc gctgtgagct ttcttgtgac cggggctttc     480
   gactgattgg acggagatcc gtgcaatgcc tgccaagccg ccgttggtct ggaactgcct     540
   actgcaggca gatgagatgc catgcattgc cattcatcac gagtggcacc tacacgtgca     600
   caaatggggt gcttcttgac tctcgctgtg actacagctg ttccagtggc taccacttgg     660
   aaggtgaccg cagccgaatc tgcatggaag atgggcgatg gagcggagga gagcctgtat     720
   gtgtagacat agatcccccc aagatccgtt gtcctcactc ccgtgagaag atggcagagc     780
   cggagaaact gaccgctcga gtatactggg acccacccgt ggtgaaagat tctgctgatg     840
   gtaccatcac caggttgaca cttcggggcc ctgagcctgg ctctcacttt ccagaaggag     900
   aacacgtgat tcgttacact gcttatgacc gagcctacaa ccgggccagc tgcaagttca     960
   ttgtaaaagt acaagtgaga cgctgcccaa ctctgaaacc accactgcat ggctacctca      1020
   cctgcacctc agcgggggac aactatggtg ccacctgtga ataccactgt gatggagggt      1080
   atgagcgcca agggacctcc tcccgggtct gccagtccag ccgccagtgg tcaggatcac      1140
   cacccgtctg tgttcctatg aagattaatg tcaatgtcaa ctcagctgct ggccttctgg      1200
   atcagttcta tgagaaacgg cgactcctca tcatctcagc ccctgatccc tccaaccgat      1260
   attataaaat gcaaatctct atgctacagc aatccacttg tggactggac ctgaggcatg      1320
   tgaccatcat cgagctggtg gggcagccac ctcaggaagt ggggcgcatc cgggagcatc      1380
   agctgtcagc caatatcatt gaggaactca ggcaatttca gcacctcact cgctcctact      1440
   tcaacatggt gttgattgac aagcagggaa ttgaccggga acgctacatg gaacctgtca      1500
   cccccgagga aatcttcacg ttcattgatg actatctact gagcaatgag gagctgatcc      1560
   aacgccggga acaaagagac atatgtgact gaacttgagt cggagcatgg ttgaggtcaa      1620
   gggaaaaggt tccctggtca gccaaaactg gggcctaatc aaagaagaaa atgctttccc      1680
   acagctctga ggaaaggact ctaaggtggg tgaggttccc tgatcctggg acatttccag      1740
   acacctttcc tcaagcaagt ctctgcttta ggtagcactg gaacagcata agaagctagg      1800
   gagggactga ggacaggccc tgggcagcag cttgtagata gaaagtctta cttctgccca      1860
   gctgtccaaa atctttcaga gaagtaaatt ctaaattcac tcact                      1905
<210> 4
   <211> 1398
   <212> DNA
   <213> Pan troglodytes
<400> 4
   atggccagtc agctaactca aagaggagct ctctttctgc tgttcttcct aactccggca      60
   gtgacaccaa catggtatgc aggttctggc tactatccgg atgaaagcta caatgaagta     120
   tatgcagagg aggtcccaca ggctcctgcc ctggactacc gagtcccccg atggtgttat     180
   acattaaata tccaggatgg agaagccaca tgctactcac cgaggggagg aaattatcac     240
   agcagcctgg gcacacgttg tgagctctcc tgtgaccggg gctttcgatt gattggaagg     300
   aggtcggtgc aatgcctgcc aagccgtcgt tggtctggaa ctgcctactg caggcagatg     360
   agatgccacg cactaccatt catcactagt ggcacttaca cctgcacaaa tggagtgctt     420
   cttgactctc gctgtgacta cagctgttcc agtggctacc acctggaagg tgatcgcagc     480
   cgaatctgca tggaagatgg gagatggagt ggaggcgagc ctgtatgtgt agacatagat     540
   ccccccaaga tccgctgtcc ccactcacgt gagaagatgg cagagccaga gaaattgact     600
   gctcgagtat actgggaccc accgttggtg aaagattctg ctgatggtac catcaccagg     660
   gtgacacttc ggggccctga gcctggctct cactttcccg aaggagagca tgtgattcgt     720
   tacactgcct atgaccgagc ctacaaccgg gccagctgca agttcattgt gaaagtacaa     780
   gtgagacgct gcccaactct gaaacctccg cagcacggct acctcacctg cacctcagcg     840
   ggggacaact atggtgccac ctgtgaatac cactgtgatg gcggttatga tcgccagggg     900
   acaccctccc gggtctgtca gtccagccgc cagtggtcag gttcaccacc aatctgtgct     960
   cctatgaaga ttaacgtcaa cgtcaactca gctgctggtc tcttggatca attctatgag    1020
   aaacagcgac tcctcatcat ctcagctcct gatccttcca accgatatta taaaatgcag    1080
   atctctatgc tacagcaatc cacctgtgga ctggatttgc ggcatgtgac catcattgaa    1140
   ctggtgggac agccacctca ggaggtgggg cgcatccggg agcaacagct gtcagccaac    1200
   atcatcgagg agctcaggca atttcagcgc ctcactcgct cctacttcaa catggtgttg    1260
   attgacaagc agggtattga ccgagaccgc tacatggaac ctgtcacccc cgaggaaatc    1320
   ttcacattca ttgatgacta cctactgagc aatcaggagt tgacccagcg tcgggagcaa    1380
   agggacatat gcgagtga                                                  1398
<210> 5
   <211> 1398
   <212> DNA
   <213> Gorilla gorilla
<400> 5
   atggccagtc aactaactca aagaggagct ctctctctgc tgttcttcct aactccggca      60
   gtgacaccaa catggtatgc aggttctggc tactatccgg atgaaagcta caatgaagta     120
   tatgcagagg aggtcccaca ggctcctgcc ctggactacc gagtcccccg atggtgttat     180
   acattaaata tccaggatgg agaagctaca tgctactcac cgaggggagg aaattatcac     240
   agcagcctgg gcacgcgttg tgagctctcc tgtgaccggg gctttcgatt gattggaagg     300
   aggtcggtgc aatgcctgcc aagccgtcgt tggtctggaa ctgcctactg caggcagatg     360
   agatgccacg cactaccatt catcactagt ggcacttaca cctgcacaaa tggagtgctt     420
   cttgactctc gctgtgacta cagctgttcc agtggctacc acctggaagg tgatcgcagc     480
   cgaatctgca tggaagatgg gagatggagt ggaggcgagc ctgtatgtgt agacatagat     540
   ccccccaaga tccgctgtcc ccactcacgt gagaagatgg cagagccaga gaaattgact     600
   gctcgagtat actgggaccc accgttggtg aaagattctg ctgatggtac catcaccagg     660
   gtgacacttc ggggccctga gcctggctct cactttcccg aaggagagca tgtgattcgt     720
   tacactgcct atgaccgagc ctacaaccga gccagctgca agttcattgt gaaagtacaa     780
   gtgagacgct gcccaactct gaaacctccg cagcacggct acctcacctg cacctcagcg     840
   ggggacaact atggtgccac ctgtgaatac cactgtgatg gcggttatga tcgccagggg     900
   acaccctccc gggtctgtca gtccagccgc cagtggtcag gttcaccacc aatctgtgct     960
   cctatgaaga ttaacgtcaa cgtcaactca gctgctggtc tcttggatca attctatgag    1020
   aaacagcgac tcctcatcat ctcagctcct gatccttcca accgatatta taaaatgcag    1080
   atctctatgc tacagcaatc cacctgtgga ctggatttgc ggcatgtgac catcattgaa    1140
   ctggtgggac agccacctca ggaggtgggg cgcatccggg agcaacagct gtcagccaac    1200
   atcatcgagg agctcaggca atttcagcgc ctcactcgct cctacttcaa catggtgttg    1260
   attgacaagc agggtattga ccgagaccgc tacatggaac ctgtcacccc cgaggaaatc    1320
   ttcacattca ttgatgacta cctactgagc aatcaggagt tgacccagcg tcgggagcaa    1380
   agggacatat gcgagtga                                                  1398
<210> 6
   <211> 1398
   <212> DNA
   <213> Papio sp
<400> 6
   atggccagtc aactaactca aagaggagct ctctctctgc tgttcttcct gactccagca      60
   gtgacaccaa catggtatgc aggttctggc tactatccag atgaaagcta caatgaagta     120
   tatgcagagg aggtcccacg ggctcctgcc ctggactacc gagttccccg atggtgttat     180
   acattaaata tccaggatgg agaagccaca tgctactcac cgaggggagg aaattatcac     240
   agcagcctgg gcacacgttg tgagctctcc tgtgaccggg gctttcgact gattggaaga     300
   aggtcggtgc aatgcctgcc aagccgtcgc tggtctggaa ctgcctactg caggcagatg     360
   agatgccacg cactgccatt catcactagt ggcacttaca cctgcacaaa tggagtgctt     420
   cttgactctc gctgtgacta tagctgttcc agtggctacc acctggaagg tgatcgcagc     480
   cgaatctgca tggaagatgg gagatggagt ggaggcgagc ctgtatgtgt agacatagat     540
   ccccccaaga tccgctgtcc ccactcacgt gagaagatgg cagagccaga gaaattgact     600
   gctcgagtat actgggaccc gccattggtg aaagattctg ctgatggtac catcaccagg     660
   gtgacacttc ggggccctga gcctggctct cactttcctg aaggggagca cgtgattcgt     720
   tacactgcct atgaccgagc ctacaaccgg gccagctgca agttcattgt gaaagtacaa     780
   gtgagacgct gcccaactct gaaacctccg cagcacggct acctcacctg cacctcagca     840
   ggggacaact atggtgccac ctgtgaatac cactgtgatg gcggttatga tcgccagggg     900
   acaccctccc gggtctgtca atccagccgc cagtggtcag gttcaccacc aatctgtact     960
   cctatgaaga ttaacgtcaa tgtcaactca gctgctggtc tcctggatca attctatgag    1020
   aaacagcgac tcctcatcat ctcagctcct gatccctcca accgatatta taaaatgcag    1080
   atctctatgc tacagcaatc cacctgtgga ctggatttgc ggcatgtgac catcattgaa    1140
   ctggtgggac agccacctca ggaggtgggg cgcatccggg agcaacagct gtcagccaac    1200
   atcatcgagg agctcaggca atttcagcgc ctcactcgct cctacttcaa tatggtgttg    1260
   attgacaagc agggcatcga ccgagaccgc tacatggaac ctgtcacccc cgaggaaatc    1320
   ttcacattca ttgatgacta cctactgagc aatcaggagt tgacccagcg ccgggagcaa    1380
   agggacatat gcgagtga                                                   1398
<210> 7
   <211> 1398
   <212> DNA
   <213> Macaca mulatta
<400> 7
   atggccagtc aactaactca aagaggagct ctctctctgc tgttcttcct gactccagca      60
   gtgacaccaa catggtatgc aggttctggc tactatccag atgaaagcta caatgaagta     120
   tatgcagagg aggtcccacg ggctcctgcc ctggactacc gagtcccccg atggtgttat     180
   acattaaata tccaggatgg agaagccaca tgctactcac cgaggggagg aaattatcac     240
   agcagcctgg gcacacgttg tgagctctcc tgtgaccggg gctttcgact gattggaaga     300
   aggtcggtgc aatgcctgcc aagccgtcgc tggtctggaa ctgcctactg caggcagatg     360
   agatgccacg cactgccatt catcactagt ggcacttaca cctgcacaaa tggagtgctt     420
   cttgactctc gctgtgacta tagctgttcc agtggctacc acctggaagg tgatcgcagc     480
   cgaatctgca tggaagatgg gagatggagt ggaggcgagc ctgtatgtgt agacatagat     540
   ccccccaaga tccgctgtcc ccactcacgt gagaagatgg cagagccaga gaaattgact     600
   gctcgagtat actgggaccc gccattggtg aaagattctg ctgatggtac catcaccagg     660
   gtgacacttc ggggccctga gcctggctct cactttcctg aaggggagca cgtgattcgt     720
   tacactgcct atgaccgagc ctacaaccgg gccagctgca agttcattgt gaaagtacaa     780
   gtgagacgct gcccaactct gaaacctccg cagcacggct acctcacctg cacctcagca     840
   ggggacaact atggtgccac ctgtgaatac cactgtgatg gcggttatga tcgccagggg     900
   acaccctccc gggtctgtca atccagccgc cagtggtcag gttcaccacc aatctgtact     960
   cctatgaaga ttaacgtcaa tgtcaactca gctgctggtc tcctggatca attctatgag    1020
   aaacagcgac tcctcatcat ctcagctcct gatccctcca accgatatta taaaatgcag    1080
   atctctatgc tacagcaatc cacctgtgga ctggatttgc ggcatgtgac catcattgaa    1140
   ctggtgggac agccacctca ggaggtgggg cgcatccggg agcaacagct gtcagccaac    1200
   atcatcgagg agctcaggca atttcagcgc ctcactcgct cctacttcaa tatggtgttg    1260
   attgacaagc agggcatcga ccgagaccgc tacatggaac ctgtcacccc cgaggaaatc    1320
   ttcacattca ttgatgacta cctactgagc aatcaggagt tgacccagcg ccgggagcaa    1380
   agggacatat gcgagtga                                                   1398
<210> 8
   <211> 1398
   <212> DNA
   <213> Hylobates sp
<400> 8
   atggccagtc aactaactca aagaggagct ctctctctgc tgttctttct aactccggca      60
   gtgacaccaa catggtatgc aggttctggc tactatccgg atgaaagcta caatgaagtg     120
   tatgcagagg aagtcccaca ggctcctgcc ctggactacc gagtcccccg atggtgttat     180
   acattaaata tccaggatgg agaagccaca tgctactcac cgaggggagg aaattatcac     240
   agcagcctgg gcacgcgttg tgagctctcc tgtgaccggg gctttcgatt gattggaagg     300
   aggtcggtgc aatgcctgcc aagccgtcgt tggtctggaa ctgcctactg caggcagatg     360
   agatgccacg cactaccatt catcactagt ggcacttaca cctgcacaaa tggagtcctt     420
   cttgactctc gctgtgacta cagctgttcc agtggctacc acctggaagg tgatcgcagc     480
   cgaatctgca tggaagatgg gagatggagt ggaggcgagc ctgtatgtgt agacatagat     540
   ccccccaaga tccgctgtcc ccactcacgt gagaagatgg cagagccaga gaaattgact     600
   gctcgagtat actgggaccc accgttggtg aaagattctg ctgatggtac catcaccagg     660
   gtgacacttc ggggccctga gcctggctct cactttcccg aaggagagca tgtgattcgc     720
   tacactgcct atgaccgagc ctacaaccgg gccagctgca agttcattgt gaaagtacaa     780
   gtgagacgct gcccaactct gaaacctccg cagcatggct acctcacctg cacctcagcg     840
   ggggacaact atggtgccac ctgtgaatac cactgtgatg gcggttatga tcgccagggg     900
   acaccttccc gggtctgtca gtccagccgc cagtggtcag gttcaccacc aatctgtact     960
   cctatgaaga ttaacgtcaa tgtcaactca gctgctggtc tcttggatca attctatgag    1020
   aaacagcgac tcctcatcat ctcagctcct gatccttcca accgatatta taaaatgcag    1080
   atctctatgc tacagcaatc cacttgtgga ctggatttgc ggcatgtgac catcattgaa    1140
   ctggtgggac agccacctca ggaggtgggg cgcatccggg agcaacagct gtcagccaac    1200
   atcatcgagg agctcaggca atttcagcgc ctcactcgct cctacttcaa catggtgttg    1260
   attgacaagc agggtattga ccgagaccgc tacatggaac ctgtcacccc tgaggaaatc    1320
   ttcacattca ttgatgacta cctactgagc aatcaggagc tgacccagcg tcgggagcaa    1380
   agggacatat gcgagtga                                                   1398
<210> 9
   <211> 1398
   <212> DNA
   <213> Pongo pygmaeus
<400> 9
   atggccagtc aactaactca aagaggagct ttctctctgc tgttctttct aactccggca      60
   gtgacaccaa cgtggtatgc aggttctggc tactatccgg atgaaagcta caatgaagta     120
   tatgcagagg aggtcccaca ggctcctgcc ctggactacc gagtcccccg atggtgttac     180
   acattaaata tccaggatgg agaagccaca tgctactcac cgaggggagg aaattatcac     240
   agcagcctgg gcacgcgttg tgagctctcc tgtgaccggg gctttcgatt gattggaagg     300
   aggtcggtgc aatgcctgcc aagtcgtcgt tggtctggaa ctgcctactg taggcagatg     360
   agatgccacg cactaccatt catcactagt ggcacttaca cctgcacaaa tggagtgctt     420
   cttgactctc gctgtgacta cagctgttcc agtggctacc acctggaagg tgatcgcagc     480
   cgaatctgca tggaagatgg gagatggagt ggaggcgagc ctgtatgtgt agacatagat     540
   ccccccaaga tccgctgtcc ccactcacgt gagaagatgg cagagccaga gaaattgact     600
   gctcgagtat actgggaccc accgttggtg aaagattctg ctgatggtac catcaccagg     660
   gtgacacttc ggggccctga gcctggctct cactttcccg aaggagagca tgtgattcgt     720
   tacactgcct atgaccgagc ctacaaccgg gccagctgca agttcattgt gaaagtacaa     780
   gtgagacgct gcccaactct gaaacctccg cagcacggct acctcacctg cacctcagcg     840
   ggggacaact atggtgccac ctgtgaatac cactgtgatg gcggttatga tcgccagggg     900
   acaccctccc gggtctgtca gtccagccgc cagtggtcag gttcaccacc aatctgtgct     960
   cctatgaaga ttaacgtcaa cgtcaactca gctgctggtc tcttggatca attctatgag    1020
   aaacagcgac tcctcatcat ctcagctcct gatccttcca accgatatta taaaatgcag    1080
   atctctatgc tacagcaatc cacctgtgga ctggatttgc ggcatgtgac catcattgaa    1140
   ctggtaggac agccacctca ggaggtgggg cgcatccggg agcaacagct gtcagccaac    1200
   atcatcgagg agctcaggca atttcagcgc ctcactcgct cctacttcaa catggtgttg    1260
   attgacaagc agggtattga ccgagaccgc tacatggaac ctgtcacccc cgaggaaatc    1320
   ttcacattca ttgatgacta cctactgagc aatcaggagt tgacgcagcg tcgggagcaa    1380
   agggacatat gcgagtga                                                   1398
<210> 10
   <211> 1654
   <212> DNA
   <213> Xenopus laevis
<400> 10
   gcaagacaag ctctacacac tattctaccc cagatgtgtt ggcttttctt taaataatct      60
   gcgtgcatgg attgatgcta caaactatcc agtgtgtaca gttcagctgc agaatccatt     120
   tcccctctgt aggtgctgca ggctgcctgc ctggaagctt ctgagaattc ctggaactga     180
   aagctcttct tgcaatggaa gcatcaataa cagtcttatt atttgctttc accaaagtag     240
   cttcatctct gtattatgaa ggttcaggac acagtgatgg tgagatccaa accaatgagg     300
   tttatgttga atcccgccct ttgggacctt acagagctcc tcgttggtgc tacgatctac     360
   atatcagtga tggggaagca acttgctatt cgccacttgg cccaaggtat cgtagcactc     420
   taggaacccg atgccgtctc tcgtgtgacc aaggtttcaa gctcattgga caaagctcag     480
   tgcaatgttt atcaagccgg cgctggtctg ggaatggcca ttgtaggcga atccagtgtc     540
   atgttctgcc ccctatattt tatggatcat atcactgctc agtaggtgtt tctgagggct     600
   ctcgttgtga ttattcctgt gcccccggct atatggtgga aggagaccgg agtcggatct     660
   gcatggaaga tggacagtgg agcggaggag aacctgtctg tgtagatctg gacccaccca     720
   aaattcagtg ccctgtgtcc cgcatgaaag tagcagagcc agagaaattg acagcaagaa     780
   tattctgggg gaacccacaa gtgaaagatt cagctgatgg tgttatcaca cgagtatttc     840
   tccgaggccc agaacctgga tctgagcttc cagagggaga gcatgtcatt cgttacacag     900
   cttatgacag ggcccacaac agggcaagct gtaaatttat agtgaaagta caagtgaggc     960
   gatgcccaga tcttacgcca cctcttcatg gttacctcac ctgtagcgct gcaggcaata    1020
   actatggagc aacctgtgag tatcactgcg agggagggta tgaacgccaa ggccctgctg    1080
   ccagagtctg ccagttcagc cagaactggg ctgggacgcc atctacctgc acacccatgc    1140
   tgataaacgt taatgtgaat tctgccgggg cattcattga tcaattcttt gagaagcaac    1200
   gtcttttatt tatctccagc cctagttctt ctgaccgcta ctatagaatg caaactacag    1260
   ctctgcagag ttcaggctgt ggtttggaac agcgccatgt attaattgtt gagttaattg    1320
   gagaatctcc cagagaagta ggacgagtgc gcaatcagca gctgtccaag gaactaatag    1380
   aagaactgag acaggttctc cgcatttccc gttcatactt taacatggtt ctcattgaca    1440
   agcacggggt tgatagagac cgctataggc agcccacagc atctgaagat atcttccttt    1500
   tcattgatac atatttattg agccctcgag agctctccca ggtggaatca aataaagaaa    1560
   actgcgattg atgaagagaa aaaaaaaaaa aaaaaaaaaa aaaaaaaaaa aaaaaaaaaa    1620
   aaaaaaaaaa aaaaaaaaaa aaaaaaaaaa aaaa                                1654
<210> 11
   <211> 25
   <212> RNA
   <213> Artificial Sequence
<220>
   <223> Synthetic primer
<400> 11
   ccugaucccu ccaaucggua cuaca 25
<210> 12
   <211> 25
   <212> RNA
   <213> Artificial Sequence
<220>
   <223> Synthetic primer
<400> 12
   aggcgagccu guauguguag acaua 25
<210> 13
   <211> 25
   <212> RNA
   <213> Artificial Sequence
<220>
   <223> Synthetic primer
<400> 13
   gggcuuucga uugauuggac ggaag 25
<210> 14
   <211> 465
   <212> PRT
   <213> Homo sapiens
<400> 14
<210> 15
   <211> 467
   <212> PRT
   <213> Mus musculus
<400> 15
   Met Thr Ser Pro Leu Thr Gln Arg Gly Ala Leu Ser Leu Leu Leu Leu
   1               5                   10                  15
   Leu Met Pro Ala Val Thr Pro Thr Trp Tyr Ala Gly Ser Gly Tyr Ser
               20                  25                  30
   Pro Asp Glu Ser Tyr Asn Glu Val Tyr Ala Glu Glu Val Pro Ala Ala
           35                  40                  45
   Arg Ala Arg Ala Leu Asp Tyr Arg Val Pro Arg Trp Cys Tyr Thr Leu
       50                  55                  60
   Asn Ile Gln Asp Gly Glu Ala Thr Cys Tyr Ser Pro Arg Gly Gly Asn
   65                  70                  75                  80
   Tyr His Ser Ser Leu Gly Thr Arg Cys Glu Leu Ser Cys Asp Arg Gly
                   85                  90                  95
   Phe Arg Leu Ile Gly Arg Lys Ser Val Gln Cys Leu Pro Ser Arg Arg
               100                 105                 110
   Trp Ser Gly Thr Ala Tyr Cys Arg Gln Ile Arg Cys His Thr Leu Pro
           115                 120                 125
   Phe Ile Thr Ser Gly Thr Tyr Thr Cys Thr Asn Gly Met Leu Leu Asp
       130                 135                 140
   Ser Arg Cys Asp Tyr Ser Cys Ser Ser Gly Tyr His Leu Glu Gly Asp
   145                 150                 155                 160
   Arg Ser Arg Ile Cys Met Glu Asp Gly Arg Trp Ser Gly Gly Glu Pro
                   165                 170                 175
   Val Cys Val Asp Ile Asp Pro Pro Lys Ile Arg Cys Pro His Ser Arg
               180                 185                 190
   Glu Lys Met Ala Glu Pro Glu Lys Leu Thr Ala Arg Val Tyr Trp Asp
           195                 200                 205
   Pro Pro Leu Val Lys Asp Ser Ala Asp Gly Thr Ile Thr Arg Val Thr
       210                 215                 220
   Leu Arg Gly Pro Glu Pro Gly Ser His Phe Pro Glu Gly Glu His Val
   225                 230                 235                 240
   Ile Arg Tyr Thr Ala Tyr Asp Arg Ala Tyr Asn Arg Ala Ser Cys Lys
                   245                 250                 255
   Phe Ile Val Lys Val Gln Val Arg Arg Cys Pro Ile Leu Lys Pro Pro
               260                 265                 270
   Gln His Gly Tyr Leu Thr Cys Ser Ser Ala Gly Asp Asn Tyr Gly Ala
           275                 280                 285
   Ile Cys Glu Tyr His Cys Asp Gly Gly Tyr Glu Arg Gln Gly Thr Pro
       290                 295                 300
   Ser Arg Val Cys Gln Ser Ser Arg Gln Trp Ser Gly Thr Pro Pro Val
   305                 310                 315                 320
   Cys Thr Pro Met Lys Ile Asn Val Asn Val Asn Ser Ala Ala Gly Leu
                   325                 330                 335
   Leu Asp Gln Phe Tyr Glu Lys Gln Arg Leu Leu Ile Val Ser Ala Pro
               340                 345                 350
   Asp Pro Ser Asn Arg Tyr Tyr Lys Met Gln Ile Ser Met Leu Gln Gln
           355                 360                 365
   Ser Thr Cys Gly Leu Asp Leu Arg His Val Thr Ile Ile Glu Leu Val
       370                 375                 380
   Gly Gln Pro Pro Gln Glu Val Gly Arg Ile Arg Glu Gln Gln Leu Ser
   385                 390                 395                 400
   Ala Gly Ile Ile Glu Glu Leu Arg Gln Phe Gln Arg Leu Thr Arg Ser
                   405                 410                 415
   Tyr Phe Asn Met Val Leu Ile Asp Lys Gln Gly Ile Asp Arg Glu Arg
               420                 425                 430
   Tyr Met Glu Pro Val Thr Pro Glu Glu Ile Phe Thr Phe Ile Asp Asp
           435                 440                 445
   Tyr Leu Leu Ser Asn Glu Glu Leu Ala Arg Arg Val Glu Gln Arg Asp
       450                 455                 460
   Leu Cys Glu
   465
<210> 16
   <211> 465
   <212> PRT
   <213> Bos taurus
<400> 16
   Met Ala Ile Gln Leu Thr Arg Arg Gly Ala Leu Ser Leu Leu Leu Phe
   1               5                   10                  15
   Leu Thr Pro Ala Val Met Pro Thr Trp Tyr Ala Gly Ser Gly Tyr Tyr
               20                  25                  30
   Pro Asp Glu Ser Tyr Asn Glu Val Tyr Ala Glu Glu Val Pro Gln Thr
           35                  40                  45
   Pro Ile Leu Asp Tyr Lys Val Pro Arg Trp Cys Tyr Thr Leu Asn Ile
       50                  55                  60
   Gln Asp Gly Glu Ala Thr Cys Tyr Ser Pro Arg Gly Gly Asn Tyr His
   65                  70                  75                  80
   Ser Ser Leu Gly Thr Arg Cys Glu Leu Ser Cys Asp Arg Gly Phe Arg
                   85                  90                  95
   Leu Ile Gly Arg Arg Ser Val Gln Cys Leu Pro Ser Arg Arg Trp Ser
               100                 105                 110
   Gly Thr Ala Tyr Cys Arg Gln Met Arg Cys His Ala Leu Pro Phe Ile
           115                 120                 125
   Thr Ser Gly Thr Tyr Thr Cys Thr Asn Gly Val Leu Leu Asp Ser Arg
       130                 135                 140
   Cys Asp Tyr Ser Cys Ser Ser Gly Tyr His Leu Glu Gly Asp Arg Ser
   145                 150                 155                 160
   Arg Ile Cys Met Glu Asp Gly Arg Trp Ser Gly Gly Glu Pro Val Cys
                   165                 170                 175
   Val Asp Ile Asp Pro Pro Lys Ile Arg Cys Pro His Ser Arg Glu Lys
               180                 185                 190
   Met Ala Glu Pro Glu Lys Leu Thr Ala Arg Val Tyr Trp Asp Pro Pro
           195                 200                 205
   Val Val Lys Asp Ser Ala Asp Gly Thr Ile Thr Arg Leu Thr Leu Arg
       210                 215                 220
   Gly Pro Glu Pro Gly Ser His Phe Pro Glu Gly Glu His Val Ile Arg
   225                 230                 235                 240
   Tyr Thr Ala Tyr Asp Arg Ala Tyr Asn Arg Ala Ser Cys Lys Phe Ile
                   245                 250                 255
   Val Lys Val Gln Val Arg Arg Cys Pro Thr Leu Lys Pro Pro Leu His
               260                 265                 270
   Gly Tyr Leu Thr Cys Thr Ser Ala Gly Asp Asn Tyr Gly Ala Thr Cys
           275                 280                 285
   Glu Tyr His Cys Asp Gly Gly Tyr Glu Arg Gln Gly Thr Ser Ser Arg
       290                 295                 300
   Val Cys Gln Ser Ser Arg Gln Trp Ser Gly Ser Pro Pro Val Cys Val
   305                 310                 315                 320
   Pro Met Lys Ile Asn Val Asn Val Asn Ser Ala Ala Gly Leu Leu Asp
                   325                 330                 335
   Gln Phe Tyr Glu Lys Arg Arg Leu Leu Ile Ile Ser Ala Pro Asp Pro
               340                 345                 350
   Ser Asn Arg Tyr Tyr Lys Met Gln Ile Ser Met Leu Gln Gln Ser Thr
           355                 360                 365
   Cys Gly Leu Asp Leu Arg His Val Thr Ile Ile Glu Leu Val Gly Gln
       370                 375                 380
   Pro Pro Gln Glu Val Gly Arg Ile Arg Glu His Gln Leu Ser Ala Asn
   385                 390                 395                 400
   Ile Ile Glu Glu Leu Arg Gln Phe Gln His Leu Thr Arg Ser Tyr Phe
                   405                 410                 415
   Asn Met Val Leu Ile Asp Lys Gln Gly Ile Asp Arg Glu Arg Tyr Met
               420                 425                 430
   Glu Pro Val Thr Pro Glu Glu Ile Phe Thr Phe Ile Asp Asp Tyr Leu
           435                 440                 445
   Leu Ser Asn Glu Glu Leu Ile Gln Arg Arg Glu Gln Arg Asp Ile Cys
       450                 455                 460
   Asp
   465
<210> 17
   <211> 465
   <212> PRT
   <213> Pan troglodytes
<400> 17
   Met Ala Ser Gln Leu Thr Gln Arg Gly Ala Leu Phe Leu Leu Phe Phe
   1               5                   10                  15
   Leu Thr Pro Ala Val Thr Pro Thr Trp Tyr Ala Gly Ser Gly Tyr Tyr
               20                  25                  30
   Pro Asp Glu Ser Tyr Asn Glu Val Tyr Ala Glu Glu Val Pro Gln Ala
           35                  40                  45
   Pro Ala Leu Asp Tyr Arg Val Pro Arg Trp Cys Tyr Thr Leu Asn Ile
       50                  55                  60
   Gln Asp Gly Glu Ala Thr Cys Tyr Ser Pro Arg Gly Gly Asn Tyr His
   65                  70                  75                  80
   Ser Ser Leu Gly Thr Arg Cys Glu Leu Ser Cys Asp Arg Gly Phe Arg
                   85                  90                  95
   Leu Ile Gly Arg Arg Ser Val Gln Cys Leu Pro Ser Arg Arg Trp Ser
               100                 105                 110
   Gly Thr Ala Tyr Cys Arg Gln Met Arg Cys His Ala Leu Pro Phe Ile
           115                 120                 125
   Thr Ser Gly Thr Tyr Thr Cys Thr Asn Gly Val Leu Leu Asp Ser Arg
       130                 135                 140
   Cys Asp Tyr Ser Cys Ser Ser Gly Tyr His Leu Glu Gly Asp Arg Ser
   145                 150                 155                 160
   Arg Ile Cys Met Glu Asp Gly Arg Trp Ser Gly Gly Glu Pro Val Cys
                   165                 170                 175
   Val Asp Ile Asp Pro Pro Lys Ile Arg Cys Pro His Ser Arg Glu Lys
               180                 185                 190
   Met Ala Glu Pro Glu Lys Leu Thr Ala Arg Val Tyr Trp Asp Pro Pro
           195                 200                 205
   Leu Val Lys Asp Ser Ala Asp Gly Thr Ile Thr Arg Val Thr Leu Arg
       210                 215                 220
   Gly Pro Glu Pro Gly Ser His Phe Pro Glu Gly Glu His Val Ile Arg
   225                 230                 235                 240
   Tyr Thr Ala Tyr Asp Arg Ala Tyr Asn Arg Ala Ser Cys Lys Phe Ile
                   245                 250                 255
   Val Lys Val Gln Val Arg Arg Cys Pro Thr Leu Lys Pro Pro Gln His
                   260                 265                 270
   Gly Tyr Leu Thr Cys Thr Ser Ala Gly Asp Asn Tyr Gly Ala Thr Cys
           275                 280                 285
   Glu Tyr His Cys Asp Gly Gly Tyr Asp Arg Gln Gly Thr Pro Ser Arg
       290                 295                 300
   Val Cys Gln Ser Ser Arg Gln Trp Ser Gly Ser Pro Pro Ile Cys Ala
   305                 310                 315                 320
   Pro Met Lys Ile Asn Val Asn Val Asn Ser Ala Ala Gly Leu Leu Asp
                   325                 330                 335
   Gln Phe Tyr Glu Lys Gln Arg Leu Leu Ile Ile Ser Ala Pro Asp Pro
               340                 345                 350
   Ser Asn Arg Tyr Tyr Lys Met Gln Ile Ser Met Leu Gln Gln Ser Thr
           355                 360                 365
   Cys Gly Leu Asp Leu Arg His Val Thr Ile Ile Glu Leu Val Gly Gln
       370                 375                 380
   Pro Pro Gln Glu Val Gly Arg Ile Arg Glu Gln Gln Leu Ser Ala Asn
   385                 390                 395                 400
   Ile Ile Glu Glu Leu Arg Gln Phe Gln Arg Leu Thr Arg Ser Tyr Phe
                   405                 410                 415
   Asn Met Val Leu Ile Asp Lys Gln Gly Ile Asp Arg Asp Arg Tyr Met
               420                 425                 430
   Glu Pro Val Thr Pro Glu Glu Ile Phe Thr Phe Ile Asp Asp Tyr Leu
           435                 440                 445
   Leu Ser Asn Gln Glu Leu Thr Gln Arg Arg Glu Gln Arg Asp Ile Cys
       450                 455                 460
   Glu
   465
<210> 18
   <211> 465
   <212> PRT
   <213> Gorilla gorilla
<400> 18
   Met Ala Ser Gln Leu Thr Gln Arg Gly Ala Leu Ser Leu Leu Phe Phe
   1               5                   10                  15
   Leu Thr Pro Ala Val Thr Pro Thr Trp Tyr Ala Gly Ser Gly Tyr Tyr
               20                  25                  30
   Pro Asp Glu Ser Tyr Asn Glu Val Tyr Ala Glu Glu Val Pro Gln Ala
           35                  40                  45
   Pro Ala Leu Asp Tyr Arg Val Pro Arg Trp Cys Tyr Thr Leu Asn Ile
       50                  55                  60
   Gln Asp Gly Glu Ala Thr Cys Tyr Ser Pro Arg Gly Gly Asn Tyr His
   65                  70                  75                  80
   Ser Ser Leu Gly Thr Arg Cys Glu Leu Ser Cys Asp Arg Gly Phe Arg
                   85                  90                  95
   Leu Ile Gly Arg Arg Ser Val Gln Cys Leu Pro Ser Arg Arg Trp Ser
               100                 105                 110
   Gly Thr Ala Tyr Cys Arg Gln Met Arg Cys His Ala Leu Pro Phe Ile
           115                 120                 125
   Thr Ser Gly Thr Tyr Thr Cys Thr Asn Gly Val Leu Leu Asp Ser Arg
       130                 135                 140
   Cys Asp Tyr Ser Cys Ser Ser Gly Tyr His Leu Glu Gly Asp Arg Ser
   145                 150                 155                 160
   Arg Ile Cys Met Glu Asp Gly Arg Trp Ser Gly Gly Glu Pro Val Cys
                   165                 170                 175
   Val Asp Ile Asp Pro Pro Lys Ile Arg Cys Pro His Ser Arg Glu Lys
               180                 185                 190
   Met Ala Glu Pro Glu Lys Leu Thr Ala Arg Val Tyr Trp Asp Pro Pro
           195                 200                 205
   Leu Val Lys Asp Ser Ala Asp Gly Thr Ile Thr Arg Val Thr Leu Arg
       210                 215                 220
   Gly Pro Glu Pro Gly Ser His Phe Pro Glu Gly Glu His Val Ile Arg
   225                 230                 235                 240
   Tyr Thr Ala Tyr Asp Arg Ala Tyr Asn Arg Ala Ser Cys Lys Phe Ile
                   245                 250                 255
   Val Lys Val Gln Val Arg Arg Cys Pro Thr Leu Lys Pro Pro Gln His
               260                 265                 270
   Gly Tyr Leu Thr Cys Thr Ser Ala Gly Asp Asn Tyr Gly Ala Thr Cys
           275                 280                 285
   Glu Tyr His Cys Asp Gly Gly Tyr Asp Arg Gln Gly Thr Pro Ser Arg
       290                 295                 300
   Val Cys Gln Ser Ser Arg Gln Trp Ser Gly Ser Pro Pro Ile Cys Ala
   305                 310                 315                 320
   Pro Met Lys Ile Asn Val Asn Val Asn Ser Ala Ala Gly Leu Leu Asp
                   325                 330                 335
   Gln Phe Tyr Glu Lys Gln Arg Leu Leu Ile Ile Ser Ala Pro Asp Pro
               340                 345                 350
   Ser Asn Arg Tyr Tyr Lys Met Gln Ile Ser Met Leu Gln Gln Ser Thr
           355                 360                 365
   Cys Gly Leu Asp Leu Arg His Val Thr Ile Ile Glu Leu Val Gly Gln
       370                 375                 380
   Pro Pro Gln Glu Val Gly Arg Ile Arg Glu Gln Gln Leu Ser Ala Asn
   385                 390                 395                 400
   Ile Ile Glu Glu Leu Arg Gln Phe Gln Arg Leu Thr Arg Ser Tyr Phe
                   405                 410                 415
   Asn Met Val Leu Ile Asp Lys Gln Gly Ile Asp Arg Asp Arg Tyr Met
               420                 425                 430
   Glu Pro Val Thr Pro Glu Glu Ile Phe Thr Phe Ile Asp Asp Tyr Leu
           435                 440                 445
   Leu Ser Asn Gln Glu Leu Thr Gln Arg Arg Glu Gln Arg Asp Ile Cys
       450                 455                 460
   Glu
   465
<210> 19
   <211> 465
   <212> PRT
   <213> Papio sp
<400> 19
   Met Ala Ser Gln Leu Thr Gln Arg Gly Ala Leu Ser Leu Leu Phe Phe
   1               5                   10                  15
   Leu Thr Pro Ala Val Thr Pro Thr Trp Tyr Ala Gly Ser Gly Tyr Tyr
               20                  25                  30
   Pro Asp Glu Ser Tyr Asn Glu Val Tyr Ala Glu Glu Val Pro Arg Ala
           35                  40                  45
   Pro Ala Leu Asp Tyr Arg Val Pro Arg Trp Cys Tyr Thr Leu Asn Ile
       50                  55                  60
   Gln Asp Gly Glu Ala Thr Cys Tyr Ser Pro Arg Gly Gly Asn Tyr His
   65                  70                  75                  80
   Ser Ser Leu Gly Thr Arg Cys Glu Leu Ser Cys Asp Arg Gly Phe Arg
                   85                  90                  95
   Leu Ile Gly Arg Arg Ser Val Gln Cys Leu Pro Ser Arg Arg Trp Ser
               100                 105                 110
   Gly Thr Ala Tyr Cys Arg Gln Met Arg Cys His Ala Leu Pro Phe Ile
           115                 120                 125
   Thr Ser Gly Thr Tyr Thr Cys Thr Asn Gly Val Leu Leu Asp Ser Arg
       130                 135                 140
   Cys Asp Tyr Ser Cys Ser Ser Gly Tyr His Leu Glu Gly Asp Arg Ser
   145                 150                 155                 160
   Arg Ile Cys Met Glu Asp Gly Arg Trp Ser Gly Gly Glu Pro Val Cys
                   165                 170                 175
   Val Asp Ile Asp Pro Pro Lys Ile Arg Cys Pro His Ser Arg Glu Lys
               180                 185                 190
   Met Ala Glu Pro Glu Lys Leu Thr Ala Arg Val Tyr Trp Asp Pro Pro
           195                 200                 205
   Leu Val Lys Asp Ser Ala Asp Gly Thr Ile Thr Arg Val Thr Leu Arg
       210                 215                 220
   Gly Pro Glu Pro Gly Ser His Phe Pro Glu Gly Glu His Val Ile Arg
   225                 230                 235                 240
   Tyr Thr Ala Tyr Asp Arg Ala Tyr Asn Arg Ala Ser Cys Lys Phe Ile
                   245                 250                 255
   Val Lys Val Gln Val Arg Arg Cys Pro Thr Leu Lys Pro Pro Gln His
               260                 265                 270
   Gly Tyr Leu Thr Cys Thr Ser Ala Gly Asp Asn Tyr Gly Ala Thr Cys
           275                 280                 285
   Glu Tyr His Cys Asp Gly Gly Tyr Asp Arg Gln Gly Thr Pro Ser Arg
       290                 295                 300
   Val Cys Gln Ser Ser Arg Gln Trp Ser Gly Ser Pro Pro Ile Cys Thr
   305                 310                 315                 320
   Pro Met Lys Ile Asn Val Asn Val Asn Ser Ala Ala Gly Leu Leu Asp
                   325                 330                 335
   Gln Phe Tyr Glu Lys Gln Arg Leu Leu Ile Ile Ser Ala Pro Asp Pro
               340                 345                 350
   Ser Asn Arg Tyr Tyr Lys Met Gln Ile Ser Met Leu Gln Gln Ser Thr
           355                 360                 365
   Cys Gly Leu Asp Leu Arg His Val Thr Ile Ile Glu Leu Val Gly Gln
       370                 375                 380
   Pro Pro Gln Glu Val Gly Arg Ile Arg Glu Gln Gln Leu Ser Ala Asn
   385                 390                 395                 400
   Ile Ile Glu Glu Leu Arg Gln Phe Gln Arg Leu Thr Arg Ser Tyr Phe
                   405                 410                 415
   Asn Met Val Leu Ile Asp Lys Gln Gly Ile Asp Arg Asp Arg Tyr Met
               420                 425                 430
   Glu Pro Val Thr Pro Glu Glu Ile Phe Thr Phe Ile Asp Asp Tyr Leu
           435                 440                 445
   Leu Ser Asn Gln Glu Leu Thr Gln Arg Arg Glu Gln Arg Asp Ile Cys
       450                 455                 460
   Glu
   465
<210> 20
   <211> 465
   <212> PRT
   <213> Macaca mulatta
<400> 20
   Met Ala Ser Gln Leu Thr Gln Arg Gly Ala Leu Ser Leu Leu Phe Phe
   1               5                   10                  15
   Leu Thr Pro Ala Val Thr Pro Thr Trp Tyr Ala Gly Ser Gly Tyr Tyr
               20                  25                  30
   Pro Asp Glu Ser Tyr Asn Glu Val Tyr Ala Glu Glu Val Pro Arg Ala
           35                  40                  45
   Pro Ala Leu Asp Tyr Arg Val Pro Arg Trp Cys Tyr Thr Leu Asn Ile
       50                  55                  60
   Gln Asp Gly Glu Ala Thr Cys Tyr Ser Pro Arg Gly Gly Asn Tyr His
   65                  70                  75                  80
   Ser Ser Leu Gly Thr Arg Cys Glu Leu Ser Cys Asp Arg Gly Phe Arg
                   85                  90                  95
   Leu Ile Gly Arg Arg Ser Val Gln Cys Leu Pro Ser Arg Arg Trp Ser
               100                 105                 110
   Gly Thr Ala Tyr Cys Arg Gln Met Arg Cys His Ala Leu Pro Phe Ile
           115                 120                 125
   Thr Ser Gly Thr Tyr Thr Cys Thr Asn Gly Val Leu Leu Asp Ser Arg
       130                 135                 140
   Cys Asp Tyr Ser Cys Ser Ser Gly Tyr His Leu Glu Gly Asp Arg Ser
   145                 150                 155                 160
   Arg Ile Cys Met Glu Asp Gly Arg Trp Ser Gly Gly Glu Pro Val Cys
                   165                 170                 175
   Val Asp Ile Asp Pro Pro Lys Ile Arg Cys Pro His Ser Arg Glu Lys
               180                 185                 190
   Met Ala Glu Pro Glu Lys Leu Thr Ala Arg Val Tyr Trp Asp Pro Pro
           195                 200                 205
   Leu Val Lys Asp Ser Ala Asp Gly Thr Ile Thr Arg Val Thr Leu Arg
       210                 215                 220
   Gly Pro Glu Pro Gly Ser His Phe Pro Glu Gly Glu His Val Ile Arg
   225                 230                 235                 240
   Tyr Thr Ala Tyr Asp Arg Ala Tyr Asn Arg Ala Ser Cys Lys Phe Ile
                   245                 250                 255
   Val Lys Val Gln Val Arg Arg Cys Pro Thr Leu Lys Pro Pro Gln His
               260                 265                 270
   Gly Tyr Leu Thr Cys Thr Ser Ala Gly Asp Asn Tyr Gly Ala Thr Cys
           275                 280                 285
   Glu Tyr His Cys Asp Gly Gly Tyr Asp Arg Gln Gly Thr Pro Ser Arg
       290                 295                 300
   Val Cys Gln Ser Ser Arg Gln Trp Ser Gly Ser Pro Pro Ile Cys Thr
   305                 310                 315                 320
   Pro Met Lys Ile Asn Val Asn Val Asn Ser Ala Ala Gly Leu Leu Asp
                   325                 330                 335
   Gln Phe Tyr Glu Lys Gln Arg Leu Leu Ile Ile Ser Ala Pro Asp Pro
               340                 345                 350
   Ser Asn Arg Tyr Tyr Lys Met Gln Ile Ser Met Leu Gln Gln Ser Thr
           355                 360                 365
   Cys Gly Leu Asp Leu Arg His Val Thr Ile Ile Glu Leu Val Gly Gln
       370                 375                 380
   Pro Pro Gln Glu Val Gly Arg Ile Arg Glu Gln Gln Leu Ser Ala Asn
   385                 390                 395                 400
   Ile Ile Glu Glu Leu Arg Gln Phe Gln Arg Leu Thr Arg Ser Tyr Phe
                   405                 410                 415
   Asn Met Val Leu Ile Asp Lys Gln Gly Ile Asp Arg Asp Arg Tyr Met
               420                 425                 430
   Glu Pro Val Thr Pro Glu Glu Ile Phe Thr Phe Ile Asp Asp Tyr Leu
           435                 440                 445
   Leu Ser Asn Gln Glu Leu Thr Gln Arg Arg Glu Gln Arg Asp Ile Cys
       450             455             460
   Glu
   465
<210> 21
   <211> 465
   <212> PRT
   <213> Hylobates sp
<400> 21
   Met Ala Ser Gln Leu Thr Gln Arg Gly Ala Leu Ser Leu Leu Phe Phe
   1               5                   10                  15
   Leu Thr Pro Ala Val Thr Pro Thr Trp Tyr Ala Gly Ser Gly Tyr Tyr
               20                  25                  30
   Pro Asp Glu Ser Tyr Asn Glu Val Tyr Ala Glu Glu Val Pro Gln Ala
           35                  40                  45
   Pro Ala Leu Asp Tyr Arg Val Pro Arg Trp Cys Tyr Thr Leu Asn Ile
       50                  55                  60
   Gln Asp Gly Glu Ala Thr Cys Tyr Ser Pro Arg Gly Gly Asn Tyr His
   65                  70                  75                  80
   Ser Ser Leu Gly Thr Arg Cys Glu Leu Ser Cys Asp Arg Gly Phe Arg
                   85                  90                  95
   Leu Ile Gly Arg Arg Ser Val Gln Cys Leu Pro Ser Arg Arg Trp Ser
               100                 105                 110
   Gly Thr Ala Tyr Cys Arg Gln Met Arg Cys His Ala Leu Pro Phe Ile
           115                 120                 125
   Thr Ser Gly Thr Tyr Thr Cys Thr Asn Gly Val Leu Leu Asp Ser Arg
       130                 135                 140
   Cys Asp Tyr Ser Cys Ser Ser Gly Tyr His Leu Glu Gly Asp Arg Ser
   145                 150                 155                 160
   Arg Ile Cys Met Glu Asp Gly Arg Trp Ser Gly Gly Glu Pro Val Cys
                   165                 170                 175
   Val Asp Ile Asp Pro Pro Lys Ile Arg Cys Pro His Ser Arg Glu Lys
               180                 185                 190
   Met Ala Glu Pro Glu Lys Leu Thr Ala Arg Val Tyr Trp Asp Pro Pro
           195                 200                 205
   Leu Val Lys Asp Ser Ala Asp Gly Thr Ile Thr Arg Val Thr Leu Arg
       210                 215                 220
   Gly Pro Glu Pro Gly Ser His Phe Pro Glu Gly Glu His Val Ile Arg
   225                 230                 235                 240
   Tyr Thr Ala Tyr Asp Arg Ala Tyr Asn Arg Ala Ser Cys Lys Phe Ile
                   245                 250                 255
   Val Lys Val Gln Val Arg Arg Cys Pro Thr Leu Lys Pro Pro Gln His
               260                 265                 270
   Gly Tyr Leu Thr Cys Thr Ser Ala Gly Asp Asn Tyr Gly Ala Thr Cys
           275                 280                 285
   Glu Tyr His Cys Asp Gly Gly Tyr Asp Arg Gln Gly Thr Pro Ser Arg
       290                 295                 300
   Val Cys Gln Ser Ser Arg Gln Trp Ser Gly Ser Pro Pro Ile Cys Thr
   305                 310                 315                 320
   Pro Met Lys Ile Asn Val Asn Val Asn Ser Ala Ala Gly Leu Leu Asp
                   325                 330                 335
   Gln Phe Tyr Glu Lys Gln Arg Leu Leu Ile Ile Ser Ala Pro Asp Pro
               340                 345                 350
   Ser Asn Arg Tyr Tyr Lys Met Gln Ile Ser Met Leu Gln Gln Ser Thr
           355                 360                 365
   Cys Gly Leu Asp Leu Arg His Val Thr Ile Ile Glu Leu Val Gly Gln
       370                 375                 380
   Pro Pro Gln Glu Val Gly Arg Ile Arg Glu Gln Gln Leu Ser Ala Asn
   385                 390                 395                 400
   Ile Ile Glu Glu Leu Arg Gln Phe Gln Arg Leu Thr Arg Ser Tyr Phe
                   405                 410                 415
   Asn Met Val Leu Ile Asp Lys Gln Gly Ile Asp Arg Asp Arg Tyr Met
               420                 425                 430
   Glu Pro Val Thr Pro Glu Glu Ile Phe Thr Phe Ile Asp Asp Tyr Leu
           435                 440                 445
   Leu Ser Asn Gln Glu Leu Thr Gln Arg Arg Glu Gln Arg Asp Ile Cys
       450                 455                 460
   Glu
   465
<210> 22
   <211> 465
   <212> PRT
   <213> Pongo pygmaeus
<400> 22
   Met Ala Ser Gln Leu Thr Gln Arg Gly Ala Phe Ser Leu Leu Phe Phe
   1               5                   10                  15
   Leu Thr Pro Ala Val Thr Pro Thr Trp Tyr Ala Gly Ser Gly Tyr Tyr
               20                  25                  30
   Pro Asp Glu Ser Tyr Asn Glu Val Tyr Ala Glu Glu Val Pro Gln Ala
           35                  40                  45
   Pro Ala Leu Asp Tyr Arg Val Pro Arg Trp Cys Tyr Thr Leu Asn Ile
       50                  55                  60
   Gln Asp Gly Glu Ala Thr Cys Tyr Ser Pro Arg Gly Gly Asn Tyr His
   65                  70                  75                  80
   Ser Ser Leu Gly Thr Arg Cys Glu Leu Ser Cys Asp Arg Gly Phe Arg
                   85                  90                  95
   Leu Ile Gly Arg Arg Ser Val Gln Cys Leu Pro Ser Arg Arg Trp Ser
               100                 105                 110
   Gly Thr Ala Tyr Cys Arg Gln Met Arg Cys His Ala Leu Pro Phe Ile
           115                 120                 125
   Thr Ser Gly Thr Tyr Thr Cys Thr Asn Gly Val Leu Leu Asp Ser Arg
       130                 135                 140
   Cys Asp Tyr Ser Cys Ser Ser Gly Tyr His Leu Glu Gly Asp Arg Ser
   145                 150                 155                 160
   Arg Ile Cys Met Glu Asp Gly Arg Trp Ser Gly Gly Glu Pro Val Cys
                   165                 170                 175
   Val Asp Ile Asp Pro Pro Lys Ile Arg Cys Pro His Ser Arg Glu Lys
               180                 185                 190
   Met Ala Glu Pro Glu Lys Leu Thr Ala Arg Val Tyr Trp Asp Pro Pro
           195                 200                 205
   Leu Val Lys Asp Ser Ala Asp Gly Thr Ile Thr Arg Val Thr Leu Arg
       210                 215                 220
   Gly Pro Glu Pro Gly Ser His Phe Pro Glu Gly Glu His Val Ile Arg
   225                 230                 235                 240
   Tyr Thr Ala Tyr Asp Arg Ala Tyr Asn Arg Ala Ser Cys Lys Phe Ile
                   245                 250                 255
   Val Lys Val Gln Val Arg Arg Cys Pro Thr Leu Lys Pro Pro Gln His
               260                 265                 270
   Gly Tyr Leu Thr Cys Thr Ser Ala Gly Asp Asn Tyr Gly Ala Thr Cys
           275                 280                 285
   Glu Tyr His Cys Asp Gly Gly Tyr Asp Arg Gln Gly Thr Pro Ser Arg
       290                 295                 300
   Val Cys Gln Ser Ser Arg Gln Trp Ser Gly Ser Pro Pro Ile Cys Ala
   305                 310                 315                 320
   Pro Met Lys Ile Asn Val Asn Val Asn Ser Ala Ala Gly Leu Leu Asp
                   325                 330                 335
   Gln Phe Tyr Glu Lys Gln Arg Leu Leu Ile Ile Ser Ala Pro Asp Pro
               340                 345                 350
   Ser Asn Arg Tyr Tyr Lys Met Gln Ile Ser Met Leu Gln Gln Ser Thr
           355                 360                 365
   Cys Gly Leu Asp Leu Arg His Val Thr Ile Ile Glu Leu Val Gly Gln
       370                 375                 380
   Pro Pro Gln Glu Val Gly Arg Ile Arg Glu Gln Gln Leu Ser Ala Asn
   385                 390                 395                 400
   Ile Ile Glu Glu Leu Arg Gln Phe Gln Arg Leu Thr Arg Ser Tyr Phe
                   405                 410                 415
   Asn Met Val Leu Ile Asp Lys Gln Gly Ile Asp Arg Asp Arg Tyr Met
               420                 425                 430
   Glu Pro Val Thr Pro Glu Glu Ile Phe Thr Phe Ile Asp Asp Tyr Leu
           435                 440                 445
   Leu Ser Asn Gln Glu Leu Thr Gln Arg Arg Glu Gln Arg Asp Ile Cys
       450                 455                 460
   Glu
   465
<210> 23
   <211> 458
   <212> PRT
   <213> Xenopus laevis
<400> 23
   Met Glu Ala Ser Ile Thr Val Leu Leu Phe Ala Phe Thr Lys Val Ala
   1               5                   10                  15
   Ser Ser Leu Tyr Tyr Glu Gly Ser Gly His Ser Asp Gly Glu Ile Gln
               20                  25                  30
   Thr Asn Glu Val Tyr Val Glu Ser Arg Pro Leu Gly Pro Tyr Arg Ala
           35                  40                  45
   Pro Arg Trp Cys Tyr Asp Leu His Ile Ser Asp Gly Glu Ala Thr Cys
       50                  55                  60
   Tyr Ser Pro Leu Gly Pro Arg Tyr Arg Ser Thr Leu Gly Thr Arg Cys
   65                  70                  75                  80
   Arg Leu Ser Cys Asp Gln Gly Phe Lys Leu Ile Gly Gln Ser Ser Val
                   85                  90                  95
   Gln Cys Leu Ser Ser Arg Arg Trp Ser Gly Asn Gly His Cys Arg Arg
               100                 105                 110
   Ile Gln Cys His Val Leu Pro Pro Ile Phe Tyr Gly Ser Tyr His Cys
           115                 120                 125
   Ser Val Gly Val Ser Glu Gly Ser Arg Cys Asp Tyr Ser Cys Ala Pro
       130                 135                 140
   Gly Tyr Met Val Glu Gly Asp Arg Ser Arg Ile Cys Met Glu Asp Gly
   145                 150                 155                 160
   Gln Trp Ser Gly Gly Glu Pro Val Cys Val Asp Leu Asp Pro Pro Lys
                   165                 170                 175
   Ile Gln Cys Pro Val Ser Arg Met Lys Val Ala Glu Pro Glu Lys Leu
               180                 185                 190
   Thr Ala Arg Ile Phe Trp Gly Asn Pro Gln Val Lys Asp Ser Ala Asp
           195                 200                 205
   Gly Val Ile Thr Arg Val Phe Leu Arg Gly Pro Glu Pro Gly Ser Glu
       210                 215                 220
   Leu Pro Glu Gly Glu His Val Ile Arg Tyr Thr Ala Tyr Asp Arg Ala
   225                 230                 235                 240
   His Asn Arg Ala Ser Cys Lys Phe Ile Val Lys Val Gln Val Arg Arg
                   245                 250                 255
   Cys Pro Asp Leu Thr Pro Pro Leu His Gly Tyr Leu Thr Cys Ser Ala
               260                 265                 270
   Ala Gly Asn Asn Tyr Gly Ala Thr Cys Glu Tyr His Cys Glu Gly Gly
           275                 280                 285
   Tyr Glu Arg Gln Gly Pro Ala Ala Arg Val Cys Gln Phe Ser Gln Asn
       290                 295                 300
   Trp Ala Gly Thr Pro Ser Thr Cys Thr Pro Met Leu Ile Asn Val Asn
   305                 310                 315                 320
   Val Asn Ser Ala Gly Ala Phe Ile Asp Gln Phe Phe Glu Lys Gln Arg
                   325                 330                 335
   Leu Leu Phe Ile Ser Ser Pro Ser Ser Ser Asp Arg Tyr Tyr Arg Met
               340                 345                 350
   Gln Thr Thr Ala Leu Gln Ser Ser Gly Cys Gly Leu Glu Gln Arg His
           355                 360                 365
   Val Leu Ile Val Glu Leu Ile Gly Glu Ser Pro Arg Glu Val Gly Arg
       370                 375                 380
   Val Arg Asn Gln Gln Leu Ser Lys Glu Leu Ile Glu Glu Leu Arg Gln
   385                 390                 395                 400
   Val Leu Arg Ile Ser Arg Ser Tyr Phe Asn Met Val Leu Ile Asp Lys
                   405                 410                 415
   His Gly Val Asp Arg Asp Arg Tyr Arg Gln Pro Thr Ala Ser Glu Asp
               420                 425                 430
   Ile Phe Leu Phe Ile Asp Thr Tyr Leu Leu Ser Pro Arg Glu Leu Ser
           435                 440                 445
   Gln Val Glu Ser Asn Lys Glu Asn Cys Asp
       450                 455
<210> 24
   <211> 19
   <212> DNA
   <213> Mus musculus
<400> 24
   atggaagatg ggcgatgga 19
<210> 25
   <211> 21
   <212> DNA
   <213> Homo sapiens
<400> 25
   tctggctctg ccattttctc a 21
<210> 26
   <211> 21
   <212> DNA
   <213> Mus musculus
<400> 26
   atgctcctgg ctctgggact c 21
<210> 27
   <211> 42
   <212> DNA
   <213> Mus musculus
<400> 27
   ctaatacgac tcactatagg gtgcagctgg tccccttcta tg 42
<210> 28
   <211> 42
   <212> DNA
   <213> Mus musculus
<400> 28
   ctaatacgac tcactatagg gatgaccagt ccactgactc ag 42
<210> 29
   <211> 21
   <212> DNA
   <213> Mus musculus
<400> 29
   cgatctcctt ctaggtggta g 21
<210> 30
   <211> 21
   <212> DNA
   <213> Mus musculus
<400> 30
   atgaccagtc cactgactca g 21
<210> 31
   <211> 42
   <212> DNA
   <213> Mus musculus
<400> 31
   ctaatacgac tcactatagg gcgatctcct tctaggtggt ag 42

## Description

### BACKGROUND OF THE INVENTION

### I. Field of the Invention

The present invention relates generally to the fields of molecular biology and oncology. More particularly there are disclosed compositions comprising an antibody for SRPX2, and associated methods of treating angiogenesis-related conditions.

### II. Description of Related Art

Angiogenesis is a multi-step cellular process of capillary sprouting and formation of neo-vasculature from preexisting blood vessels. The complex process involves disassembly of endothelial junctions, followed by endothelial cell detachment, proliferation and migration as well as subsequent re-establishment of intercellular and cell-matrix contact. As such it requires coordinated actions of a variety of vascular cell adhesion molecules and growth factors originating from endothelial cells themselves or neighboring mural cells. Indeed, angiogenesis is a tightly tuned process regulated by pro- and anti-angiogenic factors (Folkman, 1995).

Angiogenesis-related diseases result when the angiogenic process is disregulated, resulting in excessive amounts of new blood vessels or an insufficient number of blood vessels.

Numerous studies have demonstrated that excessive angiogenesis influence significantly various disease states including tumor growth, ischemic cardiovascular pathologies or chronic inflammatory diseases (Carmeliet, 2003; Carmeliet, 2005; Gariano and Gardner, 2005) as well as ocular neovascularization, arterio-venous malformations, coronary restenosis, peripheral vessel restenosis, glomerulonephritis and rheumatoid arthritis. Ocular neovascularization disorders include macular degeneration (e.g., age-related macular degeneration (AMD)), corneal graft rejection, corneal neovascularization, retinopathy of prematurity (ROP) and diabetic retinopathy.

Tumor-associated angiogenesis is the most extensively studied. In 1971, Folkman was the first to postulate that tumors cannot grow further than the size of 2-3 mm³ in the absence of neovascularization (Folkman, 1971). Angiogenesis is a prerequisite for tumor growth and blocking this process could prevent further proliferation of tumor cells. Furthermore, prevention of angiogenesis targets normal tissue which does not undergo mutagenesis as seen with tumor cells. It is thus expected that anti-angiogenic therapy be better sustained in keeping tumor growth under control than any other treatments directly addressing tumor cells. Despite the fact that vascular endothelial cell growth factor (VEGF), fibroblast growth factor (FGF) and other pro-angiogenic molecules are indispensible for vessel formations (Hanahan, 1997; Yancopoulos *et al.,* 2000), the complete molecular and cellular mechanisms governing tumor-associated angiogenesis are poorly understood.

Diseases complicated by vascular leakage and/or neovascularization in the eye are responsible for the vast majority of visual morbidity and blindness in developed countries. Retinal neovascularization occurs in ischemic retinopathies such as diabetic retinopathy and is a major cause of visual loss in working age patients (Klein *et al.,* 1984). Choroidal neovascularization occurs as a complication of age-related macular degeneration and is a major cause of visual loss in elderly patients (Ferris *et al.,* 1984). Improved treatments are needed to reduce the high rate of visual loss, and their development is likely to be facilitated by a greater understanding of the molecular pathogenesis of ocular neovascularization.

In tissue vasculature, endothelial cells are in a resting state, do rarely proliferate and participate in the maintenance of vascular function such as oxygen exchange, vascular tonus, permeability, inflammatory immune response or LDL (low-density lipoprotein) clearance (Risau, 1997). The resting status changes during tumor development, when endothelial cells get stimulated by tumor-derived angiogenic factors. Endothelial cells then change their molecular expression profile, migrate out of the blood vessel structure and start proliferating and invading the tumor tissue. Mouse endothelioma cell lines with resting and angiogenic characteristics respectively have been produced (Aurrand-Lions *et al.,* 2004). The angiogenic cell line (t.End.1V^{high}) was selected on the basis of high expression of the integrin αVβ3 and its inability to endocytose acetylated LDL. In addition, several modulations of vascular functions have been shown with these cells such as increased cell migration, lack of inflammatory response and formation of cord-like structures in three dimensional fibrin gels (Aurrand-Lions *et al.,* 2004).

In clinical trials, beneficial effects of anti-angiogenic drugs were so far reached with antibodies against VEGF in the context of colon and breast carcinomas. However, it was less successful with other tumors for which alternate factors may be involved. Thus, other molecules involved in angiogenesis should be identified and used alone or in combination with the growth factors. Targeting novel vascular molecules expressed and/or secreted by angiogenic endothelial cells represent an additional avenue. On the other hand, insufficient angiogenesis is also related to a large number of diseases and conditions, such as cardiovascular diseases (e.g., coronary artery diseases) and delayed wound healing. To date, cardiovascular diseases are the leading cause of mortality in the United States, Europe, and Israel. In the United States, approximately one million deaths per year are attributed to cardiac causes, fifty percent of which are attributed to coronary artery disease (CAD). The major morbidity from CAD is a result of obstructive coronary artery narrowing and the resultant myocardial ischemia CAD affects more than 13 million people, and its annual economic burden is in excess of sixty billion U.S. Dollars.

Mechanical revascularization of obstructive coronary stenoses by percutaneous techniques, including percutaneous transluminal angioplasty and stent implantation, is used to restore normal coronary artery blood flow. In addition, coronary artery occlusion bypass surgery is performed using arterial and venous conduits as grafts onto the coronary arterial tree. These treatment modalities have significant limitations in individuals with diffuse atherosclerotic disease or severe small vessel coronary artery disease, in diabetic patients, as well as in individuals who have already undergone surgical or percutaneous procedures.

For these reasons, therapeutic angiogenesis, aimed at stimulating new blood vessel growth, is highly desirable. The therapeutic concept of angiogenesis therapy is based on the premise that the existing potential for vascular growth inherent to vascular tissue can be utilized to promote the development of new blood vessels under the influence of the appropriate angiogenic molecules. Therapeutic angiogenesis defines the intervention used to treat local hypovascularity by stimulating or inducing neovascularization for the treatment of ischemic vascular disease.

Animal studies have proven the feasibility of enhancing collateral perfusion and function via angiogenic compounds. Those experiments proved that exogenous administration of angiogenic growth factors or their genetic constructs could promote collateral vessel growth in experimental models of chronic ischemia. Although such studies demonstrated proof of concept, additional studies raise issues that still have not been resolved, such as the duration of exposure of the vessels to angiogenic factors and the brief half-lives of such proteins. Therefore, there remains a need to search novel angiogenesis modulators for promoting or inducing angiogenesis when needed.
Previously, polynucleotides and genes that are differentially expressed in lymphatic versus blood vascular endothelial cells have been described (WO 03/080640).
Moreover, SRPX2 has previously been described as a novel therapeutic target in gastric cancer (Tanaka *et al.,* 2007).

### SUMMARY OF THE INVENTION

The present invention is based in part on the finding that SRPX2 is involved in cancer angiogenesis. For example, the inventors have found that decreased SRPX-2 expression in angiogenic cells, results in reduction of endothelial cell migration and angiogenic sprout formation. Further, the present invention is in part based on the finding that SRPX2 gene is overexpressed in angiogenic endothelial cells and in *de novo* vascular endothelium of bFGF-treated matrigel plugs or progressing tumors *in vivo.*
Thus, the present invention relates to the embodiments as characterized in the claims. Thus, it relates to the following items.
1. An antibody or a fragment thereof that binds to an SRPX2 amino acid sequence selected from SEQ ID NOs: 14-23 and inhibits the activity of SRPX2 in angiogenesis.
2. A pharmaceutical composition comprising the antibody or a fragment thereof of item 1 and a pharmaceutically acceptable carrier.
3. An antibody or a fragment thereof of item 1 for use in the treatment of an angiogenesis related condition.
4. An antibody or a fragment thereof of item 1 for use in the treatment of an angiogenesis related condition of item 3, wherein the angiogenesis-related condition is cancer, ocular neovascularization, arterio-venous malformations, coronary restenosis, peripheral vessel restenosis, glomerulonephritis, rheumatoid arthritis, ischemic cardiovascular pathologies, chronic inflammatory diseases, transplantation, cardiovascular diseases, aneurisms and wound healing.
5. An antibody or a fragment thereof of item 1 for use in the treatment of an angiogenesis related condition of item 4, wherein the said cancer is selected from the group consisting of breast cancer, lung cancer, prostate cancer, ovarian cancer, brain cancer, liver cancer, cervical cancer, colorectal cancer, renal cancer, skin cancer, head and neck cancer, bone cancer, esophageal cancer, bladder cancer, uterine cancer, lymphatic cancer, stomach cancer, pancreatic cancer, testicular cancer, lymphoma, and leukemia.
6. The pharmaceutical composition of item 2, wherein the pharmaceutical composition further comprises a lipid component.
7. The pharmaceutical composition of item 6, wherein the lipid component forms a liposome.
8. The pharmaceutical composition of item 6, wherein the lipid is 1,2-dioleoyl-sn-glycero-3-phosphatidylcholine (DOPC), egg phosphatidylcholine ("EPC"), dilauryloylphosphatidylcholine ("DLPC"), dimyristoylphosphatidylcholine ("DMPC"), dipalmitoylphosphatidylcholine ("DPPC"), distearoylphosphatidylcholine ("DSPC"), 1 -myristoyl-2-palmitoyl phosphatidylcholine ("MPPC"), 1-palmitoyl-2-myristoyl phosphatidylcholine ("PMPC"), 1-palmitoyl-2-stearoyl phosphatidylcholine ("PSPC"), 1-stearoyl-2-palmitoyl phosphatidylcholine ("SPPC"), dimyristyl phosphatidylcholine ("DMPC"), 1,2-distearoyl-sn-glycero-3-phosphocholine ("DAPC"), 1,2-diarachidoyl-sn-glycero-3-phosphocholine ("DBPC"), 1,2-dieicosenoyl-sn-glycero-3-phosphocholine ("DEPC"), palmitoyloeoyl phosphatidylcholine ("POPC"), lysophosphatidylcholine, dilinoleoylphosphatidylcholine distearoylphophatidylethanolamine ("DSPE"), dimyristoyl phosphatidylethanolamine ("DMPE"), dipalmitoyl phosphatidylethanolamine ("DPPE"), palmitoyloeoyl phosphatidylethanolamine ("POPE"), lysophosphatidylethanolamine, phosphatidylserine, phosphatidylglycerol, dimyristoyl phosphatidylserine ("DMPS"), dipalmitoyl phosphatidylserine ("DPPS"), brain phosphatidylserine ("BPS"), dilauryloylphosphatidylglycerol ("DLPG"), dimyristoylphosphatidylglycerol ("DMPG"), dipalmitoylphosphatidylglycerol ("DPPG"), distearoylphosphatidylglycerol ("DSPG"), and dioleoylphosphatidylglycerol ("DOPG").
9. The pharmaceutical composition of item 2, wherein the pharmaceutical composition further comprises cholesterol or polyethyleneglycol (PEG).
10. The pharmaceutical composition of item 2, wherein the composition is administered to a cell expressing urokinase-type plasminogen activator receptor (uPAR).

There is also disclosed an isolated nucleic acid molecule comprising a sequence that hybridizes with an SRPX2 nucleotide sequence selected from the group consisting of SEQ ID NOs:1-10 and inhibit the expression of SRPX2 in a cell. The nucleic acid in this regard is preferably an siRNA, a double stranded RNA, a short hairpin RNA, an antisense oligonucleotide, a ribozyme, a nucleic acid encoding thereof. Preferably, the nucleic acid is further defined as an siRNA or a nucleic acid encoding an siRNA. For example, the siRNA may preferably comprise one or more sequences selected from the group consisting of SEQ ID NO:11, SEQ ID NO:12 and SEQ ID NO:13, or comprise SEQ ID NO:11 and SEQ ID NO:12, or comprise SEQ ID NO:12 and SEQ ID NO:13, or comprise SEQ ID NO:11 and SEQ ID NO:13.

In still further embodiments, the The invention is directed to approaches to inhibiting the action of SRPX2, such as an antibody or a fragment thereof that binds to an SRPX2 amino acid sequence selected from the group consisting of SEQ ID NOs: 14-23 and inhibits the angiogenic activity of SRPX2. In certain aspects, a pharmaceutical composition comprising the antibody or the fragment and a pharmaceutically acceptable carrier is also provided.

There is also disclosed a pharmaceutical composition comprising one or more of the nucleic acids, or the antibody or the fragment thereof, and a pharmaceutically acceptable carrier. In certain aspects, the composition may be administered to a cell expressing urokinase-type plasminogen activator receptor (uPAR), since SRPX2 may be a ligand for uPAR to effect the angiogenic activity. The composition may optionally further comprise a lipid component, which is believed to likely give the nucleic acid an improved stability, efficacy and bioavailability, with perhaps even reduced toxicity. The lipid component may form a liposome, but this is not believed to be required. In certain aspects, the composition further comprises cholesterol or polyethyleneglycol (PEG).

Exemplary lipids include, but are not limited to, 1,2-dioleoyl-sn-glycero-3-phosphatidylcholine (DOPC), egg phosphatidylcholine ("EPC"), dilauryloylphosphatidylcholine ("DLPC"), dimyristoylphosphatidylcholine ("DMPC"), dipalmitoylphosphatidylcholine ("DPPC"), distearoylphosphatidylcholine ("DSPC"), 1-myristoyl-2-palmitoyl phosphatidylcholine ("MPPC"), 1-palmitoyl-2-myristoyl phosphatidylcholine ("PMPC"), 1-palmitoyl-2-stearoyl phosphatidylcholine ("PSPC"), 1-stearoyl-2-palmitoyl phosphatidylcholine ("SPPC"), dimyristyl phosphatidylcholine ("DMPC"), 1,2-distearoyl-sn-glycero-3-phosphocholine ("DAPC"), 1,2-diarachidoyl-sn-glycero-3-phosphocholine ("DBPC"), 1,2-dieicosenoyl-sn-glycero-3-phosphocholine ("DEPC"), palmitoyloeoyl phosphatidylcholine ("POPC"), lysophosphatidylcholine, dilinoleoylphosphatidylcholine distearoylphophatidylethanolamine ("DSPE"), dimyristoyl phosphatidylethanolamine ("DMPE"), dipalmitoyl phosphatidylethanolamine ("DPPE"), palmitoyloeoyl phosphatidylethanolamine ("POPE"), lysophosphatidylethanolamine, phosphatidylserine, phosphatidylglycerol, dimyristoyl phosphatidylserine ("DMPS"), dipalmitoyl phosphatidylserine ("DPPS"), brain phosphatidylserine ("BPS"), dilauryloylphosphatidylglycerol ("DLPG"), dimyristoylphosphatidylglycerol ("DMPG"), dipalmitoylphosphatidylglycerol ("DPPG"), distearoylphosphatidylglycerol ("DSPG"), or dioleoylphosphatidylglycerol ("DOPG").

It is contemplated that the SRPX2 inhibitory antibodies or the compositions may be used in the treatment of any disease or disorder in which angiogenesis plays a role, which will be referred to generally as an angiogenesis-related condition. It is contemplated that the invention will find applicability in any such disorder in humans or other animals. Exemplary angiogcncsis-rclatcd conditions include cancer, ocular neovascularization, arterio-venous malformations, coronary restenosis, peripheral vessel restenosis, glomerulonephritis, rheumatoid arthritis, ischemic cardiovascular pathologies, chronic inflammatory diseases and so on. In the case of cancer, exemplary angiogenic cancers include angiogenic breast cancer, lung cancer, prostate cancer, ovarian cancer, brain cancer, liver cancer, cervical cancer, colorectal cancer, renal cancer, skin cancer, head and neck cancer, bone cancer, esophageal cancer, bladder cancer, uterine cancer, lymphatic cancer, stomach cancer, pancreatic cancer, testicular cancer, lymphoma, or leukemia. Ocular neovascularization disorders include macular degeneration (*e.g.*, age-related macular degeneration (AMD)), corneal graft rejection, corneal neovascularization, retinopathy of prematurity (ROP) and diabetic retinopathy.

There is also disclosed a pharmaceutical composition for inducing angiogenesis in a subject, comprising an isolated SRPX2 protein or peptide comprising at least 10 amino acids having at least 95% identity to an amino acid sequence selected from the group consisting of SEQ ID NOs:14-23 and a pharmaceutically acceptable carrier. The composition may optionally further comprise a lipid component, which is believed to likely give the nucleic acid an improved stability, efficacy and bioavailability, with perhaps even reduced toxicity. The lipid component may form a liposome, but this is not believed to be required. In certain aspects, the composition further comprises cholesterol or polyethyleneglycol (PEG). Exemplary lipids are described as above.

There is also disclosed a method for treating an angiogenesis-related condition comprising administering to a subject in need of angiogenesis an amount of the composition that is effective to induce angiogenesis. Preferably, the subject is a human subject. Exemplary angiogenesis-related condition in need of an angiogenesis include, transplantation, cardiovascular diseases, aneurisms or wound healing. In particular embodiments, the angiogenesis-related condition is wound healing.

It is contemplated that any method or composition described herein can be implemented with respect to any other method or composition described herein.

The use of the word "a" or "an" when used in conjunction with the term "comprising" in the claims and/or the specification may mean "one," but it is also consistent with the meaning of "one or more" or "at least one." The term "about" means, in general, the stated value plus or minus 5%. The use of the term "or" in the claims is used to mean "and/or" unless explicitly indicated to refer to alternatives only or the alternative are mutually exclusive, although the disclosure supports a definition that refers to only alternatives and "and/or."

Other objects, features and advantages of the present invention will become apparent from the following detailed description. It should be understood, however, that the detailed description and the specific examples, while indicating specific embodiments of the invention, are given by way of illustration only

### BRIEF DESCRIPTION OF THE DRAWINGS

The following drawings are part of the present specification and are included to further demonstrate certain aspects of the present invention. The invention may be better understood by reference to the drawing in combination with the detailed description of specific embodiments presented herein.

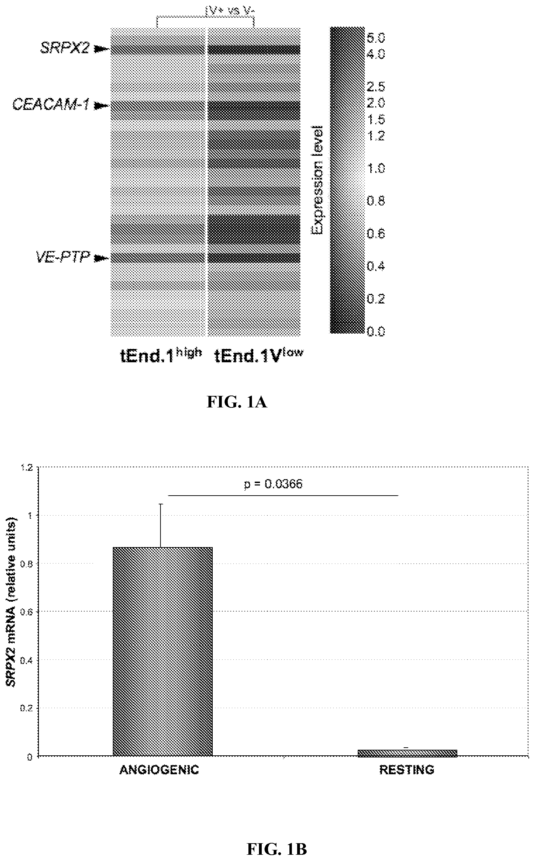

**FIGs. 1A-B****. Differential expression of *SRPX2* mRNA in tEnd.1V^{high} (angiogenic) versus tEnd.1V^{low} (resting) endothelial cells. (1A)** Selection of genes differentially expressed by tEnd.1V^{high} cells overlaid by genes up-regulated after vMIP-II activation of HUVEC, illustrated by a condition tree. Two examples of angiogenic genes with differential expression are CECAM-1 and VE-PTP. Prominent differential gene expression was obtained with the gene SRPX2. The colors are coded according to the expression level bar. (1B) Validation of data obtained by microarray analysis using quantitative RT-PCR. Bars represent the quantity of the *SRPX2* mRNA (relative units) in total RNA isolated from angiogenic and resting cells. Values for each sample were normalized to three murine house keeping genes: *β-actin, β-tubulin* and *EEF1A.* Relative values from individual experiments were averaged and plotted with standard deviation (SD) as error bars. The statistical analysis was performed using the Welch t-test (p=0.03664).

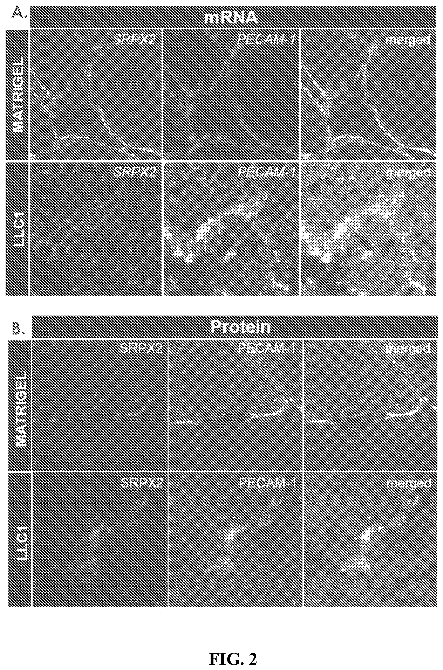

**FIGs. 2A-B.** ***In vivo* expression of SRPX2 in angiogenic tissue. (2A)** Double *in situ* mRNA hybridization on angiogenic vessels immigrated into bFGF-treated matrigel plugs (upper panel) or LLC1 tumors (lower panel). Cryo-sections were incubated with SRPX2 riboprobes (upper panel; lower panel) and PECAM-1 riboprobes (upper panel; lower panel). Double labeling illustrates that SRPX2 expressing cells are PECAM-1 positive (merged). **(2B)** Double immuno-fluorescence on angiogenic vessels immigrated into bFGF-treated matrigel. plugs (upper panel) or LLC1 tumors (lower panel). Cryo-sections were incubated with rabbit anti-SRPX2 antibody detected by mouse anti-rabbit IgG and rat anti-PECAM-1 antibody detected by rabbit anti-rat IgG. Double labeling illustrates that SRPX2 expressing cells are PECAM-1 positive (merged).

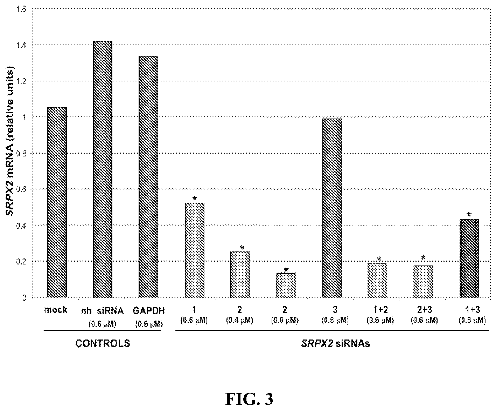

**FIG. 3****. Validation of down-regulation of *SRPX2* gene expression by siRNAs.** Inhibition of *SRPX2* expression in angiogenic cells by three siRNA sequences *(SRPX2* siRNA 1 (SEQ ID NO:11), 2 (SEQ ID NO:12) and 3 (SEQ ID NO:13)) and their combinations *(SRPX2* siRNA 1+2 (SEQ ID NO:11 and SEQ ID NO:12), 2+3 (SEQ ID NO:12 and SEQ ID NO:13), 1+3 (SEQ ID NO:11 and SEQ ID NO:13)). Transfection of *SRPX2*-targeted and control (*nh* siRNA and GAPDH) siRNAs in the concentration of 0.6 µM, except 0.4 µM for one condition of *SRPX2* siRNA 2 (SEQ ID NO:12) was carried out using Nucleofector technology(Amaxa). At 24 hours post-transfection, expression of target and control genes were analyzed by qPCR. The values were normalized to the expression levels of mouse *β-actin, β-tubulin* and *EEF1A.* Abbreviations: *nh* siRNA, non homologous siRNA; GAPDH, glyceraldehyde-3-phosphate dehydrogenase; siRNAs, small interfering RNAs; qPCR, quantitative polymerase chain reaction.

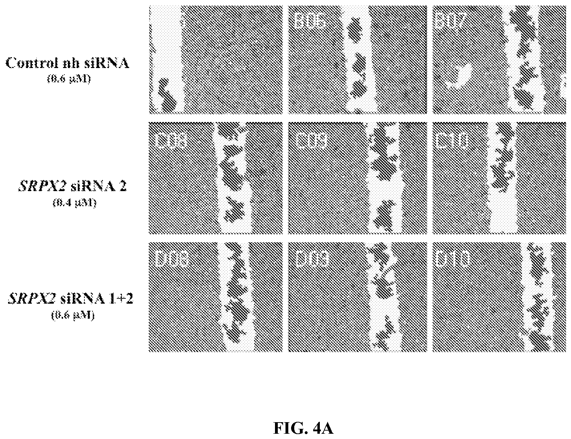

**FIGs. 4A-C****. Delayed wound healing of endothelial cells silenced for *SRPX2* expression. (4A)** Monolayer cultures of angiogenic cells from *SRPX2* silenced (*SRPX2* siRNA 2 (SEQ ID NO:12), 0.4 µM and *SRPX2* siRNA 1+2 (SEQ ID NO:11 and SEQ ID NO:12), 0.6 µM each) and control (nh siRNA, 0.6 µM) angiogenic cells were wounded with a pipette tip. Cells at the edge of the wound migrated into the wounded area. After 16 hours the cells were photographed and the migrated distance was determined. The distance of migration in µm was calculated by subtracting the yellow surface minus the violet surface and divided by 3354. One image pixel corresponds to 1.6125 µm. Each bar is the mean of three independent culture wells, and three experiments were performed. The progress of wound closure was significantly delayed in the SRPX2 siRNA 2 (SEQ ID NO:12) silenced cells compared to mock and nh siRNA silenced cells. **(4B)** The progress of wound closure, expressed as migrated distance (µm per 16 hours), was significantly delayed in the *SRPX2* siRNA 2 (SEQ ID NO:12) silenced cells and in the cells silenced with the combination of the SRPX2 siRNA 1 (SEQ ID NO:11) and 2 (SEQ ID NO:12) compared to control cells (dark grey bars). **(4C)** Migration analysis of individual cells in a wound healing assay. Measurements were taken from 200 separate distance points in three separate wells. One image pixel corresponds to 1.6125 µm. Values from individual experiments were averaged and the mean values were plotted with standard deviation (SD) as error bars. The statistical analysis was performed using t-test.

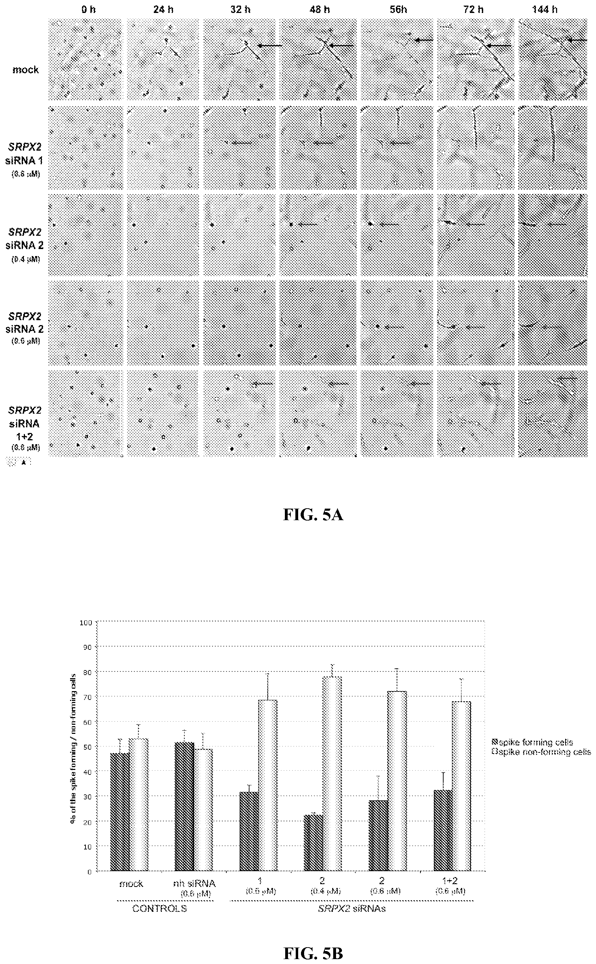

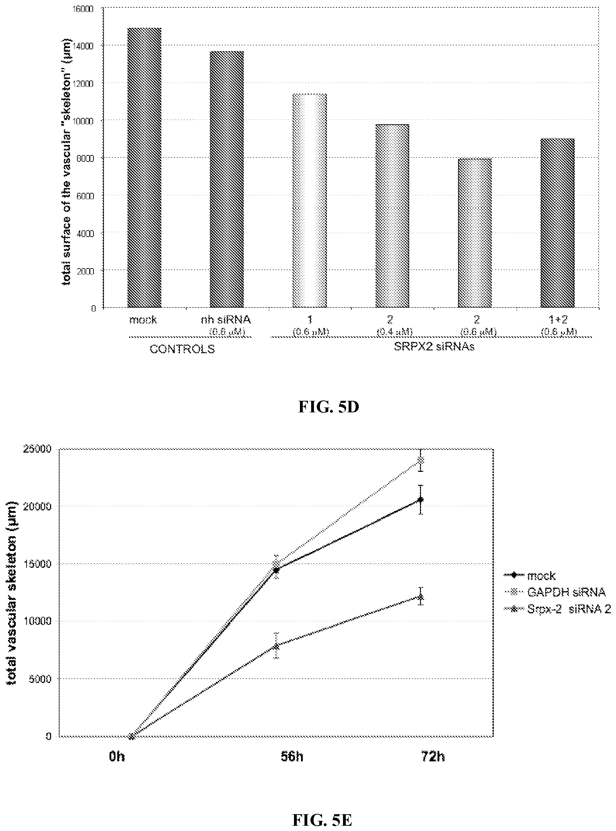

**FIGs. 5A-F****. Silencing of SRPX2 in endothelial cells attenuates the initiation and the final steps of angiogenesis *in vitro.* (5A)** Cord formation *in vitro* starts with individual endothelial cells sending out spikes (as shown by control, mock transfected cells at 24 hours, black arrowhead). It continues with cell-cell contact formation, which leads to branching of the proliferating cells forming a polygonal network (as shown by control, mock transfected cells at 32 - 144 h, black arrows). Two *SRPX2* siRNAs *(SRPX2* siRNA 1 (SEQ ID NO:11) and 2 (SEQ ID NO:12), 0.4-0.6 µM) and their combination (*SRPX-2* siRNA 1+2 (SEQ ID NO:11 and SEQ ID NO:12), 0.6 µM each) were transfected into angiogenic cells, which are then cultured in 3D fibrin gels for 144 hours. During early phases of the angiogenesis assay (24 - 32 hours), delayed formation of spikes (red arrowheads) was observed with *SRPX2*-silenced cells (SRPX-2 siRNAs 1 (SEQ ID NO:11), 2 (SEQ ID NO:12), 1+2 (SEQ ID NO:11 and SEQ ID NO:12), 0.4-0.6 µM). During later phases of the angiogenesis assay (48 - 144 hours), decreased ability for branching and cord formation was observed with silenced cells leading to a less complex network (arrows). **(5B)** Quantification of angiogenic cells numbers that form spikes at early phases during the angiogenesis assay. The spikes forming (black bars) and non-forming (light grey bars) cells during the first 24 - 32 hours in 3D fibrin gels were counted and plotted as percentiles. Delayed formation of spikes was observed with SRPX2-silenced cells (SRPX-2 siRNAs 1 (SEQ ID NO:11), 2 (SEQ ID NO:11 and SEQ ID NO:12), 1+2 (SEQ ID NO:11 and SEQ ID NO:12), 0.4-0.6 µM), when compared with the control cells (mock and nh siRNA transfected cells). The mean and standard deviation of two experiments is shown. (5C) Computer assisted image analysis of total surface of the vascular "skeleton" representing the capillary-like network of sprouting tEnd1.V^{high} cells at 56 and 72 hours. The skeleton lengths and complexity of SRPX2 silenced cells is reduced in comparison to mock transfected cells. **(5D)** Measurement of total surface of the vascular "skeleton" representing the capillary-like network at 56 hours. Development of this network decreased when *SRPX2* gene was silenced *(SRPX2* siRNAs 1 (SEQ ID NO:11), 2 (SEQ ID NO:12), 1+2 (SEQ ID NO:11 and SEQ ID NO:12), 0.4-0.6 µM) compared to control cells (mock or nh siRNA transfected cells). **(5E)** Time course of vascular skeleton formation of tEnd1.V^{high} angiogenic cells after SRPX2 silencing (SRPX2 siRNA 2, 0.6 µM) compared to mock or control (GAPDH siRNA, 0.6 µM) transfected cells. Error bars correspond to standard deviation. **(5F)** Quantification of number of apoptotic endothelial cells 6 days after seeding into 3D fibrin gels. SRPX-2 silencing did not cause significant cell death (SRPX2 siRNA 1, 2 and 1+2, 0.4-0.6 µM) when compared to control cells (mock, nh siRNA or GAPDH siRNA transfected cells, 0.6 µM).

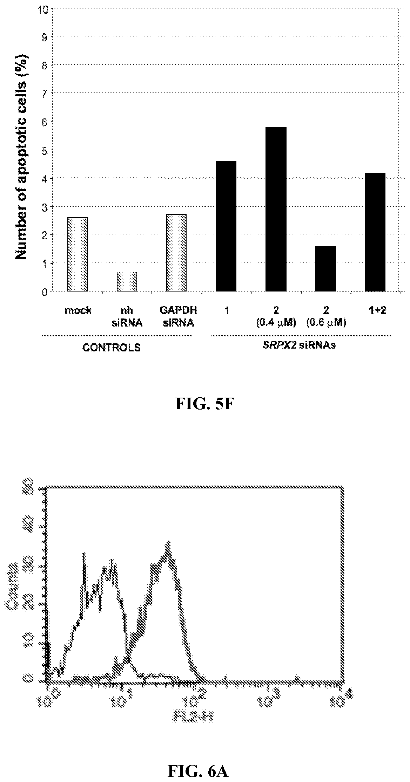

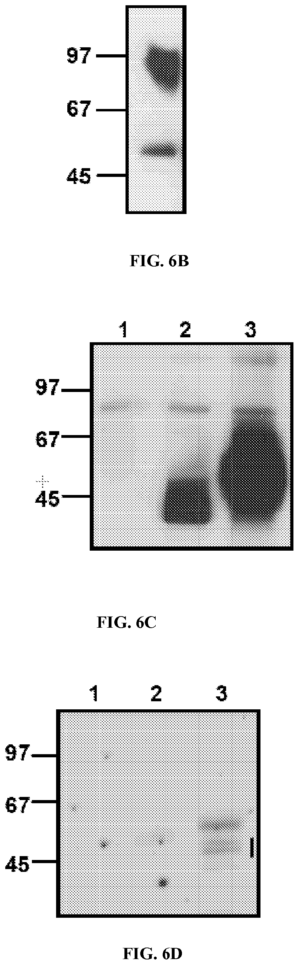

**FIGs. 6A-E****. SRPX2 binds to vascular uPAR. (6A)** Flow cytometry of tEnd1.V^{high} cells stained by mouse anti-uPAR antibody detected by FITC labeled sheep anti-mouse IgG with laser excitation at 488 nm (right peak). For control, secondary antibody only was used (black line). Mean fluorescence intensity was determined from at least 10, 000 counted cells. **(6B)** Production and purification of recombinant mouse SRPX2-FLAG. The full length mouse SRPX2 gene was cloned as a FLAG tagged construct into the expression vector pcDNA3.3™-TOPO®TA. This construct was transfected and stably expressed in MDCK cells under neomycin selection. The cell culture supernatants were collected and SRPX2 protein was then affinity purified with anti-FLAG agarose beads and eluted with FLAG peptide. Western blot analysis of purified SRPX2 was revealed with biotinylated anti-FLAG HRP labeled antibodies. Molecular weight of detected soluble SRPX2 is 54 kDa. Apparent band with the higher molecular weight of 90 kDa stems from putative dimerization of SRPX2. **(6C)** Immunoprecipitation of native uPAR. Immunoprecipitation was preformed with tEnd1.V^{high} cells after cell surface protein biotinylation, followed by Western blotting using a sheep anti-uPAR antibody. Lane 1: protein G beads only; lane 2: protein G beads and rat anti-mouse JAM-C antibody as a control; lane 3: protein G beads and commercial sheep anti-uPAR antibody (Abcam). Apparent molecular weight of detected uPAR is 45 kDa. **(6D) Pull down** assay of native SPRX2 by uPAR. HRP of the blot shown in FIG. 6C was deactivated by sodium-azide and the blot was re-incubated with a commercial rabbit anti-SRPX2 antibody (ProteinTech Group Inc.), revealed by anti-rabbit HRP labeled antibodies and detected by ECL. SRPX2 appears as a doublet of 54 kDa and 50 kDa (bar). (6E) Triple immuno-fluorescence on angiogenic vessels immigrated into bFGF-treated matrigel plugs (upper panel) or LLC1 tumors (lower panel). Cryo-sections were incubated with rabbit anti-SRPX2 antibody detected by donkey anti-rabbit IgG (light blue), sheep anti-uPAR antibody detected by donkey anti-sheep IgG (red) and rat anti-PECAM-1 antibody detected by rabbit anti-rat IgG (green). Triple labeling illustrates that SRPX2 and uPAR co-localize on vascular endothelial cells in vivo and are PECAM-1 positive (merged).

### DETAILED DESCRIPTION OF THE INVENTION

### I. The Present Invention

There are disclosed compositions and methods of delivery of an inhibitory antibody specific for SRPX2 to treat angiogenesis-related disease, such as cancer. The present invention is based on the finding that SRPX2 is a novel angiogenesis modulator. For example, the inventors have found that decreased SRPX2 expression, results in reduction of migration of angiogenic cells and attenuation of initial and final steps of angiogenesis.

### II. SRPX2

The authors have discovered that SRPX2 is involved in angiogenesis and could serve as a target for treating angiogenesis-related conditions by using SRPX2 positive or negative modulators.

Using bioinformatics work and experimental approaches the authors have analyzed the glycoprotein SRPX2 in angiogenic cell lines. The authors used the t.End.1V^{high} angiogenic and t.End.1V^{low} resting cell lines to identify novel molecules differentially expressed and associated with angiogenesis. Among the identified new angiogenesis-associated genes, which fulfill the criteria described above, the authors identified the *SRPX2* gene (Sushi repeat-containing protein, X-linked, 2). The expression of this gene is up-regulated in pro-B leukemia cells by the E2A/HLF fusion protein, a transcription factor generated by a chromosal translocation t(17;19)(q23:p13) (Kurosawa *et al.,* 1999, where SRPX2 is referred to as SRPUL (Sushi-repeat protein up-regulated in leukemia)).

SRPX2 is a 465-amino acid protein in human (SEQ ID NO:14; GenBank accession number: NP_055282) with a signal peptide at the N-terminus and three consensus sushi repeats (or complement control sequences). These consensus motifs of approximately 60 amino acids are found in proteins of the complement system (O'Leary *et al.,* 2004) and in the extra-cellular domain of selectins, adhesion molecules involved in leukocyte migration (Bevilacqua, 1993; Johnston *et al.,* 1989). Selectins are phylogenetically similar to *SRPX2* and together belong to a gene family of 5 members: SRPX2, SRPX (SRPX1/EXT1/DRS), P-selectin, E-selectin and SVEP1 (selectin-like protein) (Royer et al., 2007). Recently it was found that P-selectin is involved in ischemia-induced angiogenesis by promoting early inflammatory mononuclear cell infiltration and E-selectin supports homing of endothelial progenitors (Egami et al., 2006; Oh et al., 2007). In addition, SRPX2 has a hyaline repeat (HYR) domain, which is related to the immunoglobulin-like fold and appears to be involved in cellular adhesion processes (Callebaut *et al.,* 2000). There is also a second gene (SRPX also called ETX1) related to SRPX2; however, it only shares 47% amino acid identity with SRPX2 (Meindl *et al.,* 1995).

In addition to its expression in human, SRPX2 proteins have been characterized in a few other species including several primates, mouse, cattle, *Xenopus laevis, etc.* and share homology among vertebrates. Their protein sequences correspond to SEQ ID NOs: 14-23 and are translated from nucleotide sequences corresponding to SEQ ID NOs:1-10 as incorporated for their entirety.

SRPX2 is a soluble molecule with several receptors, one of them being uPAR (Royer-Zemmour *et al.,* 2008). It is highly expressed by the angiogenic tEnd1.V^{high}, but not the resting tEnd1.V^{low} endothelial cell line. A primary human endothelium SRPX2 expression is driven by the pro-angiogenic chemokine vMIP-II (Cherqui *et al.,* 2007). *In vivo* the authors have demonstrated SRPX2 expression by *de novo* formed blood vessels.

The highest expression of a 2.5 kb mouse SRPX2 transcript was detected in heart, ovary and placenta, while the SRPX2 protein was secreted by mouse pro-B cells (Kurosawa *et al.,* 1999). Mutations were identified in the human *SRPX2* gene as being responsible for X-linked epilepsy associated with oral and speech dyspraxia and mental retardation. The disease-causing mutation resulted in gain of glycosylation of the secreted mutant protein. In the mouse brain, SRPX2 protein expression appeared in neurons at birth, suggesting a role of *SRPX2* in the development and/or function of the perisylvian region critical for language and cognitive development (Roll *et al.,* 2006). Recently it has been found that over-expression of SRPX2 enhances cellular migration and adhesion in gastric cancer cells and SRPX2 functions in cellular migration and adhesion through FAK signaling (Tanaka *et al.,* 2009).

SRPX2 contains three sushi repeats also called complement control sequences (Kurosawa *et al.,* 1999). The sushi repeats were first identified in plasma β2 glycoprotein (Lozier *et al.,* 1984) as well as in transglutaminases, one of them is factor XIIIa (Ichinose *et al.,* 1990). More importantly, sushi repeats are also found in the selectins, adhesion molecules involved in leukocyte adhesion to endothelium (Bevilacqua, 1993). Depending on the type of selectin, a variable number (2-9) of sushi consensus repeats are present (Bevilacqua *et al.,* 1989; Collins *et al.,* 1991; Jutila *et al.,* 1992) along with one N-terminal lectin domain and one epidermal growth factor (EGF)-like domain (Tedder *et al.,* 1995).

The spatial relationship of these domains is important for the adhesive function of selectins (Tedder *et al.,* 1995). While the lectin and the EGF domains are directly involved in selectin-mediated adhesion (Kansas *et al.,* 1991; Pigott *et al.,* 1991), the sushi repeats contribute indirectly to adhesion. They stabilize the structure of the selectin, lead to oligomerization and extend the lectin-EGF domains to appropriate distance from the membrane for optimal ligand binding activity (Tedder *et al.,* 1995). Moreover, when the sushi repeats were inactivated in L- and E-selectins, the adhesive function of both molecules was disrupted (Jutila *et al.,* 1992).

A similar effect may be mediated by the sushi repeats found in SRPX2. It may increase the affinity of SRPX2 produced as an autocrine factor for uPAR, its endothelial receptor or a putative additional receptors. Alternatively, SRPX2 may associate with the ECM and form a 3D lattice offering niches for sprouting endothelial cells. This may occur similar to the formation of layers around echinoderm embryos by hyaline, a protein comprising exclusively HYR domains (hyaline repeat). The HYR interact with a hyaline cell surface receptor and this adhesion seems to be essential for early development (Wessel *et al.,* 1998). Interestingly, the sushi repeat domains of SRPX2 flank a HYR domain (Callebaut *et al.,* 2000), suggesting that SRPX2 may interact with angiogenic endothelial cells. It may associate with ECM and form a microenvironment into which sprouting endothelial cells may protrude. However, SRPX2 has never been identified as a regulator of angiogenesis nor a cancer target in any human cancers until the present invention.

There are several other molecules containing sushi repeat domains. One of them is IL-15Rα. It was shown that the sushi repeats are critical for the binding of IL-15 and the function of this protein in inhibition of inflammatory responses (Wei *et al.,* 2001). Another molecule is IL2Rα, which has two sushi repeat domains that are required for its high affinity binding to IL-2 (Robb *et al.,* 1988). Thus, the sushi repeat domains may be generally involved in protein-protein interactions and are essential for a range of ligand-receptor binding and their biological functions.

Recent studies showed that SRPX2 in the brain binds to neural urokinase-type plasminogen activator receptor (uPAR) and two more members of the cellular proteolysis machinery; the cysteine protease cathepsin B (CTSB), an activator of uPA and the metalloproteinase ADAMTS4 (Royer-Zemmour *et al.,* 2008). It is well described that uPAR is involved in invasive cell migration and in particular with angiogenesis (Blasi and Carmeliet, 2002; Colman, 2006; Das and McGuire, 2006; Lakka *et al.,* 2005; McMahon and Kwaan, 2008). Here the authors showed that SRPX2 is also a ligand for vascular uPAR.

The urokinase plasminogen activator receptor (uPAR), a glycosylphosphatidylinositol-linked glycoprotein, plays a central role in the regulation of pericellular proteolysis and participates in events leading to cell activation. The uPA/uPAR complex regulates extracellular proteolysis, integrin activity and signaling. It is involved in many physiological and pathological processes, such as pericellular proteolysis, wound healing, tissue regeneration and tumor progression (Blasi and Carmeliet, 2002; Madsen and Sidenius, 2008). More importantly, the uPAR has been directly implicated in angiogenesis during tumor progression (Madsen and Sidenius, 2008; McMahon and Kwaan, 2008).

### III. Inhibition of SRPX2 Gene Expression

The authors have found that SRPX2 is overexpressed in angiogenic endothelial cells and is also expressed in angiogenic tissue *in vivo.* Its expression level correlates with cell migration in angiogenesis-related conditions, such as wound healing. Thus, control of SRPX2 expression is considered by the authors to be effective for treatment of excessive angiogenesis, such as cancer or ocular diseases. Inhibitory nucleic acids or any ways of inhibiting gene expression known in the art are contemplated in the present disclosure.

### A. Inhibitory Nucleic Acids

As mentioned, there is disclosed the use of one or more inhibitory nucleic acid for inhibiting or reducing the angiogenic action of SRPX2. Examples of an inhibitory nucleic acid include siRNA (small interfering RNA), short hairpin RNA (shRNA), double-stranded RNA, an antisense oligonucleotide, a ribozyme and a nucleic acid encoding thereof. An inhibitory nucleic acid may inhibit the transcription of a gene or prevent the translation of a gene transcript in a cell. An inhibitory nucleic acid may be from 16 to 1000 nucleotides long, and in certain embodiments from 18 to 100 nucleotides long. In certain embodiments, the inhibitory nucleic acid is an isolated nucleic acid that binds or hybridizes to a SRPX2 nucleotide sequence selected from the group consisting of SEQ ID NOs:1-10 and inhibits the expression of a gene that encodes SRPX2.

As used herein, "isolated" means altered or removed from the natural state through human intervention. For example, an siRNA naturally present in a living animal is not "isolated," but a synthetic siRNA, or an siRNA partially or completely separated from the coexisting materials of its natural state is "isolated." An isolated siRNA can exist in substantially purified form, or can exist in a non-native environment such as, for example, a cell into which the siRNA has been delivered.

Inhibitory nucleic acids are well known in the art. For example, siRNA and double-stranded RNA have been described in U.S. Patents 6,506,559 and 6,573,099, as well as in U.S. Patent Publications 2003/0051263, 2003/0055020, 2004/0265839, 2002/0168707, 2003/0159161, and 2004/0064842.

### B. Preparation of SRPX2 siRNA

Since the discovery of RNAi by Fire and colleagues in 1998, the biochemical mechanisms have been rapidly characterized. Long double stranded RNA (dsRNA) is cleaved by Dicer, which is an RNAase III family ribonuclease. This process yields siRNAs of ∼21 nucleotides in length. These siRNAs are incorporated into a multiprotcin RNA-induccd silencing complex (RISC) that is guided to target mRNA. RISC cleaves the target mRNA in the middle of the complementary region. In mammalian cells, the related microRNAs (miRNAs) are found that are short RNA fragments (∼22 nucleotides). MiRNAs are generated after Dicer-mediated cleavage of longer (∼70 nucleotide) precursors with imperfect hairpin RNA structures. The miRNA is incorporated into a miRNA-protein complex (miRNP), which leads to translational repression of target mRNA.

In designing RNAi there are several factors that need to be considered such as the nature of the siRNA, the durability of the silencing effect, and the choice of delivery system. To produce an RNAi effect, the siRNA that is introduced into the organism will typically contain exonic sequences. Furthermore, the RNAi process is homology dependent, so the sequences must be carefully selected so as to maximize gene specificity, while minimizing the possibility of cross-interference between homologous, but not gene-specific sequences. Particularly the siRNA exhibits greater than 80, 85, 90, 95, 98% or even 100% identity between the sequence of the siRNA and a portion of SRPX2 nucleotide sequence. Sequences less than about 80% identical to the target gene are substantially less effective. Thus, the greater identity between the siRNA and the SRPX2 gene to be inhibited, the less likely expression of unrelated genes will be affected.

In addition, the size of the siRNA is an important consideration.

There are disclosed siRNA molecules that include at least about 19-25 nucleotides, and are able to modulate SRPX2 gene expression. In the context of the present disclosure, the siRNA is particularly less than 500, 200, 100, 50 or 25 nucleotides in length. More particularly, the siRNA is from about 19 nucleotides to about 25 nucleotides in length.

To improve the effectiveness of siRNA-mcdiatcd gene silencing, guidelines for selection of target sites on mRNA have been developed for optimal design of siRNA (Soutschek *et al.,* 2004; Wadhwa *et al.,* 2004). These strategies may allow for rational approaches for selecting siRNA sequences to achieve maximal gene knockdown. To facilitate the entry of siRNA into cells and tissues, a variety of vectors including plasmids and viral vectors such as adenovirus, lentivirus, and retrovirus have been used (Wadhwa *et al.,* 2004).

Within an inhibitory nucleic acid, the components of a nucleic acid need not be of the same type or homogenous throughout (*e.g*., an inhibitory nucleic acid may comprise a nucleotide and a nucleic acid or nucleotide analog). Typically, an inhibitory nucleic acid form a double-stranded structure; the double-stranded structure may result from two separate nucleic acids that are partially or completely complementary. As disclosed herein, the inhibitory nucleic acid may comprise only a single nucleic acid (polynucleotide) or nucleic acid analog and form a double-stranded structure by complementing with itself (*e.g.,* forming a hairpin loop). The double-stranded structure of the inhibitory nucleic acid may comprise 16 - 500 or more contiguous nucleobases, including all ranges therebetween. The inhibitory nucleic acid may comprise 17 to 35 contiguous nucleobases, more particularly 18 to 30 contiguous nucleobases, more particularly 19 to 25 nucleobases, more particularly 20 to 23 contiguous nucleobases, or 20 to 22 contiguous nucleobases, or 21 contiguous nucleobases that hybridize with a complementary nucleic acid (which may be another part of the same nucleic acid or a separate complementary nucleic acid) to form a double-stranded structure.

siRNA can be obtained from commercial sources, natural sources, or can be synthesized using any of a number of techniques well-known to those of ordinary skill in the art. For example, commercial sources of predesigned siRNA include Invitrogen's Stealth™ Select technology (Carlsbad, CA), Ambion®(Austin, TX),and Qiagen® (Valencia, CA). An inhibitory nucleic acid that can be applied in the compositions and methods of the present disclosure may be any nucleic acid sequence that has been found by any source to be a validated downregulator of a SRPX2.

There is generally disclosed an isolated siRNA molecule of at least 19 nucleotides, having at least one strand that is substantially complementary to at least ten but no more than thirty consecutive nucleotides of a nucleic acid that encodes SRPX2, and that reduces the expression of SRPX2. In a particular embodiment of the present disclosure the siRNA molecule has at least one strand that is substantially complementary to at least ten but no more than thirty consecutive nucleotides of the mRNA that encodes SRPX2.

It is also disclosed that the siRNA molecule may be at least 75, 80, 85, or 90% homologous, particularly at least 95%, 99%, or 100% similar or identical, or any percentages in between the foregoing (e.g., the disclosure contemplates 75% and greater, 80% and greater, 85% and greater, and so on, and said ranges are intended to include all whole numbers in between), to at least 10 contiguous nucleotides of any of the nucleic acid sequences encoding a full-length SRPX2 protein. Generally speaking, it is preferred that the sequence must only be sufficiently similar to permit the siRNA molecule to bind to the SRPX2 mRNA target intracellularly, form an RISC complex, and thereby effect downregulation of expression.

Specific examples of siRNA sequences may preferably comprise one or more sequences selected from the group consisting of SEQ ID NO:11, SEQ ID NO:12 and SEQ ID NO:13, comprises SEQ ID NO:11 and SEQ ID NO:12, comprises SEQ ID NO:12 and SEQ ID NO:13, or comprise SEQ ID NO:11 and SEQ ID NO:13.

The siRNA may also comprise an alteration of one or more nucleotides. Such alterations can include the addition of non-nucleotide material, such as to the end(s) of the 19 to 25 nucleotide RNA or internally (at one or more nucleotides of the RNA). It is also disclosed that the RNA molecule may contain a 3'-hydroxyl group. Nucleotides in the RNA molecules as disclosed herein can also comprise non-standard nucleotides, including non-naturally occurring nucleotides or deoxyribonucleotides. The double-stranded oligonucleotide may contain a modified backbone, for example, phosphorothioate, phosphorodithioate, or other modified backbones known in the art, or may contain non-natural internucleoside linkages. Additional modifications of siRNAs (*e.g.,* 2'-O-methyl ribonucleotides, 2'-deoxy-2'-fluoro ribonucleotides, "universal base" nucleotides, 5-C-methyl nucleotides, one or more phosphorothioate internucleotide linkages, and inverted deoxyabasic residue incorporation) can be found in U.S. Publication 2004/0019001 and U.S. Patent 6,673,611. Collectively, all such altered nucleic acids or RNAs described above are referred to as modified siRNAs.

Particularly, RNAi is capable of decreasing the expression of SRPX2 by at least 10%, 20%, 30%, or 40%, more particularly by at least 50%, 60%, or 70%, and most particularly by at least 75%, 80%, 90%, 95% or more or any ranges in between the foregoing.

There are also disclosed methods of inhibiting expression of a gene encoding SRPX2 in a cell by introduction of inhibitory nucleic acids into the cell. Introduction of siRNA into cells can be achieved by methods known in the art, including for example, microinjection, electroporation, or transfection of a vector comprising a nucleic acid from which the siRNA can be transcribed. Alternatively, a siRNA can be directly introduced into a cell in a form that is capable of binding to target SRPX2 mRNA transcripts. To increase durability and membrane-permeability the siRNA may be combined or modified with liposomes, poly-L-lysine, lipids, cholesterol, lipofectine or derivatives thereof. In certain aspects cholesterol-conjugated siRNA can be used (see, Song *et al.,* 2003).

### C. Hybridization

As used herein, "hybridization", "hybridizes" or "capable of hybridizing" is understood to mean the forming of a double or triple stranded molecule or a molecule with partial double or triple stranded nature. The term "anneal" or "bind" as used herein is synonymous with "hybridize." Preferably, hybridization encompasses intracellular conditions, *i.e.*, inhibitory nucleic acids hybridize with SRPX2 nucleotide sequences in a cell or under intracellular conditions, preferably, an angiogenic cell, and more preferably, an angiogenic cell in a subject in need of angiogcncsis treatment. Intracellular conditions refer to conditions such as temperature, pH and salt concentrations typically found inside a cell, *e.g.,* a mammalian cell, which are well known to those of ordinary skill in the art.

The term "hybridization", "hybridize(s)" or "capable of hybridizing" also encompasses the terms "stringent conditions)" or "high stringency" and the terms "low stringency" or "low stringency condition(s)." A polynucleotide which hybridizes under an intracellular condition as disclosed herein may for example be a polynucleotide which hybridizes under a stringent condition described below.

As used herein "stringent condition(s)" or "high stringency" are those conditions that allow hybridization between or within one or more nucleic acid strand(s) containing complementary sequence(s), but precludes hybridization of random sequences. Stringent conditions tolerate little, if any, mismatch between a nucleic acid and a target strand. Such conditions are well known to those of ordinary skill in the art, and are particularly for applications requiring high selectivity. Non-limiting applications include isolating a nucleic acid, such as a gene or a nucleic acid segment thereof, or detecting at least one specific mRNA transcript or a nucleic acid segment thereof, and the like.

The stringent condition may for example be a condition involving 2 X SSC, 1 X Denhart's solution at about 60°C. Stringent conditions may comprise low salt and/or high temperature conditions, such as provided by about 0.02 M to about 0.15 M NaCl at temperatures of about 50°C to about 70°C. It is understood that the temperature and ionic strength of a desired stringency are determined in part by the length of the particular nucleic acid(s), the length and nucleobase content of the target sequence(s), the charge composition of the nucleic acid(s), and to the presence or concentration of formamide, tetramethylammonium chloride or other solvents) in a hybridization mixture.

It is also understood that these ranges, compositions and conditions for hybridization are mentioned by way of non-limiting examples only, and that the desired stringency for a particular hybridization reaction is often determined empirically by comparison to one or more positive or negative controls. Depending on the application envisioned it is particular to employ varying conditions of hybridization to achieve varying degrees of selectivity of a nucleic acid towards a target sequence. In a non-limiting example, identification or isolation of a related target nucleic acid that does not hybridize to a nucleic acid under stringent conditions may be achieved by hybridization at low temperature and/or high ionic strength. Such conditions are termed "low stringency" or "low stringency conditions", and non-limiting examples of low stringency include hybridization performed at about 0.15 M to about 0.9 M NaCl at a temperature range of about 20°C to about 50°C. Of course, it is within the skill of one in the art to further modify the low or high stringency conditions to suit a particular application.

### IV. Therapeutic Antibodies

In certain embodiments, an antibody or a fragment thereof that binds to at least a portion of SRPX2 protein and inhibits SRPX2 activity in angiogenesis and its associated use in treatment of diseases are contemplated. In some embodiments, the anti-SRPX2 antibody is a monoclonal antibody or a polyclonal antibody. In some embodiments, the antibody is selected from the group consisting of a chimeric antibody, an affinity matured antibody, a humanized antibody, and a human antibody. In some embodiments, the antibody is an antibody fragment. In some embodiments, the antibody is a Fab, Fab', Fab'-SH, F(ab')₂, or scFv. In one embodiment, the antibody is a chimeric antibody, for example, an antibody comprising antigen binding sequences from a non-human donor grafted to a heterologous non-human, human or humanized sequence (*e.g*., framework and/or constant domain sequences). In one embodiment, the non-human donor is a mouse. In one embodiment, an antigen binding sequence is synthetic, *e.g.,* obtained by mutagenesis (*e.g.,* phage display screening, *etc.*)*.* In one embodiment, a chimeric antibody of the invention has murine V regions and human C region. In one embodiment, the murine light chain V region is fused to a human kappa light chain or a human IgG1 C region.

Examples of binding fragments suitable for the present invention include, without limitation: (i) the Fab fragment, consisting of VL, VH, CL and CH1 domains; (ii) the "Fd" fragment consisting of the VH and CH1 domains; (iii) the "Fv" fragment consisting of the VL and VH domains of a single antibody; (iv) the "dAb" fragment, which consists of a VH domain; (v) isolated CDR regions; (vi) F(ab')2 fragments, a bivalent fragment comprising two linked Fab fragments; (vii) single chain Fv molecules ("scFv"), wherein a VH domain and a VL domain are linked by a peptide linker which allows the two domains to associate to form a binding domain; (viii) bi-specific single chain Fv dimers (see U.S. Pat. No. 5,091,513) and (ix) diabodies, multivalent or multispecific fragments constructed by gene fusion (US Patent Pub. 2005/0214860). Fv, scFv or diabody molecules may be stabilized by the incorporation of disulphide bridges linking the VH and VL domains. Minibodies comprising a scFv joined to a CH3 domain may also be made (Hu *et al,* 1996).

SRPX2 nucleotide sequences (SEQ ID NOs: 1-10) may be used to produce recombinant proteins and peptides as well known to people skilled in the art or as described in detail in the next section. For example, such sequences could be engineered into a suitable expression system, *e.g.*, yeast, insect cells or mammalian cells, for production of an SRPX2 protein or peptide comprising at least 10 amino acids having at least 95% identity to an amino acid sequence selected from the group consisting of SEQ ID NOs: 14-23.

Animals may be inoculated with an antigen, such as a SRPX2 protein or peptide, in order to produce antibodies specific for an SRPX2 protein or peptides having a sequence selected from the group consisting of SEQ ID NOs: 14-23. Frequently an antigen is bound or conjugated to another molecule to enhance the immune response. As used herein, a conjugate is any peptide, polypeptide, protein or non-proteinaceous substance bound to an antigen that is used to elicit an immune response in an animal.

Antibodies produced in an animal in response to antigen inoculation comprise a variety of non-identical molecules (polyclonal antibodies) made from a variety of individual antibody producing B lymphocytes. A polyclonal antibody is a mixed population of antibody species, each of which may recognize a different epitope on the same antigen. Given the correct conditions for polyclonal antibody production in an animal, most of the antibodies in the animal's serum will recognize the collective epitopes on the antigenic compound to which the animal has been immunized. This specificity is further enhanced by affinity purification to select only those antibodies that recognize the antigen or epitope of interest.

A monoclonal antibody is a single species of antibody wherein every antibody molecule recognizes the same epitope because all antibody producing cells are derived from a single B-lymphocyte cell line. Hybridoma technology involves the fusion of a single B lymphocyte from a mouse previously immunized with a SRPX2 antigen with an immortal myeloma cell (usually mouse myeloma). This technology provides a method to propagate a single antibody-producing cell for an indefinite number of generations, such that unlimited quantities of structurally identical antibodies having the same antigen or epitope specificity (monoclonal antibodies) may be produced. However, in therapeutic applications a goal of hybridoma technology is to reduce the immune reaction in humans that may result from administration of monoclonal antibodies generated by the non-human (e.g. mouse) hybridoma cell line.

Methods have been developed to replace light and heavy chain constant domains of the monoclonal antibody with analogous domains of human origin, leaving the variable regions of the foreign antibody intact. Alternatively, "fully human" monoclonal antibodies are produced in mice transgenic for human immunoglobulin genes. Methods have also been developed to convert variable domains of monoclonal antibodies to more human form by recombinantly constructing antibody variable domains having both rodent and human amino acid sequences. In "humanized" monoclonal antibodies, only the hypervariable CDR is derived from mouse monoclonal antibodies, and the framework regions are derived from human amino acid sequences. It is thought that replacing amino acid sequences in the antibody that are characteristic of rodents with amino acid sequences found in the corresponding position of human antibodies will reduce the likelihood of adverse immune reaction during therapeutic use. A hybridoma or other cell producing an antibody may also be subject to genetic mutation or other changes, which may or may not alter the binding specificity of antibodies produced by the hybridoma.

It is possible to create engineered antibodies, using monoclonal and other antibodies and recombinant DNA technology to produce other antibodies or chimeric molecules which retain the antigen or epitope specificity of the original antibody, *i.e.,* the molecule has a binding domain. Such techniques may involve introducing DNA encoding the immunoglobulin variable region or the CDRs of an antibody to the genetic material for the framework regions, constant regions, or constant regions plus framework regions, of a different antibody. See, for instance, U.S. Pat. Nos. 5,091,513, and 6,881,557.

By known means as described herein, polyclonal or monoclonal antibodies, binding fragments and binding domains and CDRs (including engineered forms of any of the foregoing), may be created that are specific to SRPX2 protein, one or more of its respective epitopes, or conjugates of any of the foregoing, whether such antigens or epitopes are isolated from natural sources or are synthetic derivatives or variants of the natural compounds.

Antibodies may be produced from any animal source, including birds and mammals. Preferably, the antibodies are ovine, murine (e.g., mouse and rat), rabbit, goat, guinea pig, camel, horse, or chicken. In addition, newer technology permits the development of and screening for human antibodies from human combinatorial antibody libraries. For example, bacteriophage antibody expression technology allows specific antibodies to be produced in the absence of animal immunization, as described in U.S. Pat. No. 6,946,546.
These techniques are further described in: Marks (1992); Stemmer (1994); Gram *et al.* (1992); Barbas *et al.* (1994); and Schier *et al.* (1996).

Methods for producing polyclonal antibodies in various animal species, as well as for producing monoclonal antibodies of various types, including humanized, chimeric, and fully human, are well known in the art and highly predictable. Methods for producing these antibodies are also well known and predictable. For example, the following U.S. patents and patent publications provide enabling descriptions of such methods: U.S. Patent publication Nos. 2004/0126828 and 2002/0172677; and U.S. Pat. Nos. 3,817,837; 3,850,752; 3,939,350; 3,996,345; 4,196,265; 4,275,149; 4,277,437; 4,366,241; 4,469,797; 4,472,509; 4,606,855; 4,703,003; 4,742,159; 4,767,720; 4,816,567; 4,867,973; 4,938,948; 4,946,778; 5,021,236; 5,164,296; 5,196,066; 5,223,409; 5,403,484; 5,420,253; 5,565,332; 5,571,698; 5,627,052; 5,656,434; 5,770,376; 5,789,208; 5,821,337; 5,844,091; 5,858,657; 5,861,155; 5,871,907; 5,969,108; 6,054,297; 6,165,464; 6,365,157; 6,406,867; 6,709,659; 6,709,873; 6,753,407; 6,814,965; 6,849,259; 6,861,572; 6,875,434; and 6,891,024.

It is fully expected that antibodies to SRPX2 will have the ability to neutralize or counteract the cffccts of the SRPX2 regardless of the animal species, monoclonal cell line or other source of the antibody. Certain animal species may be less preferable for generating therapeutic antibodies because they may be more likely to cause allergic response due to activation of the complement system through the "Fc" portion of the antibody. However, whole antibodies may be enzymatically digested into "Fc" (complement binding) fragment, and into binding fragments having the binding domain or CDR. Removal of the Fc portion reduces the likelihood that the antigen binding fragment will elicit an undesirable immunological response and, thus, antibodies without Fc may be preferential for prophylactic or therapeutic treatments. As described above, antibodies may also be constructed so as to be chimeric, partially or fully human, so as to reduce or eliminate the adverse immunological consequences resulting from administering to an animal an antibody that has been produced in, or has sequences from, other species.

### V. SRPX2 protein or peptides

There is also disclosed a pharmaceutical composition for inducing or promoting angiogenesis comprising an SRPX2 full-length protein, or a peptide or polypeptide derived there from. SEQ ID NO:14 shows the translated product of SEQ ID NO:1 (cDNA of human SRPX2). It is contemplated that the compositions and methods disclosed herein may be utilized to express all or part of sequences selected from the group consisting of SEQ ID NOs:14-23 and derivatives thereof, particularly the human SRPX2 protein as depicted in SEQ ID NO:14. Determination of which protein or DNA molecules induce angiogenesis may be achieved using functional assays, such as measuring wound healing, which are familiar to those of skill in the art. The structure of the various polypeptides or peptides can be modeled or resolved by computer modeling, NMR, or x-ray crystallography. Such structures may be used to engineer derivatives of the various SRPX2 protein.

### A. Variants of SRPX2 Polypeptides

There are also disclosed various SRPX2 polypeptides, peptides, and derivatives thereof. The term "biologically functional equivalent" is well understood in the art and is further defined in detail herein. Accordingly, SRPX2 polypeptides or peptides include sequences that have between about 70% and about 80%; or more preferably, between about 81% and about 90%; or even more preferably, between about 91% and about 99%; or even more preferably, between about 95% and about 99%; of amino acids that are identical or functionally equivalent to the amino acids of SRPX2 polypeptides selected from the group consisting of SEQ ID NO: 14-23, provided the biological activity of the protein or peptide is maintained.

The term "functionally equivalent codon" is used herein to refer to codons that encode the same amino acid, such as the six codons for arginine or serine, and also refers to codons that encode biologically equivalent amino acids as well known to people in the art.

There are also disclosed various peptides or polypeptides of the SRPX2 protein. For example, all or part of a SRPX2 protein as set forth in SEQ ID NOs:14-23 may be used in the context of the present disclosure. As disclosed herein, a fragment of the SRPX2 protein or a SRPX2 peptide may comprise, but is not limited to at least 10, 12, 15, 20, 25, 100 amino acids and any range derivable therein.

It also will be understood that amino acid and nucleic acid sequences may include additional residues, such as additional N- or C-terminal amino acids or 5' or 3' sequences, and yet still be essentially as set forth in one of the sequences disclosed herein, so long as the sequence meets the criteria set forth above, including the maintenance of biological activity *(e.g.,* pro-angiogenesis activity) where protein expression is concerned. The addition of terminal sequences particularly applies to nucleic acid sequences that may, for example, include various non-coding sequences flanking either of the 5' or 3' portions of the coding region.

The following is a discussion based upon changing of the amino acids of an SRPX2 polypeptide or peptide to create an equivalent, or even an improved, second-generation molecule. For example, certain amino acids may be substituted for other amino acids in a protein structure without appreciable loss of interactive binding capacity with structures such as, for example, antigen-binding regions of antibodies or binding sites on substrate molcculcs. Since it is the interactive capacity and nature of a protein that defines that protein's biological functional activity, certain amino acid substitutions can be made in a protein sequence, and in its underlying DNA or RNA coding sequence, and nevertheless produce a protein with like properties. It is thus contemplated by the authors that various changes may be made in the DNA or RNA sequences of genes or coding regions without appreciable loss of their biological utility or activity, as discussed herein.

It is also disclosed that an SRPX2 polypeptide may be a fusion protein. Fusion proteins may alter the characteristics of a given polypeptide, such cellular uptake and/or permeability, antigenicity or purification characteristics. A fusion protein is a specialized type of insertional variant. This molecule generally has all or a substantial portion of the native molecule or peptide, linked at the N- or C-terminus, to all or a portion of a second polypeptide. For example, fusions typically employ leader or targeting sequences from other species to permit the recombinant expression of a protein in a heterologous host. Another useful fusion includes the addition of an immunologically active domain, such as an antibody epitope, to facilitate purification of the fusion protein. Inclusion of a cleavage site at or near the fusion junction will facilitate removal of the extraneous polypeptide after purification. Other useful fusions include linking of functional domains, such as active sites from enzymes such as a hydrolase, glycosylation domains, cellular targeting signals, or transmembrane regions.

### B. Peptides

In this disclosure, the products as disclosed herein are referred to by various terms, including "analogs," "mimetics," "peptidomimetics," and "derivatives." These terms are used interchangeably and denote equivalent compounds. Mimetics as disclosed herein comprise a structure which comprises a sequence or mimics the structure of a sequence set forth as SEQ ID NOs:14-23, and thus may comprise additional elements such as R-group substituents and a linker selected from the possibilities set forth in the instant invention.

As defined, biological activity refers to the biological activity of SRPX2 and its segments, for example, the novel activity in angiogenesis discovered by the inventors.

Mimetics as disclosed herein may include peptide derivatives or peptide analogs and their derivatives, such as C-terminal hydroxymethyl derivatives, O-modified derivatives, N-terminally modified derivatives including substituted amides such as alkylamides and hydrazides and compounds in which a C-terminal residue is replaced with a phenethylamide analogue, glycosylated peptide derivatives, polyethylene glycol modified derivatives, or biotinylated derivatives. Peptide analogs as disclosed herein include pharmaceutically acceptable salts of an analog.

The peptide analogs as disclosed herein may be coupled directly or indirectly to at least one modifying group. It is also disclosed that the term "modifying group" is intended to include structures that are directly attached to the peptidic structure (e.g., by covalent bonding or covalent coupling), as well as those that are indirectly attached to the peptidic structure (e.g., by a stable non-covalent bond association or by covalent coupling through a linker to additional amino acid residues). It is also disclosed that the term "modifying group" may also refer to mimetics, analogues or derivatives thereof. Alternatively, the modifying group can be coupled to a side chain of at least one amino acid residue of a SRPX2 peptide, or a peptidic or a peptidomimetic. In other aspects, modifying groups covalently coupled to the peptidic structure can be attached by means and using methods well known in the art for linking chemical structures.

It is also disclosed that peptides and peptide analogs are designed by replacing all or part of a structural domain with a linker or a compound that mimic such structure. It is also disclosed that all or a portion of the amino-terminal domain and all or a portion of the carboxy-terminal domain of a peptide or peptide analog may be connected with a linker. The peptide and peptide analogs may also be designed so that there are cyclized by covalent modification between residues of the peptide. A peptide analog compound as disclosed herein may be further modified to alter the specific properties of the compound while retaining the desired functionality of the compound. For example, the compound may be modified to alter a pharmacokinetic property of the compound, such as *in vivo* stability, solubility, bioavailability or half-life. The compound may be modified to label the compound with a detectable substance. The compound may be modified to couple the compound to an additional therapeutic moiety. To further chemically modify the compound, such as to alter its pharmacokinetic properties, reactive groups can be derivatized.

In an alternative chemical modification, a peptide analog compound of the disclosure may be prepared in a "prodrug" form, wherein the compound itself does not act as a peptide analog agonist, but rather is capable of being transformed, upon metabolism *in vivo,* into a peptide analog agonist or antagonist compound.

Mimetics as disclosed herein may be prepared by standard techniques known in the art. A peptide or polypeptide component of an analog may comprise, at least in part, a peptide synthesized using standard techniques. Automated peptide synthesizers are commercially available. Peptides and polypeptides may be assayed for activity in accordance with methods exemplified herein. Peptides and polypeptides may be purified by HPLC and analyzed by mass spectrometry.

The analogs as disclosed herein include peptide or polypeptide sequences wherein one or more of the amino acids have been replaced by a conservative amino acid substitution. The term "conservative amino acid substitution" refers to a peptide chain in which one of the amino acid residues is replaced with an amino acid residue having a side chain with similar properties. Families of amino acid residues having side chains with similar properties are well known in the art. These families include amino acids with acidic side chains (*e.g*., aspartic acid, glutamic acid), basic side chains (*e.g.,* lysine, arginine, histidine), uncharged polar side chains (*e.g.,* glycine, asparagine, glutamine, serine, threonine, tyrosine, cysteine), nonpolar side chains (*e.g*., alanine, valine, leucine, isoleucine, proline, phenylalanine, methionine, tryptophan), beta-branched side chains (*e.g*., threonine, valine, isoleucine) and aromatic side chains (*e.g*., tyrosine, phenylalanine, tryptophan, histidine).

### C. In vitro Production of SRPX2 Polypeptides or Peptides

Various types of expression vectors are known in the art that can be used for the production of protein or peptide products. For example, following transfection with a expression vector comprising a coding sequence selected from the group consisting of SEQ ID NOs:1-10 to a cell in culture, *e.g.,* a primary mammalian cell, a recombinant SRPX2 protein product may be prepared in various ways. A host cell strain may be chosen that modulates the expression of the inserted sequences, or that modifies and processes the gene product in the manner desired. Such modifications *(e.g.,* glycosylation) and processing *(e.g.,* cleavage) of protein products may be important for the function of the protein. Different host cells have characteristic and specific mechanisms for the post-translational processing and modification of proteins. Appropriate cell lines or host systems can be chosen to insure the correct modification and processing of the foreign protein expressed. In order for the cells to be kept viable while *in vitro* and in contact with the expression construct, it is necessary to ensure that the cells maintain contact with the correct ratio of oxygen and carbon dioxide and nutrients but are protected from microbial contamination.

Animal cells can be propagated *in vitro* in two modes: as non-anchorage-dependent cells growing in suspension throughout the bulk of the culture or as anchorage-dependent cells requiring attachment to a solid substrate for their propagation (*i.e.,* a monolayer type of cell growth).

Non-anchorage dependent or suspension cultures from continuous established cell lines are the most widely used means of large-scale production of cells and cell products. However, suspension cultured cells have limitations, such as tumorigenic potential and lower protein production than adherent cells.

As disclosed herein, other protein production methods known in the art may be used, including but not limited to prokaryotic, yeast, and other eukaryotic hosts such as insect cells and the like.

Because of their relatively small size, the SRPX2 peptides as disclosed herein can also be synthesized in solution or on a solid support in accordance with conventional techniques. Various automatic synthesizers are commercially available and can be used in accordance with known protocols. See, for example, Stewart and Young, (1984); Tam *et al.,* (1983); and Barany and Merrifield (1979).
Short peptide sequences can be readily synthesized and then screened in screening assays designed to identify biologically functional equivalent peptides.

### D. Protein Purification

It may be desirable to purify or isolate SRPX2 polypeptides and peptides, or variants and derivatives thereof. Protein purification techniques arc well known to those of skill in the art. These techniques involve, at one level, the crude fractionation of the cellular milieu to polypeptide and non-polypeptide fractions. Having separated the SRPX2 polypeptide from other proteins, the polypeptide of interest may be further purified using chromatographic and electrophoretic techniques to achieve partial or complete purification (or purification to homogeneity). Analytical methods particularly suited to the preparation of a pure peptide are ion-exchange chromatography, hydrophobic interaction chromatography, exclusion chromatography; polyacrylamide gel electrophoresis; isoelectric focusing. A particularly efficient method of purifying peptides is fast protein liquid chromatography (FPLC).

There is also disclosed the purification, and in particular embodiments, the substantial purification, of an encoded protein or peptide. The term "isolated or purified protein or peptide" as used herein, is intended to refer to a composition, isolatable from other components, wherein the protein or peptide is purified to any degree relative to its naturally obtainable state. A isolated or purified protein or peptide therefore also refers to a protein or peptide, free from the environment in which it may naturally occur.

Generally, "isolated or purified" will refer to a protein or peptide composition that has been subjected to fractionation to remove various other components, and which composition substantially retains its expressed biological activity. Where the term "substantially purified" is used, this designation will refer to a composition in which the protein or peptide forms the major component of the composition, such as constituting about 50%, about 60%, about 70%, about 80%, about 90%, about 95% or more of the proteins in the composition.

Various methods for quantifying the degree of purification of the protein or peptide will be known to those of skill in the art in light of the present disclosure. These include, for example, determining the specific activity of an active fraction, or assessing the amount of polypeptides within a fraction by SDS/PAGE analysis. A preferred method for assessing the purity of a fraction is to calculate the specific activity of the fraction, to compare it to the specific activity of the initial extract, and to thus calculate the degree of purity, herein assessed by a "-fold purification number." The actual units used to represent the amount of activity will, of course, be dependent upon the particular assay technique chosen to follow the purification and whether or not the expressed protein or peptide exhibits a detectable activity.

There is no general requirement that the protein or peptide always be provided in their most purified state. Indeed, it is contemplated that less substantially purified products will have utility in certain embodiments. Partial purification may be accomplished by using fewer purification steps in combination, or by utilizing different forms of the same general purification scheme.

### VI. Lipid Preparations

There are also disclosed methods and compositions for associating an inhibitory nucleic acid that inhibits the expression of SRPX2, such as an siRNA, or an inhibitory antibody or a fragment thereof, or an SRPX2 protein or peptide, with a lipid and/or liposome. The inhibitory nucleic acid may be encapsulated in the aqueous interior of a liposome, interspersed within the lipid bilayer of a liposome, attached to a liposome via a linking molecule that is associated with both the liposome and the polynucleotide, entrapped in a liposome, complexed with a liposome, dispersed in a solution containing a lipid, mixed with a lipid, combined with a lipid, contained as a suspension in a lipid, contained or complexed with a micelle, or otherwise associated with a lipid. The liposome or liposome/siRNA associated compositions are not limited to any particular structure in solution. For example, they may be present in a bilayer structure, as micelles, or with a "collapsed" structure. They may also simply be interspersed in a solution, possibly forming aggregates which are not uniform in either size or shape.

Lipids are fatty substances which may be naturally occurring or synthetic lipids. For example, lipids include the fatty droplets that naturally occur in the cytoplasm as well as the class of compounds which are well known to those of skill in the art which contain long-chain aliphatic hydrocarbons and their derivatives, such as fatty acids, alcohols, amines, amino alcohols, and aldehydes. An example is the lipid dioleoylphosphatidylcholine (DOPC).

"Liposome" is a generic term encompassing a variety of unilamellar, multilamellar, and multivesicular lipid vehicles formed by the generation of enclosed lipid bilayers or aggregates. Liposomes may be characterized as having vesicular structures with a phospholipid bilayer membrane and an inner aqueous medium. Multilamellar liposomes have multiple lipid layers separated by aqueous medium. They form spontaneously when phospholipids are suspended in an excess of aqueous solution. The lipid components undergo self-rearrangement before the formation of closed structures and entrap water and dissolved solutes between the lipid bilayers (Ghosh and Bachhawat, 1991). However, the present disclosure also encompasses compositions that have different structures in solution than the normal vesicular structure. For example, the lipids may assume a micellar structure or merely exist as non-uniform aggregates of lipid molecules. Also contemplated are lipofectamine-nucleic acid complexes.

Liposome-mediated polynucleotide delivery and expression of foreign DNA *in vitro* has been very successful. Wong *et al.* (1980) demonstrated the feasibility of liposome-mediated delivery and expression of foreign DNA in cultured chick embryo, HeLa and hepatoma cells. Nicolau *et al.* (1987) accomplished successful liposome-mediated gene transfer in rats after intravenous injection.

As disclosed herein, the lipid may be associated with a hemaglutinating virus (HVJ). This has been shown to facilitate fusion with the cell membrane and promote cell entry of liposome-encapsulated DNA (Kaneda *et al.,* 1989). It is also disclosed that the lipid may be complexed or employed in conjunction with nuclear non-histone chromosomal proteins (HMG-1) (Kato *et al.,* 1991). It is also disclosed that the lipid may be complexed or employed in conjunction with both HVJ and HMG-1. In that such expression vectors have been successfully employed in transfer of a polynucleotide in *vitro* and *in vivo,* then they are applicable in the context of the present disclosure.

Exemplary lipids include, diolcoylphosphatidylycholinc ("DOPC"), egg phosphatidylcholine ("EPC"), dilauryloylphosphatidylcholine ("DLPC"), dimyristoylphosphatidylcholine ("DMPC"), dipalmitoylphosphatidylcholine ("DPPC"), distearoylphosphatidylcholine ("DSPC"), 1-myristoyl-2-palmitoyl phosphatidylcholine ("MPPC"), 1-palmitoyl-2-myristoyl phosphatidylcholine ("PMPC"), 1-palmitoyl-2-stearoyl phosphatidylcholine ("PSPC"), 1-stearoyl-2-palmitoyl phosphatidylcholine ("SPPC"), dilauryloylphosphatidylglycerol ("DLPG"), dimyristoylphosphatidylglycerol ("DMPG"), dipalmitoylphosphatidylglycerol ("DPPG"), distearoylphosphatidylglycerol ("DSPG"), distearoyl sphingomyelin ("DSSP"), distearoylphophatidylethanolamine ("DSPE"), dioleoylphosphatidylglycerol ("DOPG"), dimyristoyl phosphatidic acid ("DMPA"), dipalmitoyl phosphatidic acid ("DPPA"), dimyristoyl phosphatidylethanolamine ("DMPE"), dipalmitoyl phosphatidylethanolamine ("DPPE"), dimyristoyl phosphatidylserine ("DMPS"), dipalmitoyl phosphatidylserine ("DPPS"), brain phosphatidylserine ("BPS"), brain sphingomyelin ("BSP"), dipalmitoyl sphingomyelin ("DPSP"), dimyristyl phosphatidylcholine ("DMPC"), 1,2-distearoyl-sn-glycero-3-phosphocholine ("DAPC"), 1,2-diarachidoyl-sn-glycero-3-phosphocholine ("DBPC"), 1,2-dicicosenoyl-sn-glycero-3-phosphocholine ("DEPC"), dioleoylphosphatidylethanolamine ("DOPE"), palmitoyloeoyl phosphatidylcholine ("POPC"), palmitoyloeoyl phosphatidylethanolamine ("POPE"), lysophosphatidylcholine, lysophosphatidylethanolamine, dilinoleoylphosphatidylcholine, phosphatidylcholines, phosphatidylglycerols, phosphatidylethanolamines, cholesterol.

Liposomes and lipid compositions of the present disclosure can be made by different methods. For example, a nucleotide (*e.g.*, siRNA) may be encapsulated in a neutral liposome using a method involving ethanol and calcium (Bailey and Sullivan, 2000). The size of the liposomes varies depending on the method of synthesis. A liposome suspended in an aqueous solution is generally in the shape of a spherical vesicle, and may have one or more concentric layers of lipid bilayer molecules. Each layer consists of a parallel array of molecules represented by the formula XY, wherein X is a hydrophilic moiety and Y is a hydrophobic moiety. In aqueous suspension, the concentric layers are arranged such that the hydrophilic moieties tend to remain in contact with an aqueous phase and the hydrophobic regions tend to self-associate. For example, when aqueous phases are present both within and without the liposome, the lipid molecules may form a bilayer, known as a lamella, of the arrangement XY-YX. Aggregates of lipids may form when the hydrophilic and hydrophobic parts of more than one lipid molecule become associated with each other. The size and shape of these aggregates will depend upon many different variables, such as the nature of the solvent and the presence of other compounds in the solution.

Lipids suitable for use according to the present disclosure can be obtained from commercial sources. For example, dimyristyl phosphatidylcholine ("DMPC") can be obtained from Sigma Chemical Co., dicetyl phosphate ("DCP") can be obtained from K & K Laboratories (Plainview, N.Y.); cholesterol ("Chol") can be obtained from Calbiochem-Behring; dimyristyl phosphatidylglycerol ("DMPG") and other lipids may be obtained from Avanti Polar Lipids, Inc. (Birmingham, Ala.). Stock solutions of lipids in chloroform or chloroform/methanol can be stored at about -20°C. Chloroform may be used as the only solvent since it is more readily evaporated than methanol.

Liposomes of the present disclosure can be prepared in accordance with known laboratory techniques. In certain embodiments, liposomes are prepared by mixing liposomal lipids, in a solvent in a container (e.g., a glass, pear-shaped flask). The container will typically have a volume ten-times greater than the volume of the expected suspension of liposomes. Using a rotary evaporator, the solvent may be removed at approximately 40°C under negative pressure. The solvent may be removed within about 5 minutes to 2 hours, depending on the desired volume of the liposomes. The composition can be dried further in a desiccator under vacuum. Dried lipids can be hydrated at approximately 25-50 mM phospholipid in sterile, pyrogen-free water by shaking until all the lipid film is resuspended. The aqueous liposomes can be then separated into aliquots, each placed in a vial, lyophilized and sealed under vacuum.

Liposomes can also be prepared in accordance with other known laboratory procedures: the method of Bangham *et al.* (1965); the method of Gregoriadis (1979), the method of Deamer and Uster (1983), and the reverse-phase evaporation method as described by Szoka and Papahadjopoulos (1978). The aforementioned methods differ in their respective abilities to entrap aqueous material and their respective aqueous space-to-lipid ratios.

Dried lipids or lyophilized liposomes may be dehydrated and reconstituted in a solution of inhibitory peptide and diluted to an appropriate concentration with a suitable solvent (e.g., DPBS). The mixture may then be vigorously shaken in a vortex mixer. Unencapsulated nucleic acid may be removed by centrifugation at 29,000 g and the liposomal pellets washed. The washed liposomes may be resuspended at an appropriate total phospholipid concentration (e.g., about 50-200 mM). The amount of nucleic acid encapsulated can be determined in accordance with standard methods. After determination of the amount of nucleic acid encapsulated in the liposome preparation, the liposomes may be diluted to appropriate concentrations and stored at 4°C until use.

### VII. Treatment of Diseases

The authors provide evidence that modulation of SRPX2 expression or activity may provide an effective tool for treatment of angiogenesis-related conditions or diseases for its novel function in promoting angiogenesis. Thus, compositions and methods involving SRPX2 and its regulators are contemplated in the present disclosure.

### A Definitions

"Treatment" and "treating" refer to administration or application of a therapeutic agent to a subject or performance of a procedure or modality on a subject for the purpose of obtaining a therapeutic benefit of a disease or health-related condition. For example, a treatment may include administration of a pharmaceutically effective amount of a nucleic acid that inhibits the expression of a gene that encodes a SRPX2 and a lipid for the purposes of minimizing the growth or invasion of a tumor, such as a colorectal cancer.

A "subject" refers to either a human or non-human, such as primates, mammals, and vertebrates. In particular embodiments, the subject is a human.

The term "therapeutic benefit" or "therapeutically effective" as used throughout this disclosure refers to anything that promotes or enhances the well-being of the subject with respect to the medical treatment of this condition. This includes, but is not limited to, a reduction in the frequency or severity of the signs or symptoms of a disease. For example, treatment of cancer may involve, for example, a reduction in the size of a tumor, a reduction in the invasiveness of a tumor, reduction in the growth rate of the cancer, or prevention of metastasis. Treatment of cancer may also refer to prolonging survival of a subject with cancer.

### B. Diseases

The present invention may be used to treat any condition or disease associated with increased or decreased expression of a SRPX2. For example, the disease may be an angiogenesis-related condition or disease. Angiogenesis-related condition or disease is a consequence of an imbalanced angiogenic process resulting in an excessive amount of new blood vessels or insufficient number of blood vessels.

It is disclosed that the present methods can be used to inhibit angiogenesis which is non-pathogenic; i.e., angiogenesis which results from normal processes in the subject. Examples of non-pathogenic angiogenesis include endometrial neovascularization, and processes involved in the production of fatty tissues or cholesterol. Thus, there is disclosed a method for inhibiting non-pathogenic angiogenesis, *e.g*., for controlling weight or promoting fat loss, for reducing cholesterol levels, or as an abortifacient.

The present methods can also inhibit angiogenesis which is associated with an angiogenic disease; i.e., a disease in which pathogenicity is associated with inappropriate or uncontrolled angiogenesis. For example, most cancerous solid tumors generate an adequate blood supply for themselves by inducing angiogenesis in and around the tumor site. This tumor-induced angiogenesis is often required for tumor growth, and also allows metastatic cells to enter the bloodstream.

Other angiogenic diseases include diabetic retinopathy, age-related macular degeneration (ARMD), psoriasis, rheumatoid arthritis and other inflammatory diseases. These diseases are characterized by the destruction of normal tissue by newly formed blood vessels in the area of neovascularization. For example, in ARMD, the choroid is invaded and destroyed by capillaries. The angiogenesis-driven destruction of the choroid in ARMD eventually leads to partial or full blindness. The angiogenesis-related conditions also include ocular neovascularization, arterio-venous malformations, coronary restenosis, peripheral vessel restenosis, glomerulonephritis, rheumatoid arthritis, ischemic cardiovascular pathologies, or chronic inflammatory diseases.

Exemplary eye angiogenic diseases to be treated or prevented also include choroidal neovascularization (CNV) due to any cause including age-related macular degeneration, ocular histoplasmosis, pathologic myopia, and angioid streaks. It also applies to retinal neovascularization of any cause including proliferative diabetic retinopathy, retinal vein occlusions, and retinopathy of prematurity. It also applies to iris neovascularization and corneal neovascularization of any causes.

The neovascularization may also be neovascularization associated with an ocular wound. For example, the wound may the result of a traumatic injury to the globe, such as a corneal laceration. Alternatively, the wound may be the result of ophthalmic surgery. In some embodiments, the methods of the present disclosure may be applied to prevent or reduce the risk of proliferative vitreoretinopathy following vitreoretinal surgery, prevent corneal haze following corneal surgery (such as corneal transplantation and laser surgery), prevent closure of a trabeculectomy, prevent or substantially slow the recurrence of pterygii, and so forth.

The neovascularization may be located either on or within the eye of the subject. For example, the neovascularization may be corneal neovascularization (either located on the corneal epithelium or on the endothelial surface of the cornea), iris neovascularization, neovascularization within the vitreous cavity, retinal neovasculization, or choroidal neovascularization. The neovascularization may also be neovascularization associated with conjunctival disease.

Particulary, a siRNA that binds to a nucleic acid that encodes a SRPX2 may be administered to treat a cancer. The cancer may be a solid tumor, metastatic cancer, or non-metastatic cancer. In certain embodiments, the cancer may originate in the bladder, blood, bone, bone marrow, brain, breast, colon, esophagus, duodenum, small intestine, large intestine, colon, rectum, anus, gum, head, kidney, liver, lung, nasopharynx, neck, ovary, prostate, skin, stomach, testis, tongue, or uterus.

The cancer may specifically be of the following histological type: neoplasm, malignant; carcinoma; carcinoma, undifferentiated; giant and spindle cell carcinoma; small cell carcinoma; papillary carcinoma; squamous cell carcinoma; lymphoepithelial carcinoma; basal cell carcinoma; pilomatrix carcinoma; transitional cell carcinoma; papillary transitional cell carcinoma; adenocarcinoma; gastrinoma, malignant; cholangiocarcinoma; hepatocellular carcinoma; combined hepatocellular carcinoma and cholangiocarcinoma; trabecular adenocarcinoma; adenoid cystic carcinoma; adenocarcinoma in adenomatous polyp; adenocarcinoma, familial polyposis coli; solid carcinoma; carcinoid tumor, malignant; branchiolo-alveolar adenocarcinoma; papillary adenocarcinoma; chromophobe carcinoma; acidophil carcinoma; oxyphilic adenocarcinoma; basophil carcinoma; clear cell adenocarcinoma; granular cell carcinoma; follicular adenocarcinoma; papillary and follicular adenocarcinoma; nonencapsulating sclerosing carcinoma; adrenal cortical carcinoma; endometroid carcinoma; skin appendage carcinoma; apocrine adenocarcinoma; sebaceous adenocarcinoma; ceruminous adenocarcinoma; mucoepidermoid carcinoma; cystadenocarcinoma; papillary cystadenocarcinoma; papillary serous cystadenocarcinoma; mucinous cystadenocarcinoma; mucinous adenocarcinoma; signet ring cell carcinoma; infiltrating duct carcinoma; medullary carcinoma; lobular carcinoma; inflammatory carcinoma; paget's disease, mammary; acinar cell carcinoma; adenosquamous carcinoma; adenocarcinoma w/squamous metaplasia; thymoma, malignant; ovarian stromal tumor, malignant; thecoma, malignant; granulosa cell tumor, malignant; androblastoma, malignant; sertoli cell carcinoma; leydig cell tumor, malignant; lipid cell tumor, malignant; paraganglioma, malignant; extra-mammary paraganglioma, malignant; pheochromocytoma; glomangiosarcoma; malignant melanoma; amelanotic melanoma; superficial spreading melanoma; malignant melanoma in giant pigmented nevus; epithelioid cell melanoma; blue nevus, malignant; sarcoma; fibrosarcoma; fibrous histiocytoma, malignant; myxosarcoma; liposarcoma; leiomyosarcoma; rhabdomyosarcoma; embryonal rhabdomyosarcoma; alveolar rhabdomyosarcoma; stromal sarcoma; mixed tumor, malignant; mullerian mixed tumor; nephroblastoma; hepatoblastoma; carcinosarcoma; mesenchymoma, malignant; brenner tumor, malignant; phyllodes tumor, malignant; synovial sarcoma; mesothelioma, malignant; dysgerminoma; embryonal carcinoma; teratoma, malignant; struma ovarii, malignant; choriocarcinoma; mesonephroma, malignant; hemangiosarcoma; hemangioendothelioma, malignant; kaposi's sarcoma; hemangiopericytoma, malignant; lymphangiosarcoma; osteosarcoma; juxtacortical osteosarcoma; chondrosarcoma; chondroblastoma, malignant; mesenchymal chondrosarcoma; giant cell tumor of bone; ewing's sarcoma; odontogenic tumor, malignant; ameloblastic odontosarcoma; ameloblastoma, malignant; ameloblastic fibrosarcoma; pinealoma, malignant; chordoma; glioma, malignant; ependymoma; astrocytoma; protoplasmic astrocytoma; fibrillary astrocytoma; astroblastoma; glioblastoma; oligodendroglioma; oligodendroblastoma; primitive neuroectodermal; cerebellar sarcoma; ganglioneuroblastoma; neuroblastoma; retinoblastoma; olfactory neurogenic tumor; meningioma, malignant; neurofibrosarcoma; neurilemmoma, malignant; granular cell tumor, malignant; malignant lymphoma; hodgkin's disease; hodgkin's; paragranuloma; malignant lymphoma, small lymphocytic; malignant lymphoma, large cell, diffuse; malignant lymphoma, follicular; mycosis fungoides; other specified non-hodgkin's lymphomas; malignant histiocytosis; multiple myeloma; mast cell sarcoma; immunoproliferative small intestinal disease; leukemia; lymphoid leukemia; plasma cell leukemia; erythroleukemia; lymphosarcoma cell leukemia; mast cell leukemia; megakaryoblastic leukemia; myeloid sarcoma; and hairy cell leukemia. Nonetheless, it is also recognized that the present disclosure may also be used to treat a non-cancerous disease *(e.g.,* a fungal infection, a bacterial infection, a viral infection, and/or a neurodegenerative disease). As disclosed herein, SRPX2 protein or peptide is contemplated to treat angiogenesis-related conditions in a subject in need of angiogenesis. Insufficient angiogenesis is related to a large number of diseases and conditions, such as cardiovascular diseases, transplantation, aneurisms and delayed wound healing. Therapeutic angiogenesis is aimed at stimulating new blood vessel growth. The concept of such a therapy is based on the premise that the inherent potential of vascularization in a vascular tissue can be utilized to promote the development of new blood vessels under the influence of the appropriate angiogenic molecules.

### VIII. Pharmaceutical Preparations

Pharmaceutical compositions as disclosed herein comprise an effective amount of one or more SRPX2-related agent or additional agent dissolved or dispersed in a pharmaceutically acceptable carrier. The SRPX2-related agent includes an SRPX2 inhibitor such as an SRPX2 inhibitory nucleic acid or antibody fragment, or an SRPX2 stimulator, such as an SRPX2 expression construct, depending on disease to be treated.

Where clinical application of a composition containing an inhibitory nucleic acid is undertaken, it will generally be beneficial to prepare a pharmaceutical composition appropriate for the intended application. This will typically entail preparing a pharmaceutical composition that is essentially free of pyrogens, as well as any other impurities that could be harmful to humans or animals. One may also employ appropriate buffers to render the complex stable and allow for uptake by target cells.

The phrases "pharmaceutical or pharmacologically acceptable" refers to molecular entities and compositions that do not produce an adverse, allergic or other untoward reaction when administered to an animal, such as a human, as appropriate. The preparation of a pharmaceutical composition comprising a inhibitory nucleic acid or additional active ingredient will be known to those of skill in the art in light of the present disclosure, as exemplified by Remington (2005). Moreover, for animal (e.g., human) administration, it will be understood that preparations should meet sterility, pyrogenicity, general safety and purity standards as required by FDA Office of Biological Standards.

As used herein, "pharmaceutically acceptable carrier" includes any and all solvents, dispersion media, coatings, surfactants, antioxidants, preservatives (e.g., antibacterial agents, antifungal agents), isotonic agents, absorption delaying agents, salts, preservatives, drugs, drug stabilizers, gels, binders, excipients, disintegration agents, lubricants, sweetening agents, flavoring agents, dyes, such like materials and combinations thereof, as would be known to one of ordinary skill in the art. A pharmaceutically acceptable carrier is particularly formulated for administration to a human, although in certain embodiments it may be desirable to use a pharmaceutically acceptable carrier that is formulated for administration to a non-human animal but which would not be acceptable (e.g., due to governmental regulations) for administration to a human. Except insofar as any conventional carrier is incompatible with the active ingredient, its use in the therapeutic or pharmaceutical compositions is contemplated.

The actual dosage amount of a composition of the present disclosure administered to a patient or subject can be determined by physical and physiological factors such as body weight, severity of condition, the type of disease being treated, previous or concurrent therapeutic interventions, idiopathy of the patient and on the route of administration. The practitioner responsible for administration will, in any event, determine the concentration of active ingredient(s) in a composition and appropriate dose(s) for the individual subject.

Pharmaceutical compositions may comprise. for example, at least about 0.1% of an active compound. The active compound may comprise between about 2% to about 75% of the weight of the unit, or between about 25% to about 60%, for example, and any range derivable therein. In other non-limiting examples, a dose may also comprise from about 1 to about 1000 mg/kg/body weight (this such range includes intervening doses) or more per administration, and any range derivable therein. In non-limiting examples of a derivable range from the numbers listed herein, a range of about 5 µg/kg/body weight to about 100 mg/kg/body weight, about 5 microgram/kg/body weight to about 500 milligram/kg/body weight, *etc.,* can be administered.

A SRPX2 expression inhibitor may be administered in a dose of 1-100 (this such range includes intervening doses) or more µg or any number in between the foregoing of nucleic acid per dose. Each dose may be in a volume of 1, 10, 50, 100, 200, 500, 1000 or more µl or ml or any number in between the foregoing.

**Solutions** of therapeutic compositions can be prepared in water suitably mixed with a surfactant, such as hydroxypropylcellulose. Dispersions also can be prepared in glycerol, liquid polyethylene glycols, mixtures thereof and in oils. Under ordinary conditions of storage and use, these preparations contain a preservative to prevent the growth of microorganisms.

The therapeutic compositions of the present disclosure are advantageously administered in the form of injectable compositions either as liquid solutions or suspensions; solid forms suitable for solution in, or suspension in, liquid prior to injection may also be prepared. These preparations also may be emulsified. A typical composition for such purpose comprises a pharmaceutically acceptable carrier. For instance, the composition may contain 10 mg, 25 mg, 50 mg or up to about 100 mg of human serum albumin per milliliter of phosphate buffered saline. Other pharmaceutically acceptable carriers include aqueous solutions, non-toxic excipients, including salts, preservatives, buffers and the like.

Examples of non-aqueous solvents are propylene glycol, polyethylene glycol, vegetable oil and injectable organic esters such as ethyloleate. Aqueous carriers include water, alcoholic/aqueous solutions, saline solutions, parenteral vehicles such as sodium chloride, Ringer's dextrose, *etc.* Intravenous vehicles include fluid and nutrient replenishers. Preservatives include antimicrobial agents, antioxidants, chelating agents and inert gases. The pH and exact concentration of the various components the pharmaceutical composition are adjusted according to well known parameters.

As disclosed herein, the compositions of the present disclosure are suitable for application to mammalian eyes. For example, the formulation may be a solution, a suspension, or a gel. In some embodiments, the composition is administered via a biodegradable implant, such as an intravitreal implant or an ocular insert, such as an ocular insert designed for placement against a conjunctival surface.
The therapeutic agent may coat a medical device or implantable device.

The formulation may be applied to the eye in aqueous solution in the form of drops. These drops may be delivered from a single dose ampoule which may preferably be sterile and thus rendering bacteriostatic components of the formulation unnecessary. Alternatively, the drops may be delivered from a multi-dose bottle which may preferably comprise a device which extracts preservative from the formulation as it is delivered, such devices being known in the art.

Components as disclosed herein may be delivered to the eye as a concentrated gel or similar vehicle which forms dissolvable inserts that are placed beneath the eyelids.

Additional formulations are suitable for oral administration. Oral formulations include such typical excipients as, for example, pharmaceutical grades of mannitol, lactose, starch, magnesium stearate, sodium saccharine, cellulose, magnesium carbonate and the like. The compositions take the form of solutions, suspensions, tablets, pills, capsules, sustained release formulations or powders.

The therapeutic compositions of the present disclosure may include classic pharmaceutical preparations. Administration of therapeutic compositions according to the present disclosure will be via any common route so long as the target tissue is available via that route. This includes oral, nasal, buccal, rectal, vaginal or topical. Topical administration may be particularly advantageous for the treatment of skin cancers, to prevent chemotherapy-induced alopecia or other dermal hyperproliferative disorder. Alternatively, administration may be by orthotopic, intradermal, subcutaneous, intramuscular, intraperitoneal or intravenous injection. Such compositions would normally be administered as pharmaceutically acceptable compositions that include physiologically acceptable carriers, buffers or other excipients. For treatment of conditions of the lungs, or respiratory tract, aerosol delivery can be used. Volume of the aerosol is between about 0.01 ml and 0.5 ml.

An effective amount of the therapeutic composition is determined based on the intended goal. For example, one skilled in the art can readily determine an effective amount of the siRNA of the disclosure to be administered to a given subject, by taking into account factors such as the size and weight of the subject; the extent of the neovascularization or disease penetration; the age, health and sex of the subjcct; the route of administration; and whether the administration is regional or sysemic. The term "unit dose" or "dosage" refers to physically discrete units suitable for use in a subject, each unit containing a predetermined-quantity of the therapeutic composition calculated to produce the desired responses discussed above in association with its administration, *i.e.*, the appropriate route and treatment regimen. The quantity to be administered, both according to number of treatments and unit dose, depends on the protection or effect desired.

Precise amounts of the therapeutic composition also depend on the judgment of the practitioner and are peculiar to each individual. Factors affecting the dose include the physical and clinical state of the patient, the route of administration, the intended goal of treatment (*e.g*., alleviation of symptoms versus cure) and the potency, stability and toxicity of the particular therapeutic substance.

### IX. Combination Treatments

It is also disclosed that the compositions and methods as disclosed herein involve an inhibitor of expression of SRPX2, or construct capable of expressing an inhibitor of SRPX2 expression, or an antibody or a binding fragment against SRPX2 to inhibit its activity in angiogenesis, in combination with a second or additional therapy. Such therapy can be applied in the treatment of any disease that is associated with increased expression or activity of a SRPX2. For example, the disease may be an angiogenesis-related disease.

The methods and compositions including combination therapies enhance the therapeutic or protective effect, and/or increase the therapeutic effect of another anti-angiogenesis, anti-cancer or anti-hyperproliferative therapy. Therapeutic and prophylactic methods and compositions can be provided in a combined amount effective to achieve the desired effect, such as the killing of a cancer cell and/or the inhibition of cellular hyperproliferation. This process may involve contacting the cells with both an inhibitor of gene expression and a second therapy. A tissue, tumor, or cell can be contacted with one or more compositions or pharmacological formulation(s) including one or more of the agents (*i.e.*, inhibitor of gene expression or an anti-cancer agent), or by contacting the tissue, tumor, and/or cell with two or more distinct compositions or formulations, wherein one composition provides 1) an inhibitor of gene expression; 2) an anti-cancer agent, or 3) both an inhibitor of gene expression and an anti-cancer agent. Also, it is contemplated that such a combination therapy can be used in conjunction with a chemotherapy, radiotherapy, surgical therapy, or immunotherapy.

An inhibitor of gene expression and/or activity may be administered before, during, after or in various combinations relative to an anti-cancer treatment. The administrations may be in intervals ranging from concurrently to minutes to days to weeks. In embodiments where the inhibitor of gene expression is provided to a patient separately from an anti-cancer agent, one would generally ensure that a significant period of time did not expire between the time of each delivery, such that the two compounds would still be able to exert an advantageously combined effect on the patient. In such instances, it is contemplated that one may provide a patient with the inhibitor of gene expression therapy and the anti-cancer therapy within about 12 to 24 or 72 h of each other and, more particularly, within about 6-12 h of each other. In some situations it may be desirable to extend the time period for treatment significantly where several days (2, 3, 4, 5, 6 or 7) to several weeks (1, 2, 3, 4, 5, 6, 7 or 8) lapse between respective administrations.

It is also disclosed that a course of treatment may last 1-90 days, or more (this such range includes intervening days). It is contemplated that one agent may be given on any day of day 1 to day 90 (this such range includes intervening days) or any combination thereof, and another agent is given on any day of day 1 to day 90 (this such range includes intervening days) or any combination thereof. Within a single day (24-hour period), the patient may be given one or multiple administrations of the agent(s). Moreover, after a course of treatment, it is contemplated that there is a period of time at which no anti-canccr treatment is administered. This time period may last 1-7 days, and/or 1-5 weeks, and/or 1-12 months or more (this such range includes intervening days), depending on the condition of the patient, such as their prognosis, strength, health, *etc.*

Various combinations may be employed. For the example below an inhibitor of gene expression therapy is "A" and an anti-cancer therapy is "B":
A/B/A B/A/B B/B/A A/A/B A/B/B B/A/A A/B/B/B B/A/B/B
B/B/B/A B/B/A/B A/A/B/B A/B/A/B A/B/B/A B/B/A/A
B/A/B/A B/A/A/B A/A/A/B B/A/A/A A/B/A/A A/A/B/A

Administration of any compound or therapy of the present disclosure to a patient will follow general protocols for the administration of such compounds, taking into account the toxicity, if any, of the agents. Therefore, in some embodiments there is a step of monitoring toxicity that is attributable to combination therapy. It is expected that the treatment cycles would be repeated as necessary. It also is contemplated that various standard therapies, as well as surgical intervention, may be applied in combination with the described therapy.

In specific aspects, it is contemplated that a standard therapy will include antiangiogenic therapy, chemotherapy, radiotherapy, immunotherapy, surgical therapy or gene therapy and may be employed in combination with the inhibitor of gene expression therapy, anticancer therapy, or both the inhibitor of gene expression therapy and the anti-cancer therapy, as described herein.

### A. Antiangiogenic therapy

The skilled artisan will understand that additional antiangiogenic therapies may be used in combination or in conjunction with methods as disclosed herein. For example additional antiangiogenic therapies may antagonize the VEGF and/or FGF signaling pathway. Thus, in some cases and additional therapy may comprise administration an antibody that binds to VEGF, a VEGF receptor, FGF or an FGF receptor. As disclosed herein, methods and compositions of the disclosure may be used in conjunction with AVASTIN® (bevacizumab), LUCENTIS® (ranibizumab), MACUGEN® (pegaptanib sodium) or an anti-inflammatory drug. Thus,
there is disclosed a therapeutic composition comprising an anti-SRPX2 composition and bevacizumab or pegaptanib sodium in a pharmaceutically acceptable carrier. In still further aspects a gene that regulates angiogenesis may be delivered in conjunction with the methods of the disclosure For example, a gene that regulates angiogenesis may be a tissue inhibitor of metalloproteinase, endostatin, angiostatin, endostatin XVIII, endostatin XV, kringle 1-5, PEX, the C-terminal hemopexin domain of matrix metalloproteinase-2, the kringle 5 domain of human plasminogen, a fusion protein of endostatin and angiostatin, a fusion protein of endostatin and the kringle 5 domain of human plasminogen, the monokine-induced by interferon-gamma (Mig), the interferon-alpha inducible protein 10 (IP10), a fusion protein of Mig and IP10, soluble FLT-1 (fins-like tyrosine kinase 1 receptor), and kinase insert domain receptor (KDR) gene. Such an angiogenic regulator gene may be delivered in a viral vector such as the lentiviral vectors described in U.S. Patent 7,122,181.

### B. Chemotherapy

A wide variety of chemotherapeutic agents may be used in accordance with the present disclosure. The term "chemotherapy" refers to the use of drugs to treat cancer. A "chemotherapeutic agent" is used to connote a compound or composition that is administered in the treatment of cancer. These agents or drugs are categorized by their mode of activity within a cell, for example, whether and at what stage they affect the cell cycle. Alternatively, an agent may be characterized based on its ability to directly cross-link DNA, to intercalate into DNA, or to induce chromosomal and mitotic aberrations by affecting nucleic acid synthesis. Most chemotherapeutic agents fall into the following categories: alkylating agents, antimetabolites, antitumor antibiotics, mitotic inhibitors, and nitrosoureas.

Examples of chemotherapeutic agents include alkylating agents such as thiotepa and cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); a camptothecin (including the synthetic analogue topotccan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (*e.g*., calicheamicin, especially calicheamicin gammalI and calicheamicin omegaI1; dynemicin, including dynemicin A; bisphosphonates, such as clodronate; an esperamicin; as well as neocarzinostatin chromophore and related chromoprotein enediyne antiobiotic chromophores, aclacinomysins, actinomycin, authrarnycin, azaserine, bleomycins, cactinomycin, carabicin, carminomycin, carzinophilin, chromomycinis, dactinomycin, daunorubicin, detorubicin, 6-diazo-5-oxo-L-norleucine, doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elformithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; podophyllinic acid; 2-ethylhydrazide; procarbazine; PSK polysaccharide complex; razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; taxoids, e.g., paclitaxel and docetaxel gemcitabine; 6-thioguanine; mercaptopurine; platinum coordination complexes such as cisplatin, oxaliplatin and carboplatin; vinblastine; platinum; etoposide (VP-16); ifosfamide; mitoxantrone; vincristine; vinorelbine; novantrone; teniposide; edatrexate; daunomycin; aminopterin; xeloda; ibandronate; irinotecan (*e.g*., CPT-11); topoisomerase inhibitor RFS 2000; difluorometlhylomithine (DMFO); retinoids such as retinoic acid; capecitabine; carboplatin, procarbazine, plicomycin, gemcitabien, navelbine, farnesyl-protein tansferase inhibitors, transplatinum, and pharmaceutically acceptable salts, acids or derivatives of any of the above.

Also included in this definition are anti-hormonal agents that act to regulate or inhibit hormone action on tumors such as anti-estrogens and selective estrogen receptor modulators (SERMs), including, for example, tamoxifen, raloxifene, droloxifene, 4-hydroxytamoxifen, trioxifene, keoxifene, LY117018, onapristone, and toremifene; aromatase inhibitors that inhibit the enzyme aromatase, which regulates estrogen production in the adrenal glands, such as, for example, 4(5)-imidazoles, aminoglutethimide, megestrol acetate, exemestane, formestanie, fadrozole, vorozole, letrozole, and anastrozole; and anti-androgens such as flutamide, nilutamide, bicalutamide, leuprolide, and goserelin; as well as troxacitabine (a 1,3-dioxolane nucleoside cytosine analog); antisense oligonucleotides, particularly those which inhibit expression of genes in signaling pathways implicated in abherant cell proliferation, such as, for example, PKC-alpha, Ralf and H-Ras; ribozymes such as a VEGF expression inhibitor and a HER2 expression inhibitor; vaccines such as gene therapy vaccines and pharmaceutically acceptable salts, acids or derivatives of any of the above.

### C. Radiotherapy

Other factors that cause DNA damage and have been used extensively include what are commonly known as γ-rays, X-rays, and/or the directed delivery of radioisotopes to tumor cells. Other forms of DNA damaging factors are also contemplated such as microwaves, proton beam irradiation (U.S. Patents 5,760,395 and 4,870,287) and UV-irradiation. It is most likely that all of these factors affect a broad range of damage on DNA, on the precursors of DNA, on the replication and repair of DNA, and on the assembly and maintenance of chromosomes. Dosage ranges for X-rays range from daily doses of 50 to 200 roentgens for prolonged periods of time (3 to 4 wk), to single doses of 2000 to 6000 roentgens. Dosage ranges for radioisotopes vary widely, and depend on the half-life of the isotope, the strength and type of radiation emitted, and the uptake by the neoplastic cells.

The terms "contacted" and "exposed," when applied to a cell, are used herein to describe the process by which a therapeutic construct and a chemotherapeutic or radiotherapeutic agent are delivered to a target cell or are placed in direct juxtaposition with the target cell. To achieve cell killing, for example, both agents are delivered to a cell in a combined amount effective to kill the cell or prevent it from dividing.

### D. Immunotherapy

In the context of cancer treatment, immunotherapeutics, generally, rely on the use of immune effector cells and molecules to target and destroy cancer cells. Trastuzumab (Herceptin™) is such an example. The immune effector may be, for example, an antibody specific for some marker on the surface of a tumor cell. The antibody alone may serve as an effector of therapy or it may recruit other cells to actually affect cell killing. The antibody also may be conjugated to a drug or toxin (chemotherapeutic, radionuclide, ricin A chain, cholera toxin, pertussis toxin, *etc*.) and serve merely as a targeting agent. Alternatively, the effector may be a lymphocyte carrying a surface molecule that interacts, either directly or indirectly, with a tumor cell target. Various effector cells include cytotoxic T cells and NK cells. The combination of therapeutic modalities, *i.e*., direct cytotoxic activity and inhibition or reduction of ErbB2 would provide therapeutic benefit in the treatment of ErbB2 overexpressing cancers.

Another immunotherapy could also be used as part of a combined therapy with gene silencing therapy discussed above. In one aspect of immunotherapy, the tumor cell must bear some marker that is amenable to targeting, *i.e.,* is not present on the majority of other cells. Many tumor markers exist and any of these may be suitable for targeting in the context of the present disclosure. Common tumor markers include carcinoembryonic antigen, prostate specific antigen, urinary tumor associated antigen, fetal antigen, tyrosinase (p97), gp68, TAG-72, HMFG, Sialyl Lewis Antigen, MucA, MucB, PLAP, estrogen receptor, laminin receptor, erb B and p155. An alternative aspect of immunotherapy is to combine anticancer effects with immune stimulatory effects. Immune stimulating molecules also exist including: cytokines such as IL-2, IL-4, IL-12, GM-CSF, gamma-IFN, chemokines such as MIP-1, MCP-1, IL-8 and growth factors such as FLT3 ligand. Combining immune stimulating molecules, either as proteins or using gene delivery in combination with a tumor suppressor has been shown to enhance anti-tumor effects (Ju *et al.*, 2000). Moreover, antibodies against any of these compounds can be used to target the anti-cancer agents discussed herein.

Examples of immunotherapies currently under investigation or in use are immune adjuvants *e.g*., Mycobacterium bovis, Plasmodium falciparum, dinitrochlorobenzene and aromatic compounds (U.S. Patents 5,801,005 and 5,739,169; Hui and Hashimoto, 1998; Christodoulides *et al.*, 1998), cytokine therapy, *e.g.,* interferons α, β and γ; IL-1, GM-CSF and TNF (Bukowski *et al.*, 1998; Davidson *et al.*, 1998; Hellstrand *et al.*, 1998) gene therapy, *e.g.,* TNF, IL-1, IL-2, p53 (Qin *et al.*, 1998; Austin-Ward and Villaseca, 1998; U.S. Patents 5,830,880 and 5,846,945) and monoclonal antibodies, *e.g*., anti-ganglioside GM2, anti-HER-2, anti-p185 (Pietras *et al.*, 1998; Hanibuchi *et al.*, 1998; U.S. Patent 5,824,311). It is contemplated that one or more anti-cancer therapies may be employed with the gene silencing therapies described herein.

In active immunotherapy, an antigenic peptide, polypeptide or protein, or an autologous or allogenic tumor cell composition or "vaccine" is administered, generally with a distinct bacterial adjuvant (Ravindranath and Morton, 1991; Morton *et al.*, 1992; Mitchell *et al.*, 1990; Mitchell *et al.*, 1993).

In adoptive immunotherapy, the patient's circulating lymphocytes, or tumor infiltrated lymphocytes, are isolated *in vitro,* activated by lymphokines such as IL-2 or transduced with genes for tumor necrosis, and readministered (Rosenberg *et al.*, 1988; 1989).

### E. Surgery

Approximately 60% of persons with cancer will undergo surgery of some type, which includes preventative, diagnostic or staging, curative, and palliative surgery. Curative surgery is a cancer treatment that may be used in conjunction with other therapies, such as the treatment as disclosed herein, chemotherapy, radiotherapy, hormonal therapy, gene therapy, immunotherapy and/or alternative therapies.

Curative surgery includes resection in which all or part of cancerous tissue is physically removed, excised, and/or destroyed. Tumor resection refers to physical removal of at least part of a tumor. In addition to tumor resection, treatment by surgery includes laser surgery, cryosurgery, electrosurgery, and microscopically controlled surgery (Mohs' surgery). It is also disclosed that the present disclosure may be used in conjunction with removal of superficial cancers, precancers, or incidental amounts of normal tissue.

Upon excision of part or all of cancerous cells, tissue, or tumor, a cavity may be formed in the body. Treatment may be accomplished by perfusion, direct injection or local application of the area with an additional anti-cancer therapy. Such treatment may be repeated, for example, every 1, 2, 3, 4, 5, 6, or 7 days, or every 1, 2, 3, 4, and 5 weeks or every 1, 2, 3, 4, 5, 6, 7, 8, 9, 10, 11, or 12 months. These treatments may be of varying dosages as well.

### F. Other Agents

It is also disclosed that other agents may be used in combination with the present disclosure to improve the therapeutic efficacy of treatment. These additional agents include immunomodulatory agents, agents that affect the upregulation of cell surface receptors and GAP junctions, cytostatic and differentiation agents, inhibitors of cell adhesion, agents that increase the sensitivity of the hyperproliferative cells to apoptotic inducers, or other biological agents. Immunomodulatory agents include tumor necrosis factor; interferon alpha, beta, and gamma; IL-2 and other cytokines; F42K and other cytokine analogs; or MIP-1, MIP-1beta, MCP-1, RANTES, and other chemokines. It is further disclosed that the upregulation of cell surface receptors or their ligands such as Fas / Fas ligand, DR4 or DR5 / TRAIL (Apo-2 ligand) would potentiate the apoptotic inducing abilities as disclosed herein by establishment of an autocrine or paracrine effect on hyperproliferative cells. Increases intercellular signaling by elevating the number of GAP junctions would increase the anti-hyperproliferative effects on the neighboring hyperproliferative cell population. It is also disclosed that cytostatic or differentiation agents can be used in combination with the present invention to improve the anti-hyperproliferative efficacy of the treatments. Inhibitors of cell adhesion are contemplated to improve the efficacy of the present disclosure Examples of cell adhesion inhibitors are focal adhesion kinase (FAKs) inhibitors and Lovastatin. It is further disclosed that other agents that increase the sensitivity of a hyperproliferative cell to apoptosis, such as the antibody c225, could be used in combination with the present disclosure to improve the treatment efficacy.

There have been many advances in the therapy of cancer following the introduction of cytotoxic chemotherapeutic drugs. However, one of the consequences of chemotherapy is the development/acquisition of drug-resistant phenotypes and the development of multiple drug resistance. The development of drug resistance remains a major obstacle in the treatment of such tumors and therefore, there is an obvious need for alternative approaches such as gene therapy.

Another form of therapy for use in conjunction with chemotherapy, radiation therapy or biological therapy includes hyperthermia, which is a procedure in which a patient's tissue is exposed to high temperatures (up to 106°F). External or internal heating devices may be involved in the application of local, regional, or whole-body hyperthermia. Local hyperthermia involves the application of heat to a small area, such as a tumor. Heat may be generated externally with high-frequency waves targeting a tumor from a device outside the body. Internal heat may involve a sterile probe, including thin, heated wires or hollow tubes filled with warm water, implanted microwave antennae, or radiofrequency electrodes.

A patient's organ or a limb is heated for regional therapy, which is accomplished using devices that produce high energy, such as magnets. Alternatively, some of the patient's blood may be removed and heated before being perfused into an area that will be internally heated. Whole-body heating may also be implemented in cases where cancer has spread throughout the body. Warm-water blankets, hot wax, inductive coils, and thermal chambers may be used for this purpose.

Hormonal therapy may also be used in conjunction with the present disclosure or in combination with any other cancer therapy previously described. The use of hormones may be employed in the treatment of certain cancers such as breast, prostate, ovarian, or cervical cancer to lower the level or block the effects of certain hormones such as testosterone or estrogen. This treatment is often used in combination with at least one other cancer therapy as a treatment option or to reduce the risk of metastases.

### X. Kits and Diagnostics

There is also disclosed a kit containing therapeutic agents and/or other therapeutic and delivery agents.
There is also disclosed a kit for preparing and/or administering a therapy of the disclosure. The kit may comprise one or more sealed vials containing any of the pharmaceutical compositions of the present disclosure. It is disclosed that the lipid may be in one vial, and the nucleic acid component is in a separate vial. The kit may include, for example, at least one inhibitor of SRPX2 expression/activity or an SRPX2 protein or peptide, one or more lipid component, as well as reagents to prepare, formulate, and/or administer the components as disclosed herein or perform one or more steps of the disclosed methods.

The kit may also comprise a suitable container means, which is a container that will not react with components of the kit, such as an eppendorf tube, an assay plate, a syringe, a bottle, or a tube. The container may be made from sterilizable materials such as plastic or glass.

The kit may further include an instruction sheet that outlines the procedural steps of the methods set forth herein, and will follow substantially the same procedures as described herein or are known to those of ordinary skill. The instruction information may be in a computer readable media containing machine-readable instructions that, when executed using a computer, cause the display of a real or virtual procedure of delivering a pharmaceutically effective amount of a therapeutic agent.

### XI. Examples

The following examples are included to further illustrate various aspects of the. It should be appreciated by those of skill in the art that the techniques disclosed in the examples that follow represent techniques and/or compositions discovered by the authors to function well in the practice of the disclosure and thus can be considered to constitute particular modes for its practice.

### Example 1 - Differential SRPX2 Gene Expression in Angiogenic and Resting Endothelial Cells Detected by DNA Microarray Analysis

The inventors previously isolated subpopulations from an endothelioma cell line with molecular characteristics of angiogenic (t.End.1V^{high}) and resting (t.End.1V^{low}) endothelium (Aurrand-Lions *et al.*, 2004). To identify novel genes involved in angiogenesis the inventors examined the gene expression profiles of t.End.1V^{high} angiogenic and t.End.1V^{low} resting endothelial cells by DNA microarray technique (GeneChip® Mouse Genome 430 2.0 Array, Affimetrix). Normalization for each gene and comparative analysis between the expression profiles was carried out using GeneSpring software. The t.End.1V^{high} cells express high levels of the integrin αVβ3 and do not endocytose acetylated LDL while t.End.1V^{low} have low αVβ3 integrin levels and efficiently take up acetylated LDL. In addition, t.End.1V^{high} show increased cell migration, lack of inflammatory response and form cord-like structures in three dimensional (3D) fibrin gels (Aurrand-Lions *et al*., 2004). All these characteristics are generally associated with the angiogenic state of endothelial cells.

The microarray data analysis resulted in more than 3500 differentially expressed genes in the two cell lines, while more than 1700 genes showed two- or more-fold over-expression in tEnd.1V^{high} angiogenic cells. Analysis of the gene expression profiles was carried out using GeneSpring software platform (GeneSpringGX, Agilent). In order to narrow down the number of differentially expressed genes, which may be linked to angiogenesis, the microarray data were overlaid with the gene expression profiles provided by the National Institutes of Health Gene Expression Omnibus (GEO) public site (available through world wide web at ncbi.nlm.nih.gov/entrez/query.fcgi/). The GSE3601 microarray dataset contains angiogenesis-associated genes, differentially expressed by human umbilical cord vein endothelial cells (HUVEC) after lentiviral gene delivery of a vMIP-II protein (viral macrophage inflammatory protein II) with a potent proangiogenic activity (Cherqui *et al.*, 2007). Genes over-expressed at least 2x in tEnd.1V^{high} angiogenic cells (total of 612 genes) were compared to genes over-expressed at least 2x in vMIP-II-activated HUVEC (total of 781 genes) and 38 genes were selected that are ≥ 2-fold over-expressed in tEnd.1V^{high} and vMIP-II activated HUVEC cells **(****FIG. 1A**). Several genes that were identified by this comparative method are already associated with angiogenesis, such as CEACAM1 (Horst *et al.*, 2006; Oliveira-Ferrer *et al.*, 2004) or VE-PTP (Dominguez *et al.*, 2007; Mellberg *et al.*, 2009). However, the inventors identified using the same method ***SRPX2*** gene with so far unknown function in angiogenesis. The SRPX2 gene was more than 20- and 3.3-fold over-expressed in mouse (t.End.1V^{high} cells) and human angiogenesis associated microarray datasets, respectively.

In order to validate the result, the inventors used quantitative real-time RT-PCR (qPCR). Total RNA from tEnd.1V^{high} angiogenic and tEnd.1V^{low} resting cells were reverse-transcribed and subjected to qPCR. The values for *SRPX-2* were normalized with those of three house keeping genes: *β-actin, EEF1A1* and *β-tubulin.* The ratio of angiogenic and resting samples was calculated and shown as relative values **(****FIG. 1B****).** Indeed, the 25-fold up-regulation of the *SRPX2* gene in angiogenic t.End.1V^{high} cells validated the selection of this gene as a novel angiogenesis associated target. The statistical analysis, using the Welch t-test, confirmed the significance of the data (p<0.03664).

Microarray data analysis and data mining - Total RNA was extracted from mouse t.End.1Vhigh angiogenic and t.End.1Vlow resting cells. The cultured cells were harvested and lysed using RNeasy Mini kit (Qiagen), according to the manufacturer's instructions. The purified RNA was quantified by a UV spectrophotometer, and RNA quality was evaluated by capillary electrophoresis on an Agilent 2100 Bioanalyser (Agilent Technologies). Total RNA was reverse transcribed using the cDNA synthesis kit (Roche). Labeled cDNA were hybridized to an Affimetrix Mouse Genome 430 2.0 Array GeneChip. Normalization for each gene and comparative analysis between expression profiles was carried out using GeneSpring GC 7.3 software. Comparative analysis was done with the data extracted from the NIH GEO Datasets database (available through world wide web at www.ncbi.nlm.nih.gov/entrez/query.fcgi?CMD=search&DB=gds). This dataset was provided by the National Institutes of Health Gene Expression Omnibus (GEO) public site (available through world wide web at ncbi.nlm.nih.gov/entrez/query.fcgi/).

Quantitative real time RT-PCR - Validation of microarray data was done using quantitative real time RT-PCR (qPCR). Total RNA was extracted from t.End.1Vhigh angiogenic cells 24 hours after nucleofection using RNeasy Mini kit (Qiagen). The isolated total RNA was quantified by a UV spectrophotometer and reverse transcribed using the cDNA synthesis kit (Roche), following manufacturer's instructions.

QPCR was performed according to the standard protocol used by in house facility (available at world wide web through frontiers-in-genetics.org/en/index.php?id=genomics). Primers used were the following: mouse SRPX2_RNAi-764 Forward: 5'-ATGGAAGATGGGCGATGGA-3' (SEQ ID NO: 24); and SRPX2_RNAi-864 Reverse: 5'-TCTGGCTCTGCCATTTTCTCA-3' (SEQ ID NO: 25) as well as standard qPCR primers for three murine house keeping genes: β-actin, β-tubulin and EEF1A1. Reactions were performed in triplicate with the Power SYBR Green PCR kit and primer assay (Applied Biosystems, Inc) on a PCR system (Prism 7000; ABI). The results were quantified using the system software (SDS Prism 7000; ABI). Measurements of β-actin, β-tubulin and EEF1A1 house keeping genes were used for normalization of expression levels across samples.

### Example 2 - In vivo expression of SRPX2 gene in angiogenic tissue

Using *in situ* mRNA hybridization, the inventors found strong vascular expression of *SRPX2* in mouse angiogenic tissues. For this purpose, the inventors induced new blood vessel formation by injecting subcutaneously bFGF-treated matrigel plugs in mice. After 14 days, vascularized plugs were harvested, subjected to cryo-sectioning and *in situ* mRNA hybridization. Double labeling with riboprobes of the vascular marker PECAM-1 and SRPX2 revealed robust expression of *SRPX2* mRNA in *de novo* formed blood vessels in the matrigel plugs **(****FIG. 2A****,** upper panel). Then, the inventors investigated whether *SRPX2* mRNA would also be expressed in newly formed blood vessels induced by growing Lewis lung carcinoma (LLC1) in mice. Similarly to the bFGF-treated matrigel plugs, *SRPX2* was co-expressed by *PECAM-1-*positive blood vessels in tumors **(****FIG. 2A****,** lower panel). The inventors then investigated ***SRPX2*** expression at the protein level using the same tissue. Double immuno-fluorescence showed that the SRPX2 protein is present in vascular endothelial cells of both tissue **(****FIG. 2B****).** Antibody staining clearly showed that SRPX2 is specifically expressed in endothelial cells and not in surrounding tissue **(****FIG. 2B****).** In summary, SRPX2 is a novel molecule expressed by *de novo* formed blood vessels.

Double-labeling *in situ* mRNA hybridization - The digoxigenin (DIG)-labeled and fluorescein riboprobes were prepared after PCR amplification of mouse *PECAM-1* gene with corresponding forward and reverse primers, latter containing the T7 polymerase binding site (underline) (mPECAM1-as-for1: ATG CTC CTG GCT CTG GGA CTC (SEQ ID NO:26) and mPECAM1-as-rev1: CTA ATA CGA CTC ACT ATA GGG TGC AGC TGG TCC CCT TCT ATG (SEQ ID NO:27)); the *SRPX2* gene with corresponding primers for sense and anti-sense riboprobes, respectively: mSrpx2-s-forl: CTA ATA CGA CTC ACT ATA GGG ATG ACC AGT CCA CTG ACT CAG (SEQ ID NO:28); mSrpx2-s-revl: CGA TCT CCT TCT AGG TGG TAG (SEQ ID NO:29); mSrpx2-as-forl: ATG ACC AGT CCA CTG ACT CAG (SEQ ID NO:30) and mSrpx2-as-revl: CTA ATA CGA CTC ACT ATA GGG CGA TCT CCT TCT AGG TGG TAG (SEQ ID NO:31). Double labeling *in situ* hybridization was carried out on cryo-sections of LLC1 tumors or bFGF-treated Matrigel plugs as following: cryo-sections were incubated in hybridization solution (50% formamide, 5x SSC, 0.1% Tween 20, 0.1% CHAPS, 1 Denhardt's solution, 0.01% heparine, 0.02% tRNA in DEPC H₂O) containing 1 to 2 µg/ml DIG- or fluorescein-labeled mouse *PECAM-1* RNA probe and a DIG- or fluorescein-labeled *SRPX2* RNA probe for 16 h at 55°C. For detection, samples were incubated with two antibodies simultaneously, the sheep anti-DIG Fab fragments coupled to alkaline phosphatase (1:2000, Roche) and the sheep anti-fluorescein Fab fragments coupled to horseradish peroxidase (HRP) (1:100, Roche) during 1 h at RT. Unbound antibodies were removed using TNT buffer (150 mM NaCl, 100 mM Tris HCl, pH 7.5, 0.05% Tween-20) 3x 5 min and incubated in biotinyl-tyramide mix diluted in the amplification buffer (1:50, PerkinElmer Life Sciences) for 30 min at RT, before being washed again in TNT buffer 3x 5 min. Alexa-488-conjugated streptavidin antibody (1:100, Molecular Probes) was added in the amplification buffer and incubation was carried out at RT for 30 min. Cryosections were subsequently washed in TNT buffer 3x 5 min and stained with Fast Red (DAKO Cytomation) for 30 min at RT in the dark. Staining was stopped by washing in TNT buffer 3x 5 min at RT. Samples were stained with nuclear TO-PRO dye (Molecular probes ; 1 :2000) or DAPI (1:10000), mounted in Moewiol/DABCO (Sigma) mix and screened for fluorescent signals on a Zeiss LSM 510 Meta confocal microscope.

Immunofluorescence labeling - Double or triple immunofluorescence labeling was carried out on cryo-sections of LLC1 tumors or bFGF-treated Matrigel plugs as following: cryo-sections were fixed in cold methanol for 5 minutes at RT, dried completely and re-hydrated in 0.02% gelatin; 0.05% Tween 20 in PBS for 15 minutes at RT. For detection, samples were incubated with two or three antibodies simultaneously, the rabbit anti-SRPX2 (1:50, gift from Dr. Pierre Szepetowski), the sheep anti-uPAR (1:100, Abcam) and rat anti-PECAMl (1:500) during 1 h at RT. Unbound antibodies were removed using 0.02% gelatine; 0.05% Tween 20 in PBS 3x 5 minutes at RT. Rabbit anti-SRPX2 antibody was detected by mouse or donkey anti-rabbit IgG coupled to TexasRed (red) or APC (light blue), respectively; sheep anti-uPAR antibody detected by donkey anti-sheep IgG coupled to TexasRed (red) and rat anti-PECAM-1 antibody detected by rabbit anti-rat IgG coupled to FITC (green) for 1 hour at RT in the dark. Staining was stopped by washing in 0.02% gelatin; 0.05% Tween 20 in PBS 3x 5 min at RT. Samples were stained with nuclear counter-dye DAPI (1 :10000), mounted in Moewiol/DABCO (Sigma) mix and screened for fluorescent signals on a Zeiss LSM 510 Meta confocal microscope.

bFGF-treated matrigel assay *in vivo* - The Matrigel plug angiogenesis assay included implantation of Matrigel supplemented with the proangiogenic factor bFGF (FGF-2) into mice. Implantation was performed via ventral and subcutaneous injection of 400 µl Matrigel loaded with 500 ng/ml bFGF per animal. The plugs were then collected and prepared for cryo-sectioning.

### Example 3 - Silencing of SRPX2 Gene in t.End.1V^{high} Angiogenic Cells

To further characterize the up-regulation of *SRPX2* in tEnd.1V^{high} angiogenic cells, the inventors used the small interfering RNAs (siRNAs) approach to transiently knock down its expression. The tEnd.1V^{high} angiogenic cells were transiently transfected using Nucleofactor technology (Amaxa Inc.). Three siRNAs were designed to target non-overlapping regions of the *SRPX2* gene (Stealth™ ***SRPX2*** siRNA 1 (SEQ ID:11), 2 (SEQ ID:12) and 3 (SEQ ID:13), Invitrogen). The inventors obtained approximately 90% siRNA transfection efficiency (data not shown), largely sufficient to detect functional effects of the targeted gene. The efficiency of silencing of *SRPX2* expression in the tEnd.1V^{high} angiogenic cells was evidenced by quantitative PCR 24 hours after transfection **(****FIG. 3****).**

As shown in **FIG. 3****,** *SRPX2* siRNA 2 (SEQ ID:12) blocked the ***SRPX2*** gene expression by 75-95% in a dose-dependent manner. The *SRPX2* siRNA 1 (SEQ ID NO:11) appeared to be less efficient, reducing SRPX2 gene expression only by 50% and *SRPX2* siRNA 3 (SEQ ID NO:13) failed to silence even when applied at higher concentrations. In order to observe possible additive effects, three different combinations of the *SRPX2* siRNAs (*SRPX2* siRNAs 1+2 (SEQ ID NO:11 and SEQ ID NO:12), 2+3 (SEQ ID NO:12 and SEQ ID NO:13) and 1+3 (SEQ ID NO:11 and SEQ ID NO:13), 0.6 µM each) were transfected into angiogenic cells. The expression of the *SRPX2* gene directly correlated with the presence of *SRPX2* siRNA 2 (SEQ ID NO:12), identifying it as the most potent silencer of the three. As negative controls the inventors used mock transfected tEnd.1V^{high} cells or cells transfected with siRNAs for the mouse *GAPDH* gene (*GAPDH* siRNA, Ambion) and a sequence, non-homologous to mouse genes (*nh* siRNA, Ambion). As expected, two control siRNAs failed to modify the *SRPX2* gene expression and the level was similar to the one observed for the mock transfected controls **(****FIG. 3****).**

Cell cultures and transfection - The t.End.1Vhigh angiogenic and t.End.1Vlow resting cells were cultured as previously described by (Aurrand-Lions *et al.*, 2004). They were used at low passages (up to third passage). Transient transfection of t.End.1V^{high} angiogenic cells were performed with a Nucleofector kit V (Amaxa) according to the manufacturer's instructions. For transfection, the following chemically modified duplex siRNAs were engaged: three siRNAs directed against mouse SRPX2 gene (SRPX2MSS229325 (SEQ ID NO:11), SRPX2MSS229326 (SEQ ID NO:12), SRPX2MSS229327 (SEQ ID NO:13) here named as SRPX2 siRNA 1, 2 and 3, respectively) (Stealth TM Select technology, Invitrogen), a siRNA against mouse GAPDH (Ambion) and a non-targeting negative control siRNA (nh siRNA) (Ambion). Single siRNAs or combinations of two different siRNAs were nucleofected at a concentration range of 0.4 to 0.6 µM. Transfected cells were engaged immediately after transfection in the experiments.

### Example 4 - Silencing of SRPX2 Gene Leads to Modulation of Migratory Capacities of t.End.1V^{high} Angiogenic Cells in vitro

During angiogenesis endothelial cells migrate to form sprouts and vascular tubes. Since the inventors previously showed that tEnd.1V^{high} cells migrate efficiently, they used them in order to study weather *SRPX2* would influence cell migration in the wound-healing assay. Since angiogenesis is dependent on cell migration the inventors further evaluated whether silencing of the *SRPX2* gene in angiogenic cells would affect their migration, as tested by a wound-healing assay using matrigel™-coated plates. The disruption of the t.End.1V^{high} monolayers induced the cells at the edge of the wound to spread rapidly and migrate onto the matrigel™. The leading front of the cell monolayer migrated homogenously as a unit during 16 hours **(****FIG. 4A****).** Photographs of the migrating cells were taken by the ImageXpress device at the beginning of the healing process and after 16 hours. This period was sufficient to obtain nearly closed wounds. By using Metamorph software the inventors compared the distance of migration of control and *SRPX2*-silenced angiogenic cells.

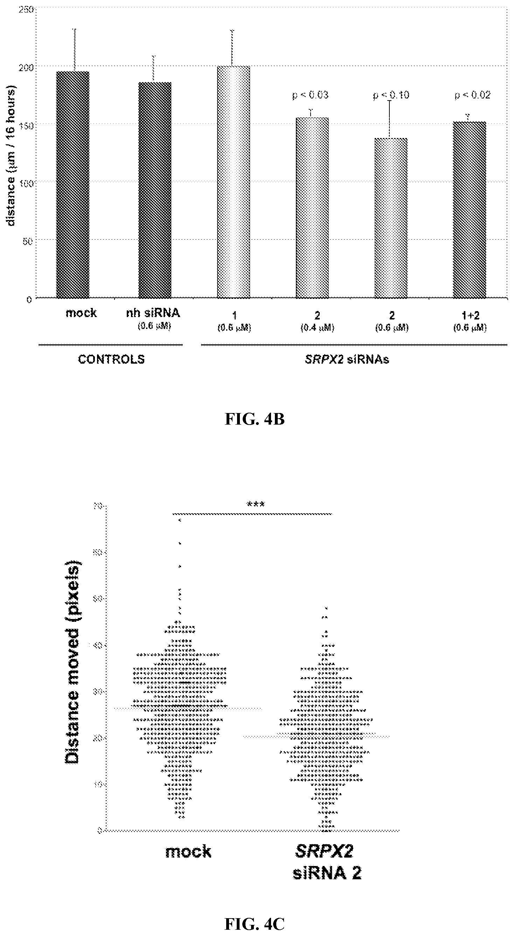

The silenced cells migrated 25 to 32.5% less efficient when compared with control cells as shown in **FIG. 4B****.** The highest reduction of cell migration was obtained with cells transfected with *SRPX2* siRNA 2 in which *SRPX2* silencing was the most efficient **(****FIGs. 3** **and** **4A-C****).** Cell migration was proportional to the level of silencing suggesting that *SRPX2* has a direct impact on cell migration. The best statistical relevance for the difference in migration was obtained when migrating cells were analyzed individually **(****FIG. 4C****).** The evidence that silencing of the *SRPX2* gene can attenuate migration of tEnd.1V^{high} angiogenic cells *in vitro,* suggested a functional importance of this molecule in angiogenesis.

Wound healing assay - 1.5 x 10⁴ tEnd.1V^{high} angiogenic cells were seeded onto matrigel™-coated 96-well plates and grown to confluence. Monolayers were wounded using a pipette tip and cell migration was monitored using an ImageXpress automated microscope equipped with 4x objective. The distance of migration was calculated using the Metamorph software. Wound healing assays for each sample were performed in triplicates and three independent experiments were carried out for each sample. Statistical analysis was preformed and standard deviation was calculated.

### Example 5 - Silencing of SRPX2 in Endothelial Cells Attenuates the Initiation and the Final Steps of Angiogenesis in vitro

In addition to the differences described above, the t.End.1V^{high} angiogenic cells form a capillary-like network of ramified cords in three-dimensional (3D) fibrin gels (Aurrand-Lions *et al.*, 2004; Pepper *et al.*, 1996). Sprout or cord formation *in vitro* starts with individual endothelial cells sending out spikes by 24-32 hours after seeding of the cells (**FIG. 5A****,** arrowheads). These spikes initiate contacts with other cells in the vicinity; the cells then align and form capillary-like structures starting at 48 hours **(****FIG. 5A****).** The spikes of each cell can eventually initiate an alignment, which leads to a branched polygonal structure, resembling a capillary-like network. This assay represents a simple but powerful model for studying induction and/or inhibition of angiogenesis (Montesano *et al.*, 1990; Pepper *et al.*, 1996). The inventors studied spike formation, the initial phase and branching, the late phase of this angiogenesis assay. Thus, the inventors silenced the *SRPX2* gene in the t.End.1V^{high} cells and used them in this sprout formation assay **(****FIG. 5A****).** Photographs of the cultured endothelial cell were taken every 24 hours over six days **(****FIG. 5A****).**

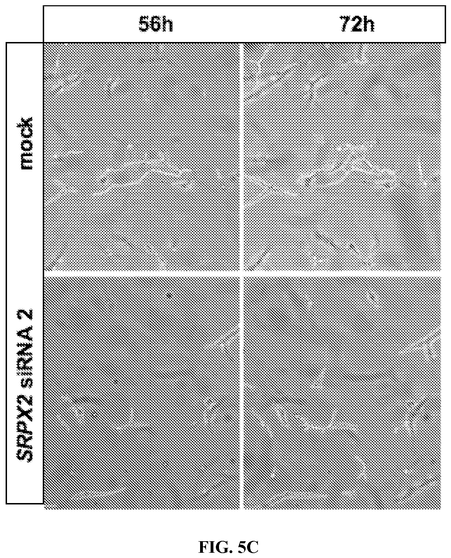

About 50% of control, mock or *GAPDH* siRNA-transfected t.End.1V^{high} cells formed spikes within 24 hours, whereas silencing of *SRPX2* delayed the efficiency of spike formation and reduced it at 24 hours to 25%-35% **(****FIG. 5B****).** As a further control, mock or *nh* siRNA transfected also did not show any difference. The early time point at 24 hours was important since it defines the potential degree of the future branching points. At 56 hours the inventors measured the total surface of the vascular "skeleton" representing the capillary-like network using an image analysis program specifically adapted for this purpose (Metamorph) **(****FIG. 5C****).** Development of this network decreased up to 46% when *SRPX2* was silenced when compared to the total surface of control cells **(****FIGs. 5D-E****).** This was not due to cell death, since the number of apoptotic cells was as low as 0.5-6% of total transfected cells even after 6 days **(****FIG. 5F****).** In conclusion, abrogation of SRPX2 can attenuate the initiation and final steps of cord formation *in vitro,* demonstrating the functional importance of SRPX2 in modulating angiogenesis.

Tube formation assay in three-dimensional fibrin gels - Fibrin gels were prepared as previously described (Pepper *et al.*, 1996). The t.End.1V^{high} angiogenic cells were seeded in suspension into 100 µl of fibrin gels at 1.2 x 10⁴ cells per gel. Then 100 µl of DMEM containing 10% fetal calf serum and 200U of the proteinase inhibitor Trasylol (Aprotinin, Bayer) was added to each well above fibrin gels. During 6 days the cultures were photographed every 24 hours using an ImageXpress automated microscope. The number of sprouting cells per field was counted manually and statistical analysis was done on 3 to 10 fields per sample (standard deviation was calculated). Around 50-100 cells were analyzed per field. The total surface of vascular "skeleton" representing the capillary-like network was quantified using the Metamorph software.

### Example 6 - SRPX2 binds to vascular uPAR

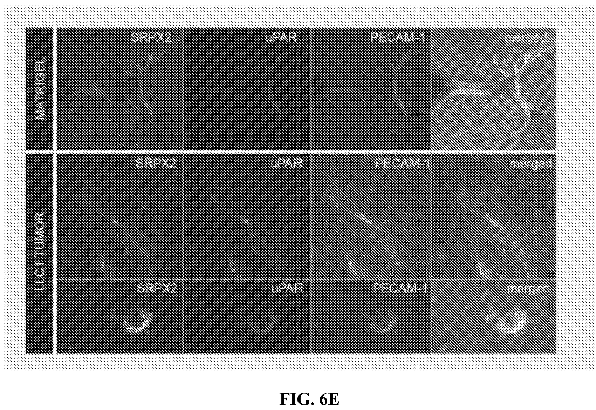

In a neural system it was recently shown that SRPX2 protein binds to uPAR (Royer-Zemmour *et al.*, 2008). As the uPA/uPAR system plays important role in angiogenesis, we firstly analyzed whether t.End.1V^{high} cells express uPAR. Flow cytometry revealed high expression level of uPAR in t.End.1V^{high} cells **(****FIG. 6A****)** and this was confirmed by Western blot **(****FIG. 6C****).** In order to explore whether SRPX2 binds to uPAR expressed by vascular endothelial cells, we produced recombinant FLAG-tagged SRPX2 protein in mammalian cells and preformed pull-down assays. As shown in **FIGs. 6B** and **6D****,** SRPX2 appears as 54 kDa band on Western blot and nicely pulls-down uPAR (45 kDa) with anti-uPAR antibody on Western blots **(****FIG. 6C****).** These experiments show that SRPX2 binds to vascular uPAR. Then, we investigated *in vivo* if SRPX2 would co-localize with uPAR on angiogenic vascular endothelium in bFGF-treated matrigel plug assays and LLC1 tumors **(****FIG. 6E****).** Triple immunofluorescence clearly co-localizes SRPX2, uPAR with the endothelial marker PECAM-1 **(****FIG. 6E****)** in de novo formed endothelial cells of the bFGF-treated matrigel plugs as well as endothelial cells of LLC1 tumors. These findings suggest that SRPX2 interferes with uPAR system during angiogenesis.

Cloning strategy for the production of the recombinant SRPX2 protein tagged with a FLAG sequence - The mouse *SRPX2* full length cDNA was obtained by PCR, performed on MGC full length *SRPX2* clone in the pCMV-SPORT6 vector (Invitrogen, MGC cDNA clone, ID3488526). The *SRPX2* PCR fragment was inserted into the pcDNA 3.1 vector containing FLAG sequence (the pLig10-12), where a FLAG sequence was inserted downstream to and in-frame with the *SRPX2* coding sequence. The *SRPX2*-FLAG PCR fragment was than inserted into the pcDNA™3.3 TOPO®TA vector (Invitrogen). This plasmid was multiplied in DH5α *E. coli,* purified by EndoFree Plasmid maxi preparation (Qiagen) and used for the production of the recombinant protein.

Production and purification of recombinant mouse SRPX2-FLAG - The full length mouse *SRPX2* gene cloned as a FLAG tagged construct into the expression vector pcDNA3.3™-TOPO®TA was used for transfection of MDCK cells. Stably transfected cells were selected by Neomycin and the cell culture supernatant collected. The protein was then affinity purified with anti-FLAG agarose beads (M2, Sigma), eluted with FLAG peptide (100 µg/ml) and revealed with biotinylated anti-FLAG and HRP labeled antibodies and detected by ECL.

Pull down assay of SRPX2 by uPAR - The tEnd1.V^{high} cells were cell surface biotinylated (0.5 mg/ml of biotin in DPBS) for 15 min, blocked with 10% FCS in PBS and lysed in lysis buffer with addition of 0.5% CHAPS and proteinase inhibitors (Sigma). Pre-clearing was preformed subsequently with the protein G beads (40 µl beads / 1 ml lysate) (Protein G Sepharose 4 fast flow, General Electrics) for 3 x 5 min at RT. Immuno-precipitation was performed by adding sheep anti-uPAR antibodies, precipitated with protein G beads and eluted with a lysis buffer. Immunoblots were preformed with sheep anti-uPAR antibodies and detected by anti-sheep HRP labeled antibodies and visualized by ECL. Subsequently, HRP enzyme was denatured with sodium-azide and the blots were incubated with rabbit anti-SRPX2 antibodies and revealed with anti-rabbit HRP labeled antibodies.

All of the compositions and/or methods disclosed and claimed herein can be made and executed without undue experimentation in light of the present disclosure.

### XII. References

U.S. Patent 3,817,837
U.S. Patent 3,850,752
U.S. Patent 3,939,350
U.S. Patent 3,996,345
U.S. Patent 4,196,265
U.S. Patent 4,275,149
U.S. Patent 4,277,437
U.S. Patent 4,366,241
U.S. Patent 4,469,797
U.S. Patent 4,472,509
U.S. Patent 4,606,855
U.S. Patent 4,659,774
U.S. Patent 4,682,195
U.S. Patent 4,683,202
U.S. Patent 4,703,003
U.S. Patent 4,742,159
U.S. Patent 4,767,720
U.S. Patent 4,816,567
U.S. Patent 4,816,571
U.S. Patent 4,867,973
U.S. Patent 4,870,287
U.S. Patent 4,938,948
U.S. Patent 4,946,778
U.S. Patent 4,959,463
U.S. Patent 5,021,236
U.S. Patent 5,091,513
U.S. Patent 5,091,513
U.S. Patent 5,141,813
U.S. Patent 5,164,296
U.S. Patent 5,196,066
U.S. Patent 5,214,136
U.S. Patent 5,223,409
U.S. Patent 5,223,618
U.S. Patent 5,264,566
U.S. Patent 5,378,825
U.S. Patent 5,403,484
U.S. Patent 5,420,253
U.S. Patent 5,428,148
U.S. Patent 5,446,137
U.S. Patent 5,466,786
U.S. Patent 5,470,967
U.S. Patent 5,539,082
U.S. Patent 5,539,082
U.S. Patent 5,554,744
U.S. Patent 5,565,332
U.S. Patent 5,571,698
U.S. Patent 5,574,146
U.S. Patent 5,602,240
U.S. Patent 5,602,244
U.S. Patent 5,610,289
U.S. Patent 5,614,617
U.S. Patent 5,623,070
U.S. Patent 5,627,052
U.S. Patent 5,645,897
U.S. Patent 5,652,099
U.S. Patent 5,656,434
U.S. Patent 5,670,663
U.S. Patent 5,672,697
U.S. Patent 5,700,922
U.S. Patent 5,705,629
U.S. Patent 5,708,154
U.S. Patent 5,714,331
U.S. Patent 5,714,331
U.S. Patent 5,719,262
U.S. Patent 5,719,262
U.S. Patent 5,736,336
U.S. Patent 5,739,169
U.S. Patent 5,760,395
U.S. Patent 5,763,167
U.S. Patent 5,766,855
U.S. Patent 5,770,376
U.S. Patent 5,773,571
U.S. Patent 5,777,092
U.S. Patent 5,786,461
U.S. Patent 5,789,208
U.S. Patent 5,792,847
U.S. Patent 5,801,005
U.S. Patent 5,821,337
U.S. Patent 5,824,311
U.S. Patent 5,830,880
U.S. Patent 5,844,091
U.S. Patent 5,846,945
U.S. Patent 5,858,657
U.S. Patent 5,858,988
U.S. Patent 5,859,221
U.S. Patent 5,861,155
U.S. Patent 5,871,907
U.S. Patent 5,872,232
U.S. Patent 5,886,165
U.S. Patent 5,891,625
U.S. Patent 5,891,625
U.S. Patent 5,969,108
U.S. Patent 6,054,297
U.S. Patent 6,165,464
U.S. Patent 6,365,157
U.S. Patent 6,406,867
U.S. Patent 6,506,559
U.S. Patent 6,573,099
U.S. Patent 6,673,611
U.S. Patent 6,709,659
U.S. Patent 6,709,873
U.S. Patent 6,753,407
U.S. Patent 6,814,965
U.S. Patent 6,849,259
U.S. Patent 6,861,572
U.S. Patent 6,875,434
U.S. Patent 6,881,557
U.S. Patent 6,891,024
U.S. Patent 6,946,546
U.S. Patent Appln. Ser. No. 117,363
U.S. Patent Publn. 2002/0168707
U.S. Patent Publn. 2002/0172677
U.S. Patent Publn. 2003/0051263
U.S. Patent Publn. 2003/0055020
U.S. Patent Publn. 2003/0159161
U.S. Patent Publn. 2004/0019001
U.S. Patent Publn. 2004/0064842
U.S. Patent Publn. 2004/0126828
U.S. Patent Publn. 2004/0265839
U.S. Patent Publn. 2005/0214860
Aurrand-Lions et al., J. Pathol., 203:700-709, 2004.
Austin-Ward and Villaseca, Revista Medica de Chile, 126(7):838-845, 1998.
Bailey and Sullivan, Biochimica. Biophys. Acts., 239-252, 2000.
Bangham et al., J. Mol. Biol., 13(1):238-252; 253-259, 1965.
Barany and Merrifield, In: The Peptides, Gross and Meienhofer (Eds.), Academic Press, NY, 1-284, 1979.
Barbas et al., Proc. Natl. Acad. Sci., USA, 91:3809-3813, 1994.
Blasi and Carmeliet, Nat. Rev. Mol. Cell. Biol., 3:932-943, 2002.
Bevilacqua, Annu. Rev. Immunol., 11767-804, 1993.
Bevilacqua et al., Science, 243:1160-1165, 1989.
Bukowski et al., Clinical Cancer Res., 4(10):2337-2347, 1998.
Callebaut et al., Protein Sci., 9:1382-1390, 2000.
Carmeliet, Nat. Med., 9:653-660, 2003.
Carmeliet, Nature, 438:932-936, 2005.
Cherqui et al., Mol. Ther., 15:1264-1272, 2007.
Christodoulides et al., Microbiology, 144(Pt 11):3027-3037, 1998.
Collins et al., J. Biol. Chem., 266:2466-2473, 1991.
Colman et al., Curr. Pharm. Des., 12: 2595-2607, 2006.
Das and McGuire, Semin. Ophtamol., 21: 23-27, 2006.
Davidson et al., J. Immunother., 21(5):389-398, 1998.
Deamer and Uster, In: Liposome Preparation: Methods and Mechanisms, Ostro (Ed.), Liposomes, 1983.
Dominguez et al., Proc. Natl. Acad. Sci. USA, 104: 3243-3249, 2007.
Egami et al., J.Leukoc. Biol., 79: 971-976, 2006.
Ferris et al., Arch. Ophthalmol., 102:1640-1642, 1984.
Fire et al., Nature, 391(6669):806-811, 1998.
Folkman, N. Engl. J. Med., 285:1182-1186, 1971.
Folkman, Nat. Med., 1:27-31, 1995.
Gariano and Gardner, Nature, 438:960-966, 2005.
Ghosh and Bachhawat, In: Liver Diseases, Targeted Diagnosis and Therapy Using Specific Receptors and Ligands, Wu et al. (Eds.), Marcel Dekker, NY, 87-104, 1991.
Gram et al., Proc. Natl. Acad. Sci. USA, 89:3576-3580, 1992.
Gregoriadis, In: Drug Carriers in Biology and Medicine, Gregoriadis (Ed.), 287-341, 1979.
Hanahan, Science, 277:48-50, 1997.
Hanibuchi et al., Int. J. Cancer, 78(4):480-485, 1998.
Hellstrand et al., Acta Oncologica, 37(4):347-353, 1998.
Horst et al., J.Clin. Invest., 116: 1596-1605, 2006.
Hu et al, Cancer Res., 56:3055-3061, 1996.
Hui and Hashimoto, Infection Immun., 66(11):5329-5336, 1998.
Ichinose et al., J. Biol. Chem., 265:13411-13414, 1990.
Lakka et al., Brain Pathol., 15: 327-341, 2005.
Johnston et al., J. Biol. Chem., 264:1816-1823, 1989.
Ju et al., Gene Ther., 7(19):1672-1679, 2000.
Jutila et al., J. Exp. Med., 175:1565-1573, 1992.
Kaneda et al., Science, 243:375-378, 1989.
Kansas et al., J. Cell Biol., 114:351-358, 1991.
Kato et al, J. Biol. Chem., 266:3361-3364, 1991.
Klein et al., Arch. Ophthalmol., 102:520-526, 1984.
Kurosawa et al., Blood, 93:321-332, 1999.
Lozier et al., Proc. Natl. Acad. Sci. USA, 81:3640-3644, 1984.
Madsen and Sidenius, Eur. J. Cell Biol., 87: 617-629, 2008.
McMahon and Kwaan, Pathophysiol. Haemost. Thromb., 36: 184-194, 2008.
Marks, Bio/Technology, 10:779-783, 1992.
Meindl et al., Hum. Mol. Genet., 4:2339-2346, 1995.
Mellberg et al., FASEB J., 2009.
Mitchell et al., Ann. NY Acad. Sci., 690:153-166, 1993.
Mitchell et al., J. Clin. Oncol., 8(5):856-869, 1990.
Montesano et al., Cell, 62:435-445, 1990.
Morton et al., Arch. Surg., 127:392-399, 1992.
Nicolau et al., Methods Enzymol., 149:157-176, 1987.
Oh et al., Blood, 110: 3891-3899, 2007.
O'Leary et al., Protein Sci., 13:1238-1250, 2004.
Oliveira-Ferrer et al., Cancer Res., 64: 8932-8938, 2004.
Pepper et al., Enzyme Protein, 49:138-162, 1996.
Pietras et al., Oncogene, 17(17):2235-2249, 1998.
Pigott et al., J. Immunol., 147:130-135, 1991.
Qin et al., Proc. Natl. Acad. Sci. USA, 95(24):14411-14416, 1998.
Ravindranath and Morton, Intern. Rev. Immunol., 7: 303-329, 1991.
Remington's Science and Practice of Pharmacy, 21st Ed., Mack Printing Company, 2005.
Risau, Nature, 386:671-674, 1997.
Robb et al., Proc. Natl. Acad. Sci. USA, 85:5654-5658, 1988.
Roll et al., Hum. Mol. Genet., 15:1195-207, 2006.
Rosenberg et al., Ann. Surg. 210(4):474-548, 1989.
Rosenberg et al., N. Engl. J. Med., 319:1676, 1988.
Royer et al., BMC Genet 8: 72, 2007.
Royer-Zemmour et al., Hum. Mol. Genet., 17:3617-3630, 2008.
Schier et al., J. Mol. Biol. 263:551-567, 1996.
Song et al., Nature Med. 9:347-351, 2003.
Soutschek et al., Nature, 432:173-178, 2004.
Stemmer, Nature, 370:389-391, 1994
Stewart and Young, In: Solid Phase Peptide Synthesis, 2d. ed., Pierce Chemical Co., 1984.
Szoka and Papahadjopoulos, Proc. Natl. Acad. Sci. USA, 75:4194 4198, 1978.
Tam et al., J. Am. Chem. Soc., 105:6442, 1983.
Tanaka et al., Proceedings of the American Association for Cancer Research Annual Meeting, Vol. 48, April 2007, page 474.

Tanaka et al., Int. J. Cancer, 124:1072-1080, 2009.
Tedder et al. Faseb J, 9, 866-873, 1995.
Wadhwa et al., Curr. Opin. Mol. Ther., 6(4):367-372, 2004.
Wei et al., J. Immunol., 167, 277-282, 2001.
Wessel et al., Dev. Biol., 193, 115-126, 1998.
Wong et al., Gene, 10:87-94, 1980.
Yancopoulos et al., Nature, 407, 242-248, 2000.

## Claims

1. An antibody or a fragment thereof that binds to an SRPX2 amino acid sequence selected from SEQ ID NOs: 14-23 and inhibits the activity of SRPX2 in angiogenesis.

2. A pharmaceutical composition comprising the antibody or a fragment thereof of claim 1 and a pharmaceutically acceptable carrier.

3. An antibody or a fragment thereof of claim 1 for use in the treatment of an angiogenesis related condition.

4. An antibody or a fragment thereof of claim 1 for use in the treatment of an angiogenesis related condition of claim 3, wherein the angiogenesis-related condition is cancer, ocular neovascularization, arterio-venous malformations, coronary restenosis, peripheral vessel restenosis, glomerulonephritis, rheumatoid arthritis, ischemic cardiovascular pathologies, chronic inflammatory diseases.

5. An antibody or a fragment thereof of claim 1 for use in the treatment of an angiogenesis related condition of claim 4, wherein the said cancer is selected from the group consisting of breast cancer, lung cancer, prostate cancer, ovarian cancer, brain cancer, liver cancer, cervical cancer, colorectal cancer, renal cancer, skin cancer, head and neck cancer, bone cancer, esophageal cancer, bladder cancer, uterine cancer, lymphatic cancer, stomach cancer, pancreatic cancer, testicular cancer, lymphoma, and leukemia.

6. The pharmaceutical composition of claim 2, wherein the pharmaceutical composition further comprises a lipid component.

7. The pharmaceutical composition of claim 6, wherein the lipid component forms a liposome.

8. The pharmaceutical composition of claim 6, wherein the lipid is 1,2-dioleoyl-sn-glycero-3-phosphatidylcholine (DOPC), egg phosphatidylcholine ("EPC"), dilauryloylphosphatidylcholine ("DLPC"), dimyristoylphosphatidylcholine ("DMPC"), dipalmitoylphosphatidylcholine ("DPPC"), distearoylphosphatidylcholine ("DSPC"), 1-myristoyl-2-palmitoyl phosphatidylcholine ("MPPC"), I-palmitoyl-2-myristoyl phosphatidylcholine ("PMPC"), 1-palmitoyl-2-stearoyl phosphatidylcholine ("PSPC"), 1-stearoyl-2-palmitoyl phosphatidylcholine ("SPPC"), dimyristyl phosphatidylcholine ("DMPC"), 1,2-distearoyl-sn-glycero-3-phosphocholine ("DAPC"), 1,2-diarachidoyl-sn-glycero-3-phosphocholine ("DBPC"), 1,2-dieicosenoyl-sn-glycero-3-phosphocholine ("DEPC"), palmitoyloeoyl phosphatidylcholine ("POPC"), lysophosphatidylcholine, dilinoleoylphosphatidylcholine, distearoylphophatidylethanolamine ("DSPE"), dimyristoyl phosphatidylethanolamine ("DMPE"), dipalmitoyl phosphatidylethanolamine ("DPPE"), palmitoyloeoyl phosphatidylethanolamine ("POPE"), lysophosphatidylethanolamine, phosphatidylserine, phosphatidylglycerol, dimyristoyl phosphatidylserine ("DMPS"), dipalmitoyl phosphatidylserine ("DPPS"), brain phosphatidylserine ("BPS"), dilauryloylphosphatidylglycerol ("DLPG"), dimyristoylphosphatidylglycerol ("DMPG"), dipalmitoylphosphatidylglycerol ("DPPG"), distearoylphosphatidylglycerol ("DSPG"), or dioleoylphosphatidylglycerol ("DOPG").

9. The pharmaceutical composition of claim 2, wherein the pharmaceutical composition further comprises cholesterol or polyethyleneglycol (PEG).

## Patentansprüche

1. Antikörper oder Fragment davon, der/das an eine SRPX2 Aminosäuresequenz bindet, die ausgewählt ist aus SEQ ID NOs:14-23 und die Aktivität von SRPX2 in der Angiogenese inhibiert.

2. Arzneimittel, das den Antikörper oder das Fragment davon nach Anspruch 1 und einen pharmazeutisch verträglichen Träger umfasst.

3. Antikörper oder Fragment davon nach Anspruch 1 zur Verwendung bei der Behandlung eines Zustands, der mit Angiogenese in Zusammenhang steht.

4. Antikörper oder Fragment davon nach Anspruch 1 zur Verwendung bei der Behandlung eines Zustands, der mit Angiogenese in Zusammenhang steht, nach Anspruch 3, wobei der Zustand, der mit Angiogenese in Zusammenhang steht, Krebs, okuläre Neovaskularisation, arteriovenöse Fehlbildungen, koronare Restenose, periphere Gefäßrestenose, Glomerulonephritis, rheumatoide Arthritis, ischämische kardiovaskuläre Erkrankungen oder chronische Entzündungserkrankungen ist.

5. Antikörper oder Fragment davon nach Anspruch 1 zur Verwendung bei der Behandlung eines Zustands, der mit Angiogenese in Zusammenhang steht, nach Anspruch 4, wobei der Krebs ausgewählt ist aus der Gruppe bestehend aus Brustkrebs, Lungenkrebs, Prostatakrebs, Eierstockkrebs, Hirntumor, Lebertumor, Gebärmutterhalskrebs, Darmkrebs, Nierenkrebs, Hautkrebs, Kopf- und Halskrebs, Knochenkrebs, Speiseröhrenkrebs, Blasenkrebs, Gebärmutterkrebs, Lymphkrebs, Magenkrebs, Bauchspeicheldrüsenkrebs, Hodenkrebs, Lymphom und Leukämie.

6. Arzneimittel nach Anspruch 2, wobei das Arzneimittel zudem eine Lipidkomponente umfasst.

7. Arzneimittel nach Anspruch 6, wobei die Lipidkomponente ein Liposom bildet.

8. Arzneimittel nach Anspruch 6, wobei das Lipid 1,2-Dioleoyl-sn-glycero-3-phosphatidylcholin (DOPC), Ei-Phosphatidylcholin ("EPC"), Dilauryloylphosphatidylcholin ("DLPC"), Dimyristoylphosphatidylcholin ("DMPC"), Dipalmitoylphosphatidylcholin ("DPPC"), Distearoylphosphatidylcholin ("DSPC"), 1-Myristoyl-2-palmitoylphosphatidylcholin ("MPPC"), 1-Palmitoyl-2-myristoylphosphatidylcholin ("PMPC"), 1-Palmitoyl-2-stearoylphosphatidylcholin ("PSPC"), 1-Stearoyl-2-palmitoylphosphatidylcholin ("SPPC"), Dimyristylphosphatidylcholin ("DMPC"), 1,2-Distearoyl-sn-glycero-3-phosphocholin ("DAPC"), 1,2-Diarachidoyl-sn-glycero-3-phosphocholin ("DBPC"), 1,2-Dieicosenoyl-sn-glycero-3-phosphocholin ("DEPC"), Palmitoyloeoylphosphatidylcholin ("POPC"), Lysophosphatidylcholin, Dilinoleoylphosphatidylcholin, Distearoylphophatidylethanolamin ("DSPE"), Dimyristoylphosphatidylethanolamin ("DMPE"), Dipalmitoylphosphatidylethanolamin ("DPPE"), Palmitoyloeoylphosphatidylethanolamin ("POPE"), Lysophosphatidylethanolamin, Phosphatidylserin, Phosphatidylglycerol, Dimyristoylphosphatidylserin ("DMPS"), Dipalmitoyl-phosphatidylserin ("DPPS"), Hirn-Phosphatidylserin ("BPS"), Dilauryloylphosphatidylglycerol ("DLPG"), Dimyristoylphosphatidylglycerol ("DMPG"), Dipalmitoylphosphatidylglycerol ("DPPG"), Distearoylphosphatidylglycerol ("DSPG") oder Dioleoylphosphatidylglycerol ("DOPG") ist.

9. Arzneimittel nach Anspruch 2, wobei das Arzneimittel zudem Cholesterol oder Polyethylenglycol (PEG) umfasst.

## Revendications

1. Anticorps ou fragment de celui-ci qui se lie à une séquence d'acides aminés de SRPX2 sélectionnée parmi SEQ ID NO: 14-23 et qui inhibe l'activité de SRPX2 dans l'angiogenèse.

2. Composition pharmaceutique comprenant l'anticorps ou un fragment de celui-ci selon la revendication 1 et un véhicule pharmaceutiquement acceptable.

3. Anticorps ou fragment de celui-ci selon la revendication 1 destiné à être utilisé dans le traitement d'une affection associée à l'angiogenèse.

4. Anticorps ou fragment de celui-ci selon la revendication 1 destiné à être utilisé dans le traitement d'une affection associée à l'angiogenèse selon la revendication 3, où l'affection associée à l'angiogenèse est le cancer, la néovascularisation oculaire, des malformations artério-veineuses, une resténose coronarienne, une resténose d'un vaisseau périphérique, la glomérulonéphrite, la polyarthrite rhumatoïde, des pathologies cardiovasculaires ischémiques, des maladies inflammatoires chroniques.

5. Anticorps ou fragment de celui-ci selon la revendication 1 destiné à être utilisé dans le traitement d'une affection associée à l'angiogenèse selon la revendication 4, où ledit cancer est sélectionné dans le groupe consistant en le cancer du sein, le cancer du poumon, le cancer de la prostate, le cancer de l'ovaire, le cancer du cerveau, le cancer du foie, le cancer du col de l'utérus, le cancer colorectal, le cancer du rein, le cancer de la peau, le cancer de la tête et du cou, le cancer des os, le cancer de l'oesophage, le cancer de la vessie, le cancer de l'utérus, le cancer du système lymphatique, le cancer de l'estomac, le cancer du pancréas, le cancer du testicule, un lymphome et une leucémie.

6. Composition pharmaceutique selon la revendication 2, où la composition pharmaceutique comprend en outre un composant lipidique.

7. Composition pharmaceutique selon la revendication 6, dans laquelle le composant lipidique forme un liposome.

8. Composition pharmaceutique selon la revendication 6, dans laquelle le lipide est la 1,2-dioléoyl-sn-glycéro-3-phosphatidylcholine (DOPC), la phosphatidylcholine de l'oeuf (« EPC »), la dilauryloylphosphatidylcholine (« DLPC »), la dimyristoylphosphatidylcholine (« DMPC »), la dipalmitoylphosphatidylcholine (« DPPC »), la distéaroylphosphatidylcholine (« DSPC »), la 1-myristoyl-2-palmitoylphosphatidylcholine (« MPPC »), la 1-palmitoyl-2-myristoylphosphatidylcholine (« PMPC »), la 1-palmitoyl-2-stéaroylphosphatidylcholine (« PSPC »), la 1-stéaroyl-2-palmitoylphosphatidylcholine (« SPPC »), la dimyristoylphosphatidylcholine (« DMPC »), la 1,2-distéaroyl-sn-glycéro-3-phosphocholine (« DAPC »), la 1,2-diarachidoyl-sn-glycéro-3-phosphocholine (« DBPC »), la 1,2-diéicosénoyl-sn-glycéro-3-phosphocholine (« DEPC »), la palmitoyloléoylphosphatidylcholine (« POPC »), la lysophosphatidylcholine, la dilinoléoylphosphatidylcholine, la distéaroylphosphatidyléthanolamine (« DSPE »), la dimyristoylphosphatidyléthanolamine (« DMPE »), la dipalmitoylphosphatidyléthanolamine (« DPPE »), la palmitoyloléoylphosphatidyléthanolamine (« POPE »), la lysophosphatidyléthanolamine, la phosphatidylsérine, le phosphatidylglycérol, la dimyristoylphosphatidylsérine (« DMPS »), la dipalmitoylphosphatidylsérine (« DPPS »), la phosphatidylsérine cérébrale (« BPS »), le dilauryloylphosphatidylglycérol (« DLPG »), le dimyristoylphosphatidylglycérol (« DMPG »), le dipalmitoylphosphatidylglycérol (« DPPG »), le distéaroylphosphatidylglycérol (« DSPG »), où le dioléoylphosphatidylglycérol (« DOPG »).

9. Composition pharmaceutique selon la revendication 2, où la composition pharmaceutique comprend en outre du cholestérol ou du polyéthylène glycol (PEG).
